# NEUE EUROPÄISCHE PATENTSCHRIFT

(11) **EP 3 210 248 B2**
(45) Veröffentlichungstag und Bekanntmachung des Hinweises auf die Entscheidung über den Einspruch: **10.04.2024**
(45) Hinweis auf die Patenterteilung: 15.08.2018
(21) Anmeldenummer: 15775626.3
(22) Anmeldetag: 25.09.2015
(51) Int. Cl.: H10K 50/115, H10K 85/30, H10K 85/60

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 24.10.2014 EP 14003629; 07.08.2015 EP 15180258
(43) Veröffentlichungstag der Anmeldung: 30.08.2017
(73) Patentinhaber: Merck Patent GmbH, 64293 Darmstadt (DE)
(72) Erfinder: VOGES, Frank, 67098 Bad Duerkheim (DE); STIEBER, Frank, 64683 Einhausen (DE); STOESSEL, Philipp, 60389 Frankfurt am Main (DE); MUJICA-FERNAUD, Teresa, 64293 Darmstadt (DE); PFLUMM, Christof, 64291 Darmstadt (DE); KAISER, Joachim, 64289 Darmstadt (DE)
(74) Vertreter: Stolmár & Partner Patentanwälte PartG mbB
(86) Internationale Anmeldenummer: PCT/EP2015/001910
(87) Internationale Veröffentlichungsnummer: WO 2016/062368

(56) Entgegenhaltungen:
- WO-A1-2013/129836
- WO-A1-2015/018539
- WO-A1-2015/018539
- WO-A2-2013/182389
- WO-A2-2013/182389
- DE-A1-102007 018 456
- KR-A- 20120 009 984
- US-A1- 2011 266 533
- US-A1- 2013 316 134
- US-A1- 2013 334 517
- US-A1- 2014 027 747
- US-A1- 2014 217 392
- US-A1- 2014 264 313
- US-A1- 2014 264 313
- US-A1- 2015 123 047
- EVGENY V. DIKAREV ET AL: "Tuning the Properties at Heterobimetallic Core: Mixed-Ligand Bismuth-Rhodium Paddlewheel Carboxylates", JOURNAL OF THE AMERICAN CHEMICAL SOCIETY, Bd. 128, Nr. 9, 1. März 2006 (2006-03-01), Seiten 2814-2815, XP055088058, ISSN: 0002-7863, DOI: 10.1021/ja058294h
- GÜNTER SCHMID ET AL: "Fluorinated Copper(I) Carboxylates as Advanced Tunable p-Dopants for Organic Light-Emitting Diodes", ADVANCED MATERIALS, Bd. 26, Nr. 6, 20. Dezember 2013 (2013-12-20), Seiten 878-885, XP055107776, ISSN: 0935-9648, DOI: 10.1002/adma.201303252

## Beschreibung

Die vorliegende Anmeldung betrifft ein Material, enthaltend ein Mono-Arylamin einer definierten Formel und einen Metallkomplex einer definierten Formel. Weiterhin betrifft die vorliegende Anmeldung die Verwendung des genannten Materials in einer organischen Schicht einer elektronischen Vorrichtung, wobei die Vorrichtung bevorzugt eine organische Elektrolumineszenzvorrichtung (OLED) ist.

Unter dem Begriff "enthaltend" wird im Rahmen dieser Anmeldung verstanden, dass weitere Bestandteile oder Schritte vorhanden sein können. Der unbestimmte Artikel "ein" schließt nicht den Plural aus.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten.

Der Aufbau von OLEDs, in denen organische Verbindungen als funktionelle Materialien eingesetzt werden, ist beispielsweise in US 4539507, US 5151629, EP 0676461 und WO 98/27136 beschrieben. Allgemein werden unter der Bezeichnung OLEDs elektronische Vorrichtungen verstanden, welche eine oder mehrere Schichten enthaltend organische Verbindungen aufweisen und unter Anlegen von elektrischer Spannung Licht emittieren.

Einen großen Einfluss auf die Leistungsdaten von elektronischen Vorrichtungen haben Schichten mit lochtransportierender Funktion (lochtransportierende Schichten), wie beispielsweise Lochinjektionsschichten, Lochtransportschichten und Elektronenblockierschichten.

Es ist im Stand der Technik bekannt, Mono-Arylamine als Materialien für lochtransportierende Schichten zu verwenden. Solche Mono-Arylamine sind beispielsweise in JP 1995/053955, WO 2006/123667, JP 2010/222268, WO 2012/034627, WO 2013/120577, WO 2014/015938 und WO 2014/015935 beschrieben.

Weiterhin ist es im Stand der Technik bekannt, p-Dotanden in Kombination mit Lochtransportmaterialien in lochtransportierenden Schichten von OLEDs einzusetzen. Unter einem p-Dotanden wird dabei eine Verbindung verstanden, welche, wenn sie als Mindermengenkomponente einer Hauptkomponente hinzugefügt wird, deren Leitfähigkeit für Löcher signifikant erhöht.

Als p-Dotanden im Stand der Technik bekannt sind organische Elektronenakzeptorverbindungen, wie beispielsweise 7,7,8,8-Tetracyano-2,3,5,6-tetrafluorchinodimethan (F4TCNQ). Weiterhin im Stand der Technik als p-Dotanden bekannt sind Metallkomplexe von Übergangsmetallkationen und Hauptgruppenmetallkationen, wie beispielsweise aus WO 2011/33023 und WO 2013/182389.

Aus den im Stand der Technik bekannten Lochtransportmaterialien und den im Stand der Technik bekannten p-Dotanden ergibt sich eine große Vielfalt von potentiell möglichen Kombinationen. Hiervon sind nur einige im Stand der Technik offenbart. Beispielhaft ist hier die Kombination von Hauptgruppenmetallkomplexen als p-Dotanden mit Tetra-Aminen wie beispielsweise 2,2',7,7'-Tetra(N,N -di-p-tolyl)amino-9,9-spirobifluoren in der Lochtransportschicht einer OLED zu nennen. Diese ist in WO 2013/182389 offenbart. Ein weiteres Beispiel aus dem Stand der Technik ist die Kombination von F4TCNQ mit Mono-Arylaminen wie beispielsweise Tris-para-biphenylamin in der Lochtransportschicht einer OLED. Diese ist in WO 2013/135352 offenbart.

OLEDs mit diesen Materialien in der Lochtransportschicht weisen jedoch Verbesserungsbedarf in Bezug auf die Lebensdauer und die Effizienz auf.

Weiterhin besteht Bedarf an p-Dotanden, welche in der Lage sind, Lochtransportmaterialien mit tiefliegendem HOMO, insbesondere solche mit einem HOMO im Bereich von -5.2 bis -5.7 eV, effizient zu dotieren, so dass Dotand-Lochtransportmaterial-Kombinationen erhalten werden können, welche sowohl eine geeignete Leitfähigkeit aufweisen, als auch ein niedriges HOMO-Energieniveau. Geeignete und bevorzugte Leitfähigkeiten liegen dabei im Bereich von 10⁻⁴ S/m bis 10⁻³ S/m. Die Verwendung von Lochtransportmaterialien mit tiefliegendem HOMO ist hoch erwünscht, weil damit die Notwendigkeit entfällt, zwischen Lochtransportschicht und emittierender Schicht eine weitere Schicht einzufügen, die ein niedriges HOMO aufweist. Dies ermöglicht einen einfacheren Aufbau der OLED und damit ein effizienteres Herstellungsverfahren. Wird eine weitere Schicht zwischen Lochtransportschicht und emittierender Schicht eingefügt, so ist es im gewünschten Fall einer Lochtransportschicht mit niedrigem HOMO möglich, eine Löcher-Barriere und damit einen Spannungsabfall zwischen der Lochtransportschicht und der emittierenden Schicht zu vermeiden, indem das HOMO der Lochtransportschicht gleich hoch oder niedriger liegt als das HOMO der Schicht zwischen Lochtransportschicht und emittierender Schicht. Dies ist beispielsweise durch die Verwendung des gleichen Materials in Lochtransportschicht und weiterer Schicht zwischen Lochtransportschicht und emittierender Schicht möglich.

Weiterhin besteht Bedarf an Lochtransportmaterial-Dotand-Kombinationen, welche nur geringe Absorption im sichtbaren Bereich (VIS-Bereich) aufweisen. Die im Stand der Technik bekannten p-Dotanden, beispielsweise NDP-2 oder Molybdänoxid-Dotanden, weisen in Kombination mit üblichen Lochtransportmaterialien Absorptionen im VIS-Bereich auf. Geringe Absorptionsbanden im VIS-Bereich sind hoch erwünscht, da Absorptionen im VIS-Bereich die Emissionscharakteristik der OLEDs beeinflussen und ihre Effizienz verschlechtern.

Bei Untersuchungen zu möglichen Kombinationen von p-Dotanden und Lochtransportmaterialien zur Verwendung in Lochtransportschichten wurde nun unerwartet festgestellt, dass ein Material, enthaltend ein Mono-Arylamin, das gewählt ist aus Verbindungen gemäß einer der Formeln (A-II) und (A-IV) bis (A-IX), oder aus Verbindungen gemäß Formel (A-I) und (A-III), und einen Bismut-Komplex, hervorragende Werte verglichen mit dem Stand der Technik in Bezug auf Lebensdauer und Effizienz liefert. Weiterhin weist das erfindungsgemäße Material geringere Leckströme bei der Verwendung in OLEDs auf als Materialien gemäß dem Stand der Technik. Dies mag, ohne an diese Theorie gebunden zu sein, durch eine geringere laterale Leitfähigkeit der dotierten Schicht der OLED verursacht sein. Leckströme sind bei kleiner werdenden Pixel in Displays ein großes Problem, da sie zu einem Crosstalk (Übersprechen) zwischen den Pixeln führen können. Ihre Vermeidung ist daher wünschenswert. Nochmals weiterhin weist das erfindungsgemäße Material keine Absorptionsbande im VIS-Bereich auf. Nochmals weiterhin können mit dem erfindungsgemäßen Material aufgrund der tiefen HOMO-Lage des Mono-Arylamins OLEDs hergestellt werden, welche keine zusätzliche Schicht zwischen der Lochtransportschicht enthaltend das erfindungsgemäße Material und der emittierenden Schicht aufweisen müssen und daher effizienter hergestellt werden können.

Gegenstand der vorliegenden Erfindung ist daher ein Material, enthaltend eine Verbindung P, die ein Komplex von Bismut ist, und eine Verbindung A, die gewählt ist aus Verbindungen gemäß einer der Formeln (A-II) und (A-IV) bis (A-IX)

| | |
|---|---|
| | 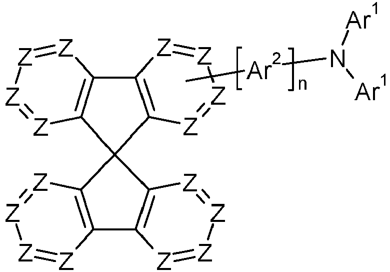 |
| | Formel (A-II) |
| 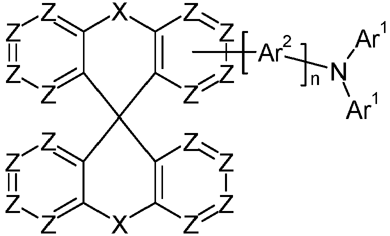 | |
| Formel (A-IV) | |
| 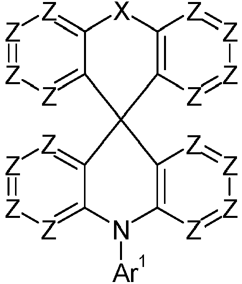 | 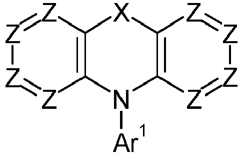 |
| Formel (A-V) | Formel (A-VI) |
| 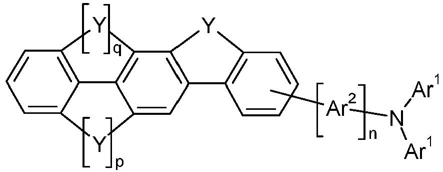 | 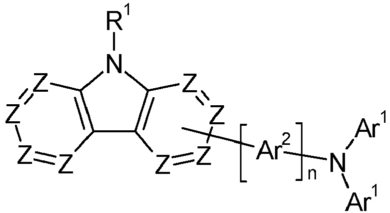 |
| Formel (A-VII) | Formel (A-VIII) |
| 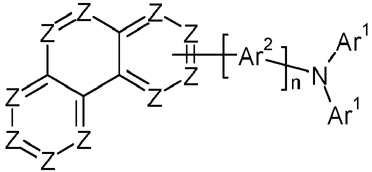 | |
| Formel (A-IX) | |

wobei an allen unsubstituiert dargestellten Positionen ein oder mehrere Reste R¹ gebunden sein können, und für die auftretenden Variablen gilt:
- Z: ist bei jedem Auftreten gleich oder verschieden CR¹ oder N;
- X: ist bei jedem Auftreten gleich oder verschieden eine Einfachbindung, O, S, C(R¹)₂, Si(R¹)₂, PR¹, C(R¹)₂-C(R¹)₂, oder CR¹=CR¹;
- Y: ist bei jedem Auftreten gleich oder verschieden O, S, C(R¹)₂, Si(R¹)₂, PR¹, NR¹, C(R¹)₂-C(R¹)₂, oder CR¹=CR¹;
- Ar¹: ist bei jedem Auftreten gleich oder verschieden ein aromatisches Ringsystem mit 6 bis 60 aromatischen Ringatomen, das mit einem oder mehreren Resten R¹ substituiert sein kann, oder ein heteroaromatisches Ringsystem mit 5 bis 60 aromatischen Ringatomen, das mit einem oder mehreren Resten R¹ substituiert sein kann; dabei können Gruppen Ar¹ untereinander über Reste R¹ verbunden sein;
- Ar²: ist ein aromatisches Ringsystem mit 6 bis 20 aromatischen Ringatomen, das mit einem oder mehreren Resten R¹ substituiert sein kann, oder ein heteroaromatisches Ringsystem mit 5 bis 20 aromatischen Ringatomen, das mit einem oder mehreren Resten R¹ substituiert sein kann;
- R¹: ist bei jedem Auftreten gleich oder verschieden gewählt aus H, D, F, C(=O)R², CN, Si(R²)₃, P(=O)(R²)₂, OR², S(=O)R², S(=O)₂R², geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R² substituiert sein können; und wobei eine oder mehrere CH₂-Gruppen in den genannten Alkyl-, Alkoxy-, Alkenyl- und Alkinylgruppen durch -R²C=CR²-, -C≡C-, Si(R²)₂, C=O, C=NR², -C(=O)O-, -C(=O)NR²-, P(=O)(R²), -O-, -S-, SO oder SO₂ ersetzt sein können;
- R²: ist bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R² miteinander verknüpft sein können und einen Ring bilden können; und wobei die genannten Alkylgruppen, aromatischen Ringsysteme und heteroaromatischen Ringsysteme mit F oder CN substituiert sein können;
- n,p,q: ist gleich oder verschieden 0 oder 1;
oder die gewählt ist aus Verbindungen gemäß Formel (A-I) und (A-III):

| |
|---|
| 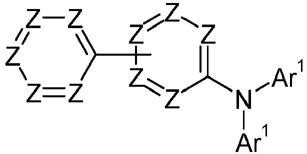 |
| Formel (A-I) |
| 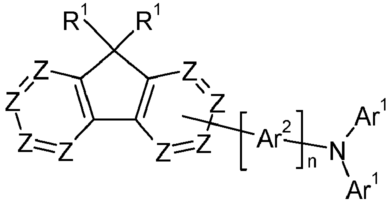 |
| Formel (A-III), |

wobei an allen unsubstituiert dargestellten Positionen ein oder mehrere Reste R¹ gebunden sein können, und
mindestens eine Gruppe Ar¹ bei jedem Auftreten gleich oder verschieden gewählt ist aus den untenstehend genannten Gruppen (Ar¹-1) bis (Ar¹-88), die jeweils an allen unsubstituiert dargestellten Positionen mit einem oder mehreren Resten R¹ substituiert sein können;
und wobei die sonstigen Variablen wie oben definiert sind;

wobei vom Umfang der Formeln (A-I) bis (A-IX) jeweils Verbindungen der folgenden Formel (B) ausgenommen sind 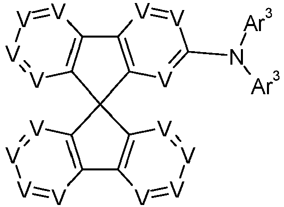 bei der für die neu auftretenden Variablen gilt:
- V: ist gleich CR¹;
- Ar³: ist bei jedem Auftreten gleich oder verschieden ein aromatisches Ringsystem mit 6 bis 30 aromatischen Ringatomen, das mit einem oder mehreren Resten R¹ substituiert sein kann, oder ein heteroaromatisches Ringsystem mit 5 bis 30 aromatischen Ringatomen, das mit einem oder mehreren Resten R¹ substituiert sein kann, dabei können Gruppen Ar¹ untereinander über Reste R¹ verbunden sein.

Ein aromatisches Ringsystem im Sinne dieser Erfindung enthält 6 bis 60 C-Atome im Ringsystem. Es umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Ein heteroaromatisches Ringsystem im Sinne dieser Erfindung enthält 5 bis 60 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 20 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Die Verbindung A ist bevorzugt ein Mono-Arylamin. Unter einem Mono-Arylamin wird dabei eine Verbindung verstanden, die eine einzige Arylaminogruppe aufweist, und nicht mehrere. Bevorzugt ist die Verbindung eine Mono-Triarylaminoverbindung, d.h. sie weist eine einzige Triarylaminogruppe auf. Unter der Bezeichnung Triarylaminogruppe werden dabei bevorzugt auch Verbindungen verstanden, die Heteroarylgruppen gebunden an den Amino-Stickstoff enthalten. Weiterhin bevorzugt weist die Verbindung A eine einzige Aminogruppe auf. Es wird angemerkt, dass gemäß der Definition der vorliegenden Anmeldung Carbazolgruppen nicht als Arylaminogruppen bzw. Aminogruppen gelten.

Gemäß einer weiteren bevorzugten Ausführungsform der Erfindung enthält die Verbindung A keine kondensierte Arylgruppe mit mehr als 10 aromatischen Ringatomen und keine kondensierte Heteroarylgruppe mit mehr als 14 aromatischen Ringatomen.

Ar¹ ist in den Verbindungen der Formeln (A-II) und (A-IV) bis (A-IX) bevorzugt bei jedem Auftreten gleich oder verschieden ein aromatisches Ringsystem mit 6 bis 40 aromatischen Ringatomen, das mit einem oder mehreren Resten R¹ substituiert sein kann, oder ein heteroaromatisches Ringsystem mit 5 bis 40 aromatischen Ringatomen, das mit einem oder mehreren Resten R¹ substituiert sein kann.

Bevorzugt ist mindestens eine Gruppe Ar¹ in den Verbindungen der Formeln (A-II) und (A-IV) bis (A-IX) eine wahlweise mit einem oder mehreren Resten R¹ substituierte Gruppe gewählt aus Phenyl, Biphenyl, Terphenyl, Quaterphenyl, Naphthyl, Phenanthryl, Fluoranthenyl, Fluorenyl, Indenofluorenyl, Spirobifluorenyl, Furanyl, Benzofuranyl, Isobenzofuranyl, Dibenzofuranyl, Thiophenyl, Benzothiophenyl, Isobenzothiophenyl, Dibenzothiophenyl, Indolyl, Isoindolyl, Carbazolyl, Indolocarbazolyl, Indenocarbazolyl, Pyridyl, Chinolinyl, Isochinolinyl, Acridyl, Phenanthridyl, Benzimidazolyl, Pyrimidyl, Pyrazinyl, und Triazinyl; besonders bevorzugt sind darunter Phenyl, Biphenyl, Terphenyl, Quaterphenyl, Naphthyl, Phenanthryl, Fluoranthenyl, Fluorenyl, Indenofluorenyl, Spirobifluorenyl, Dibenzofuranyl, Dibenzothiophenyl, Carbazolyl, Acridyl, und Phenanthridyl.

R¹ ist in den Verbindungen der Formeln (A-I) bis (A-IX) bevorzugt bei jedem Auftreten gleich oder verschieden gewählt aus H, D, F, CN, Si(R²)₃, geradkettigen Alkyl- oder Alkoxygruppen mit 1 bis 10 C-Atomen, verzweigten oder cyclischen Alkyl- oder Alkoxygruppen mit 3 bis 10 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R² substituiert sein können; und wobei in den genannten Alkyl- oder Alkoxygruppen eine oder mehrere CH₂-Gruppen durch -C≡C-, -R²C=CR²-, Si(R²)₂, C=O, C=NR², -O-, -S-, -C(=O)O- oder -C(=O)NR²- ersetzt sein können.

Bevorzugt ist mindestens eine Gruppe Ar¹, besonders bevorzugt sind alle Gruppen Ar¹ in den Verbindungen der Formeln (A-II) und (A-IV) bis (A-IX) bei jedem Auftreten gleich oder verschieden gewählt aus den folgenden Gruppen, die jeweils an allen unsubstituiert dargestellten Positionen mit einem oder mehreren Resten R¹ substituiert sein können:

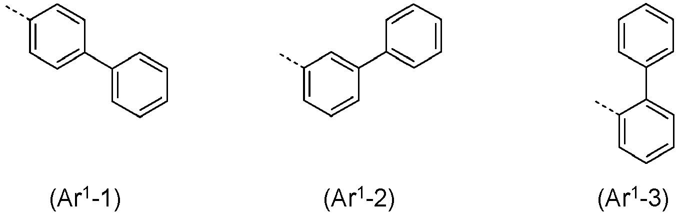

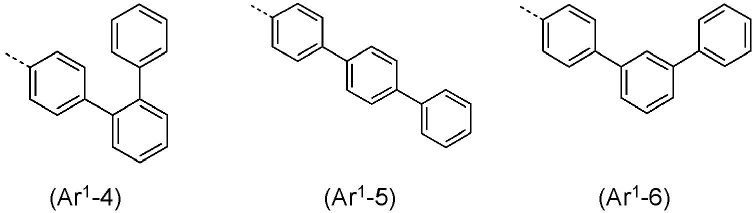

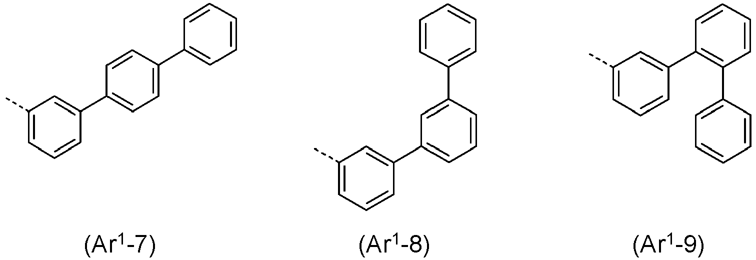

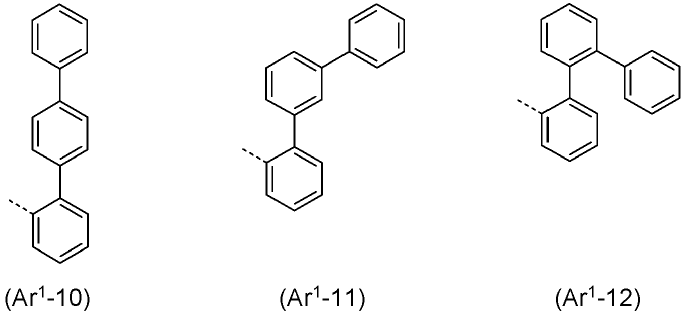

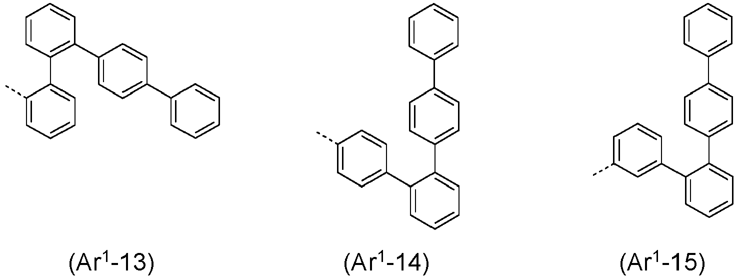

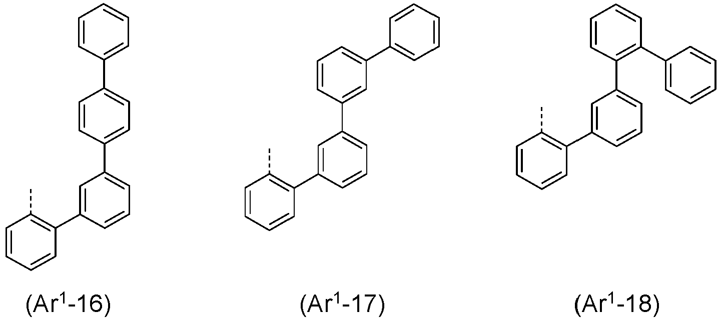

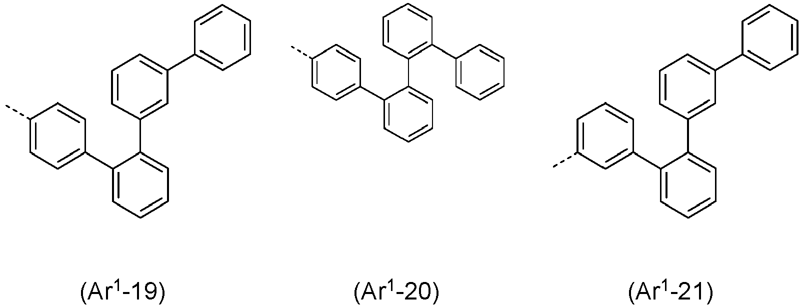

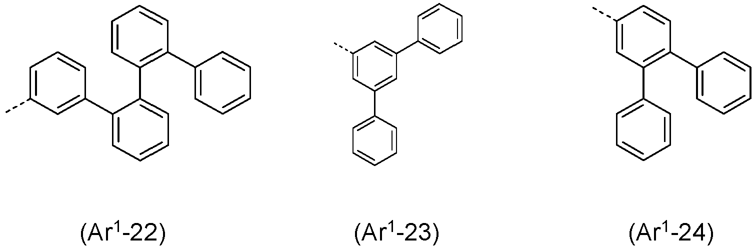

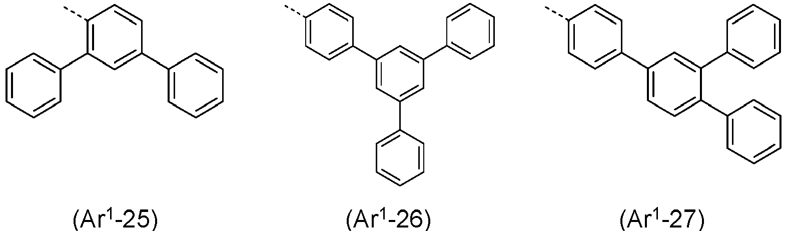

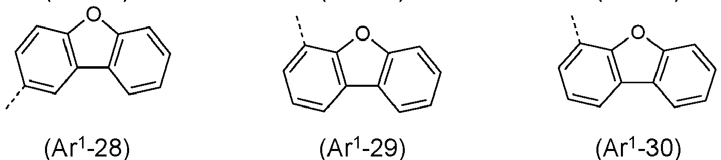

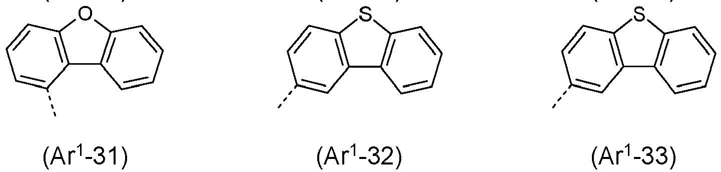

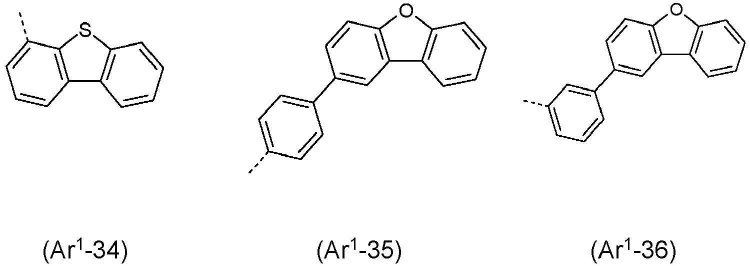

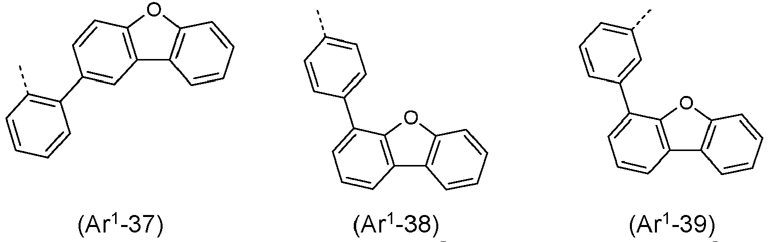

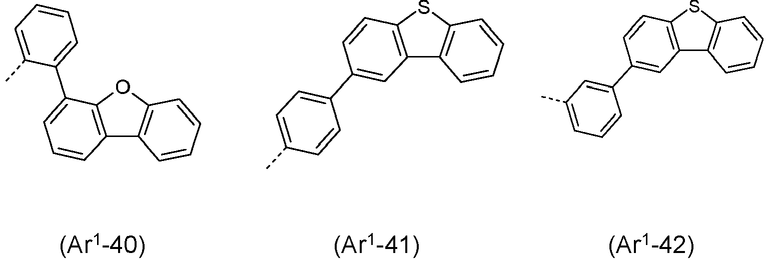

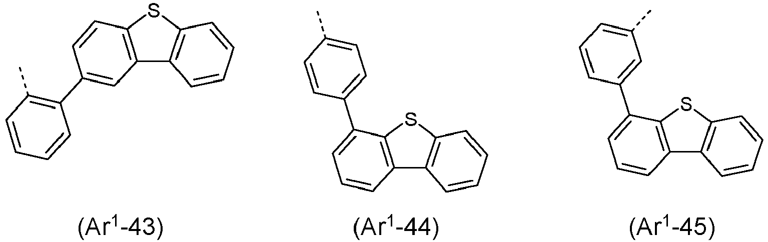

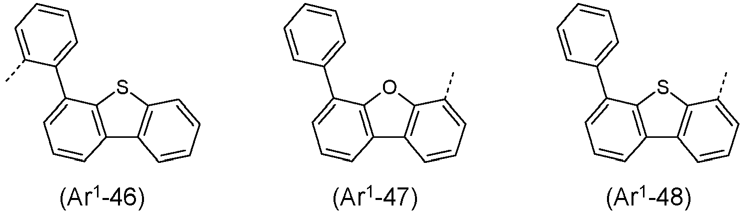

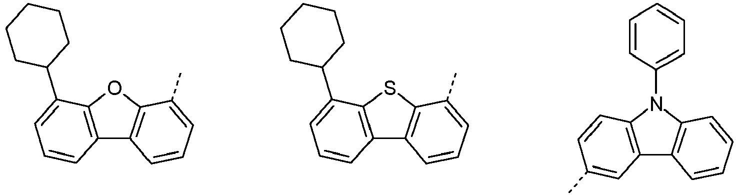

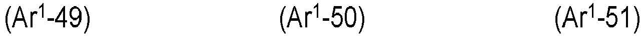

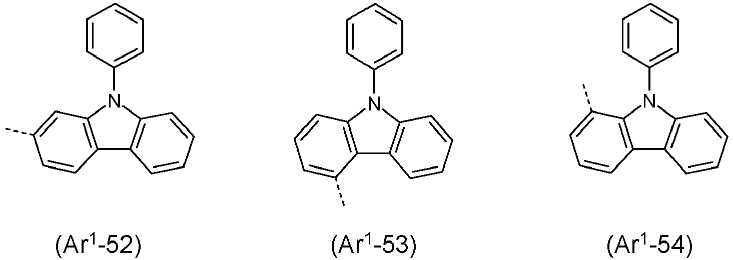

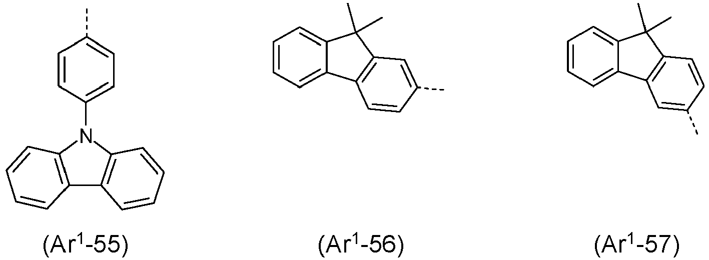

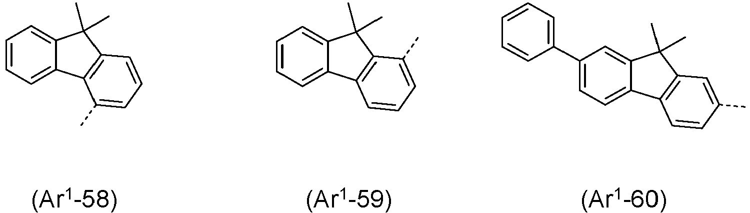

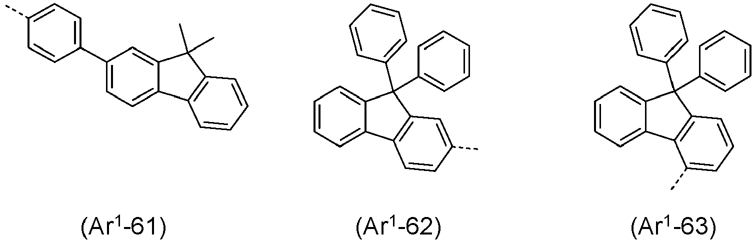

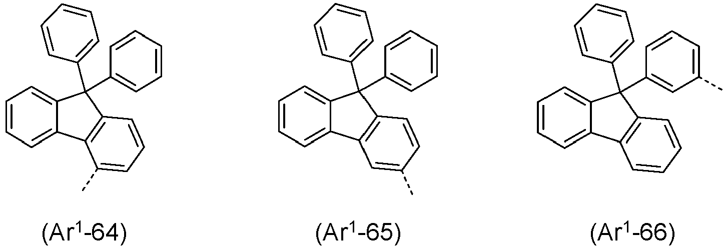

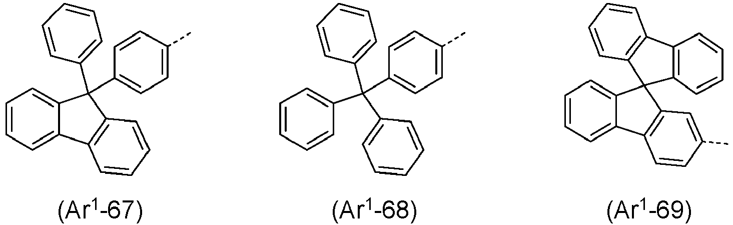

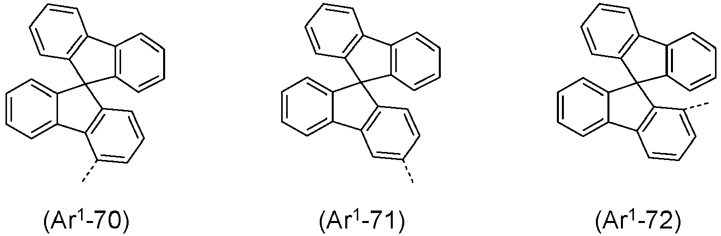

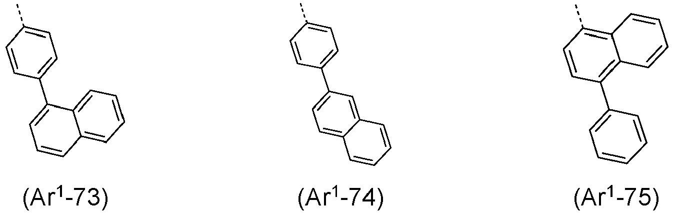

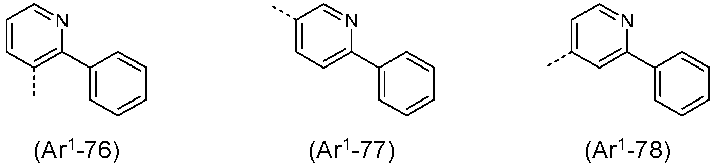

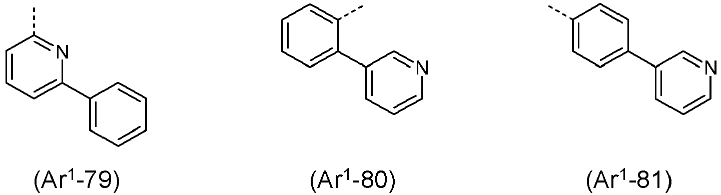

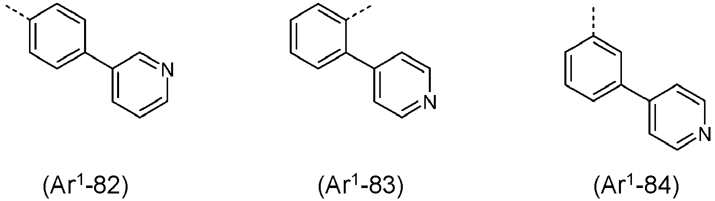

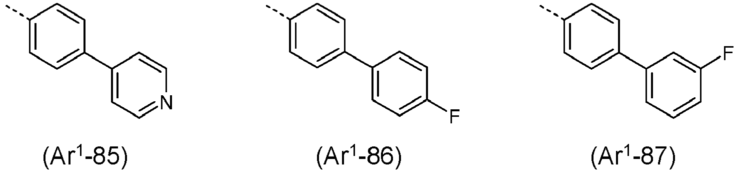

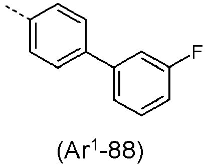

Erfindungsgemäß ist mindestens eine Gruppe Ar¹, bevorzugt sind alle Gruppen Ar¹ in den Verbindungen der Formeln (A-I) und (A-III) bei jedem Auftreten gleich oder verschieden gewählt ist aus den oben genannten Gruppen (Ar¹-1) bis (Ar¹-88), die jeweils an allen unsubstituiert dargestellten Positionen mit einem oder mehreren Resten R¹ substituiert sein können.

Es ist bevorzugt, dass in einem Ring der oben genannten Formeln (A-I) bis (A-IX) nicht mehr als drei Gruppen Z gleich N sind. Bevorzugt sind nicht mehr als 2 benachbarte Gruppen Z gleich N. Besonders bevorzugt ist Z gleich CR¹.

Es ist bevorzugt, dass X bei jedem Auftreten gleich oder verschieden eine Einfachbindung, O, S oder (CR¹)₂ ist.

Bevorzugt ist mindestens einer der Indices p und q gleich 1. Bevorzugt ist die Summe der Indices p und q gleich 1.

Bevorzugt ist Ar¹ in den oben genannten Formeln (A-I) bis (A-IX) gewählt aus den oben genannten bevorzugten Ausführungsformen von Ar¹.

Bevorzugt umfasst Ar² mindestens eine Gruppe gewählt aus Benzol, Naphthalin, Phenanthren, Fluoranthen, Biphenyl, Terphenyl, Quaterphenyl, Fluoren, Indenofluoren, Spirobifluoren, Furan, Benzofuran, Isobenzofuran, Dibenzofuran, Thiophen, Benzothiophen, Isobenzothiophen, Dibenzothiophen, Indol, Isoindol, Carbazol, Indolocarbazol, Indenocarbazol, Pyridin, Chinolin, Isochinolin, Acridin, Phenanthridin, Benzimidazol, Pyrimidin, Pyrazin, und Triazin, wobei die genannten Gruppen mit einem oder mehreren Resten R¹ substituiert sein können. Bevorzugt besteht Ar² ausschließlich aus einer der oben genannten Gruppen oder aus einer Kombination von mehreren der oben genannten Gruppen.

Bevorzugt ist Ar² bei jedem Auftreten gleich oder verschieden gewählt aus den folgenden Gruppen, die jeweils an allen unsubstituiert dargestellten Positionen mit einem oder mehreren Resten R¹ substituiert sein können:

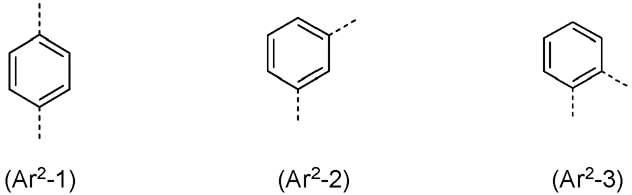

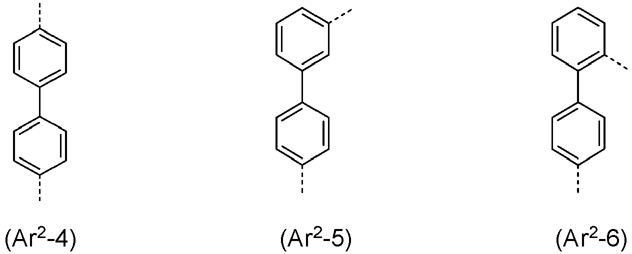

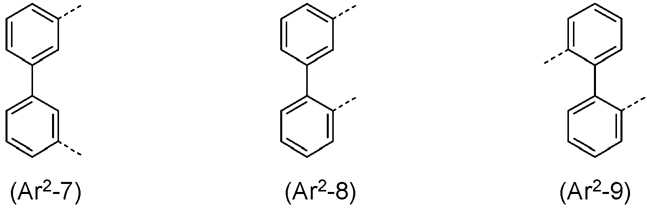

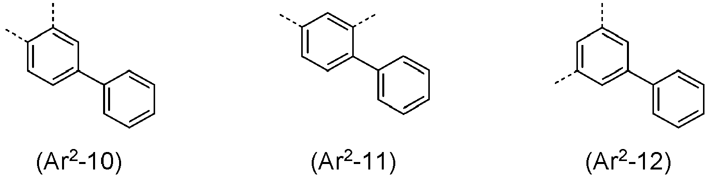

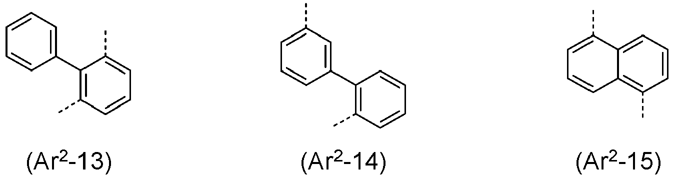

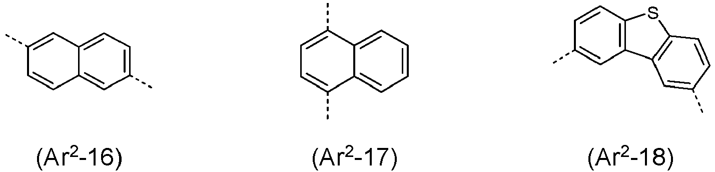

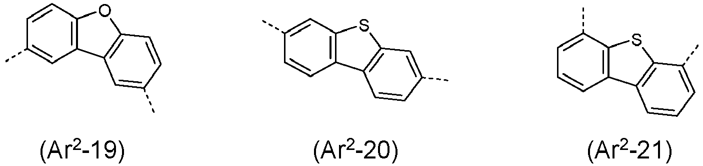

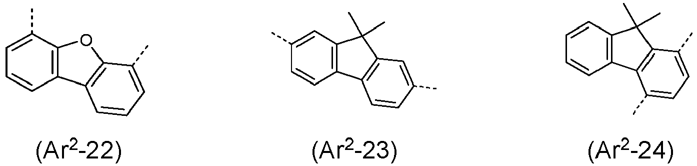

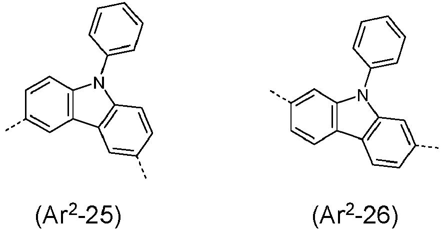

Bevorzugte Ausführungsformen von Verbindungen der Formeln (A-I) bis (A-IX) sind die folgenden Verbindungen:

| | | |
|---|---|---|
| 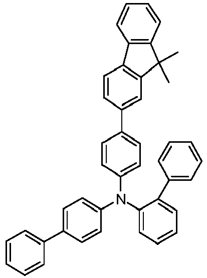 | 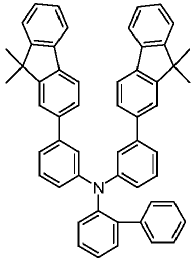 | 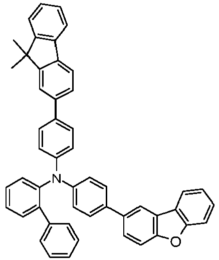 |
| 1 | 2 | 3 |
| 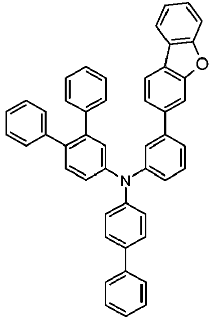 | 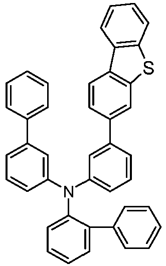 | 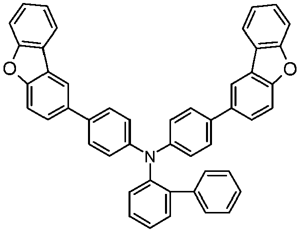 |
| 4 | 5 | 6 |
| 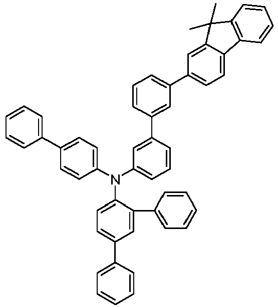 | 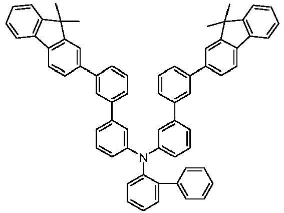 | 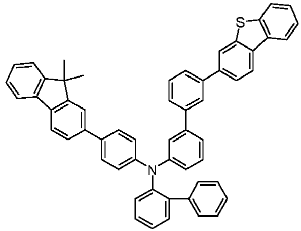 |
| 7 | 8 | 9 |
| 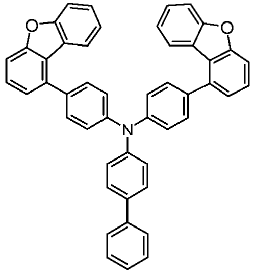 | 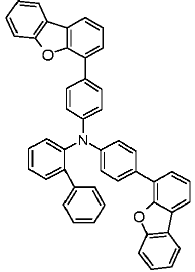 | 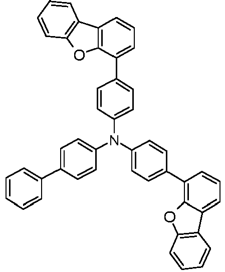 |
| 10 | 11 | 12 |
| 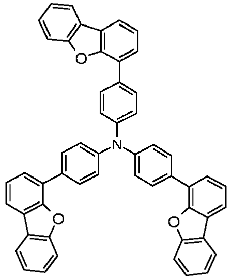 | 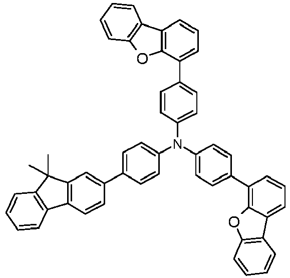 | 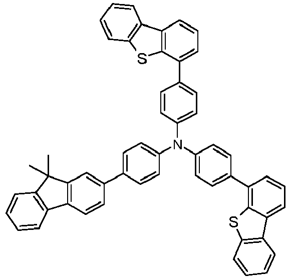 |
| 13 | 14 | 15 |
| 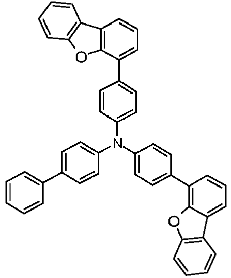 | 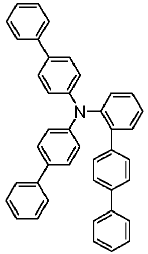 | 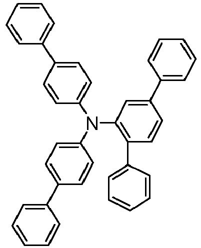 |
| 16 | 17 | 18 |
| 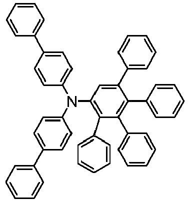 | 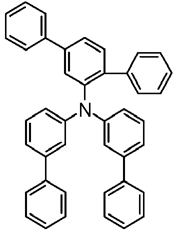 | 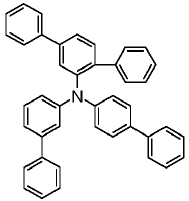 |
| 19 | 20 | 21 |
| 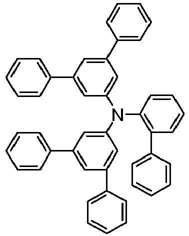 | 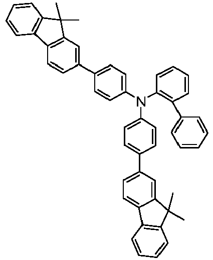 | 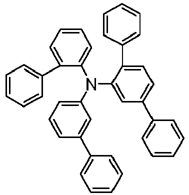 |
| 22 | 23 | 24 |
| 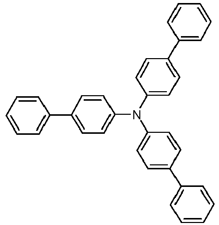 | 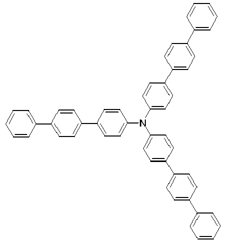 | 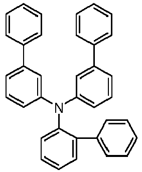 |
| 25 | 26 | 27 |
| 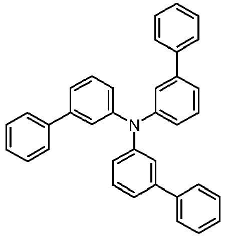 | 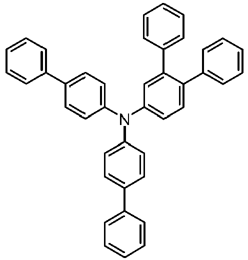 | 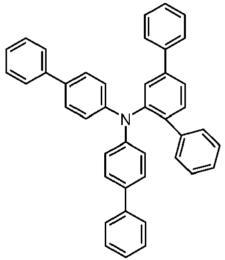 |
| 28 | 29 | 30 |
| 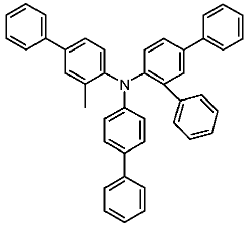 | 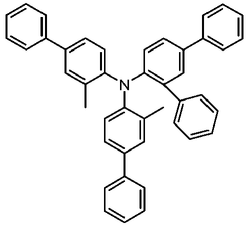 | 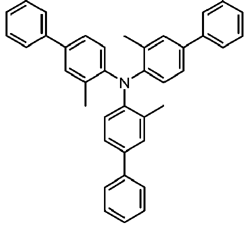 |
| 31 | 32 | 33 |
| 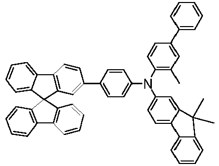 | 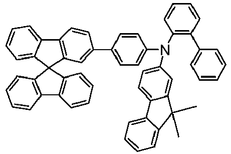 | 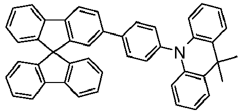 |
| 34 | 35 | 36 |
| 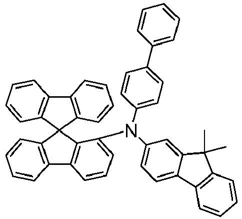 | 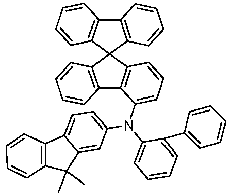 | 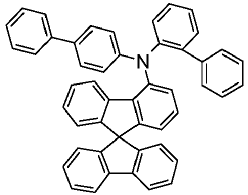 |
| 37 | 38 | 39 |
| 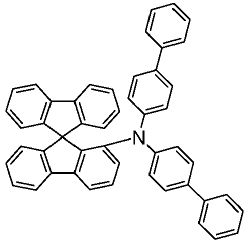 | 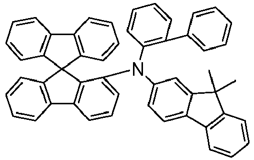 | 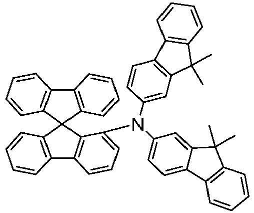 |
| 40 | 41 | 42 |
| 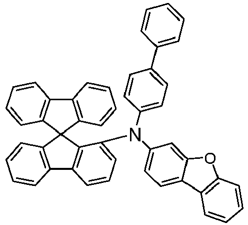 | 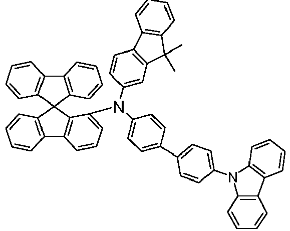 | 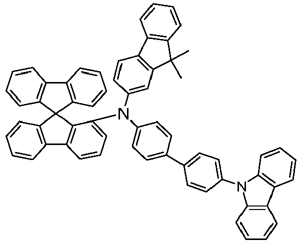 |
| 43 | 44 | 45 |
| 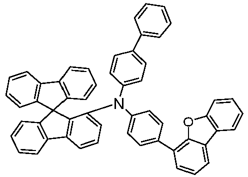 | 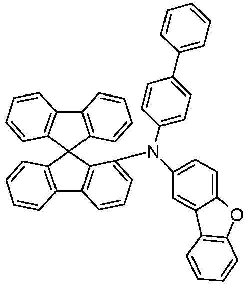 | 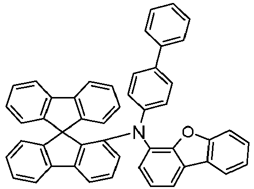 |
| 46 | 47 | 48 |
| 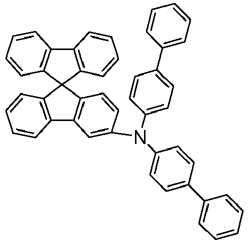 | 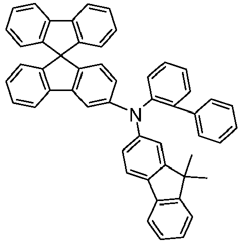 | 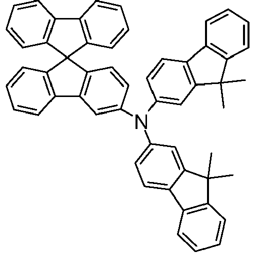 |
| 49 | 50 | 51 |
| 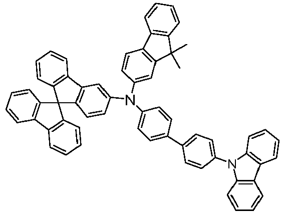 | 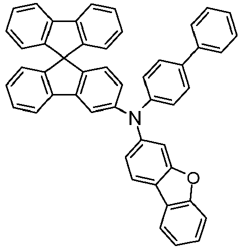 | 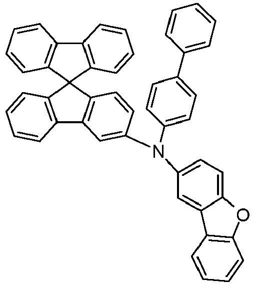 |
| 52 | 53 | 54 |
| 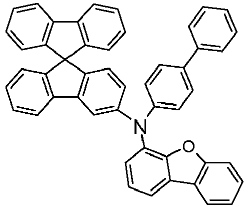 | 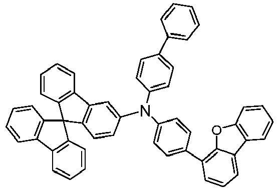 | 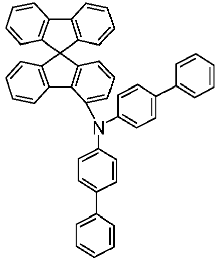 |
| 55 | 56 | 57 |
| 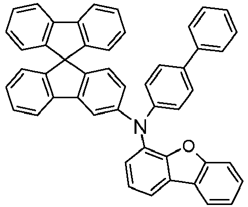 | 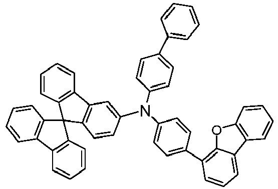 | 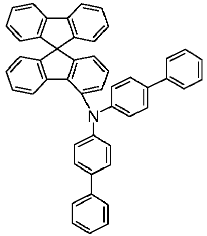 |
| 58 | 59 | 60 |
| 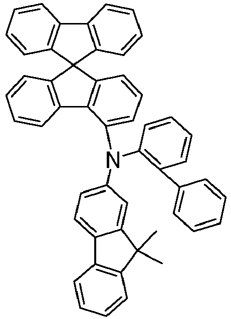 | 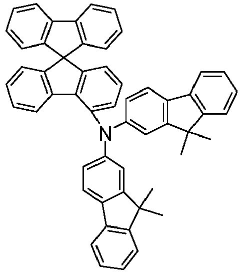 | 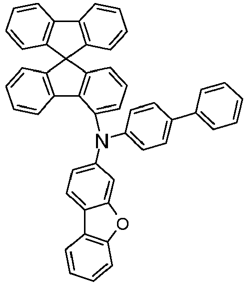 |
| 61 | 62 | 63 |
| 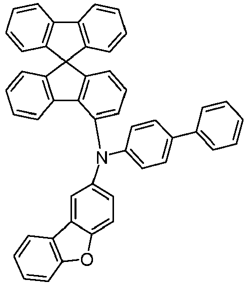 | 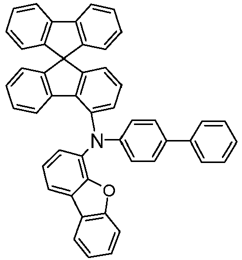 | 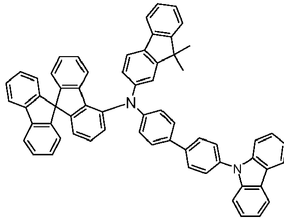 |
| 64 | 65 | 66 |
| 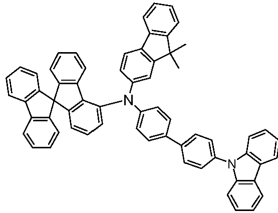 | 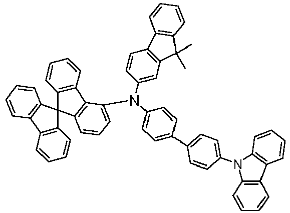 | 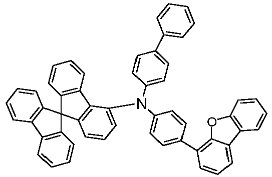 |
| 67 | 68 | 69 |
| 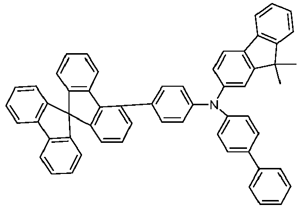 | | 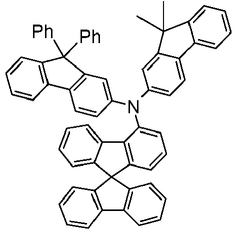 |
| 70 | | 71 |
| 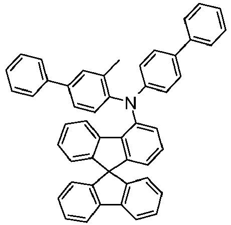 | 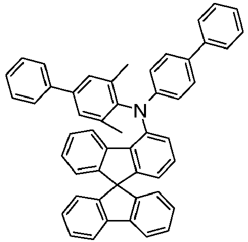 | |
| 72 | 73 | |
| 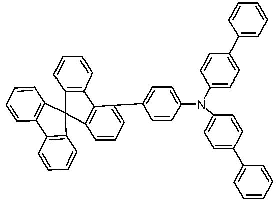 | 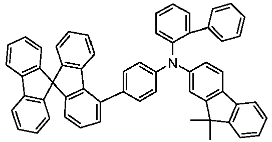 | 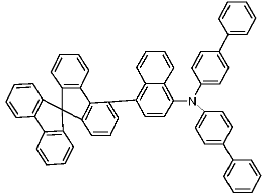 |
| 74 | 75 | 76 |
| 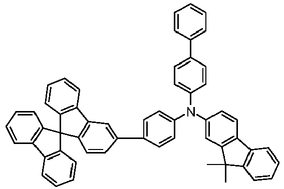 | 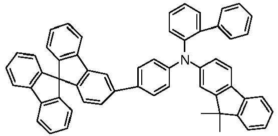 | 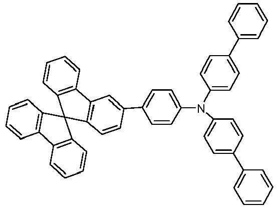 |
| 77 | 78 | 79 |
| 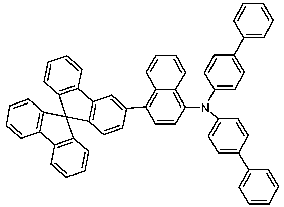 | 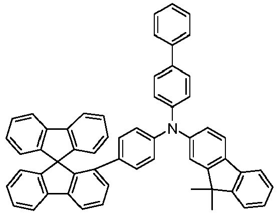 | 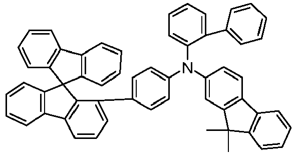 |
| 80 | 81 | 82 |
| 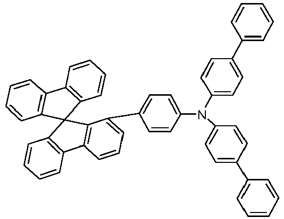 | 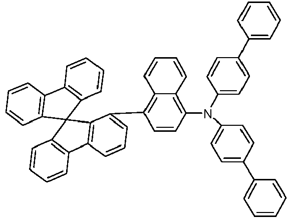 | 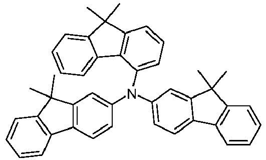 |
| 83 | 84 | 85 |
| 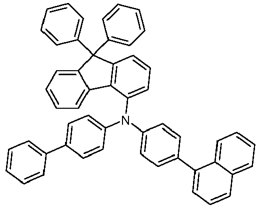 | 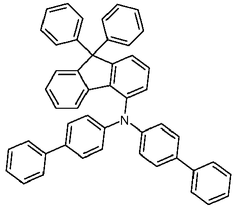 | 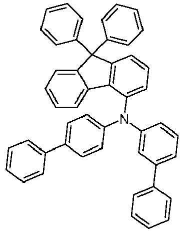 |
| 86 | 87 | 88 |
| 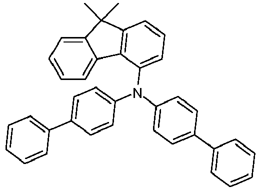 | 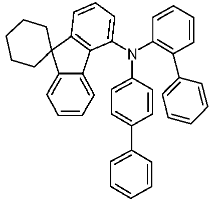 | 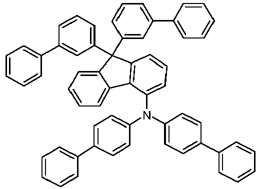 |
| 89 | 90 | 91 |
| 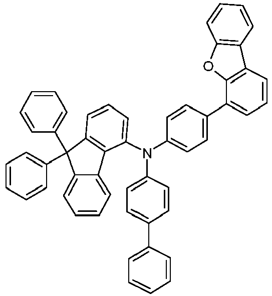 | 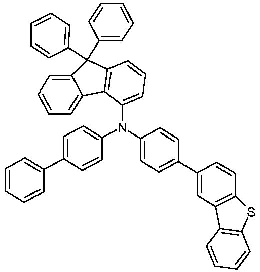 | 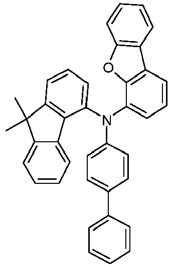 |
| 92 | 93 | 94 |
| 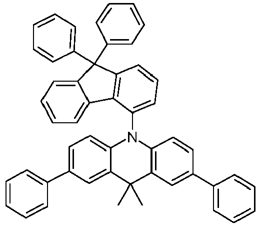 | 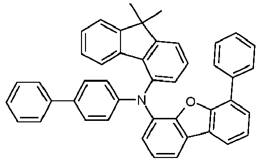 | 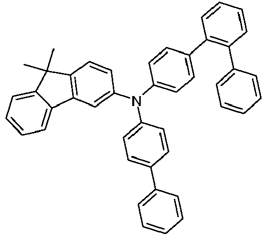 |
| 95 | 96 | 97 |
| 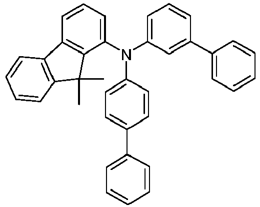 | 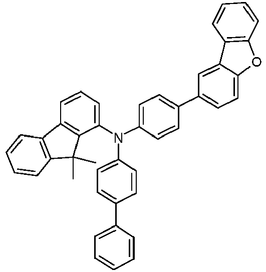 | 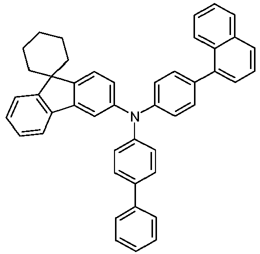 |
| 98 | 99 | 100 |
| 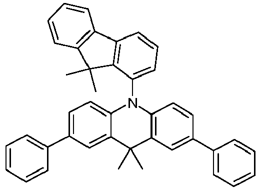 | 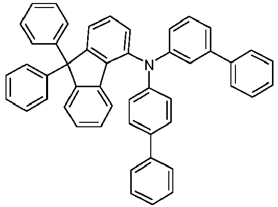 | 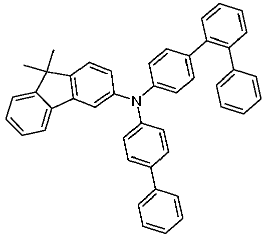 |
| 101 | 102 | 103 |
| 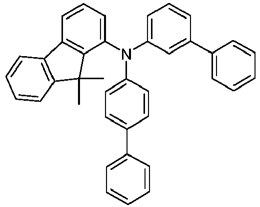 | 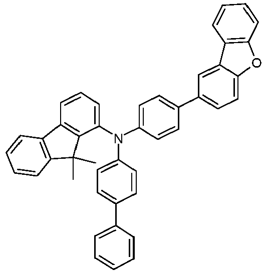 | 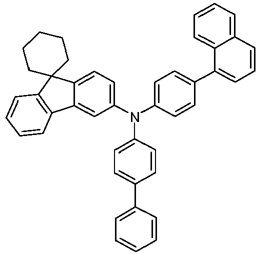 |
| 104 | 105 | 106 |
| 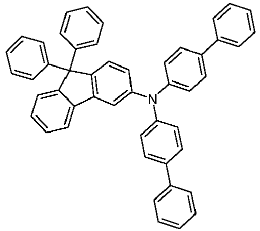 | 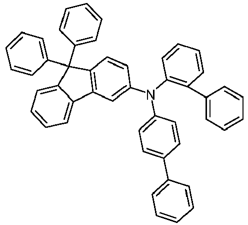 | 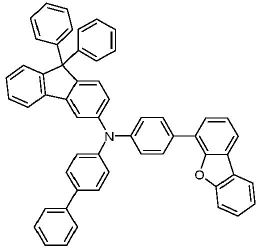 |
| 107 | 108 | 109 |
| 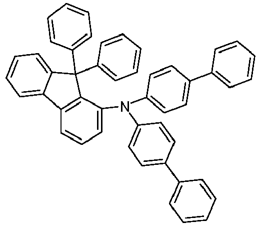 | 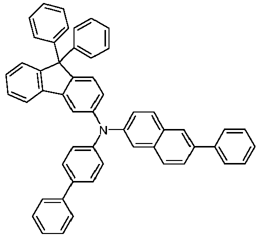 | 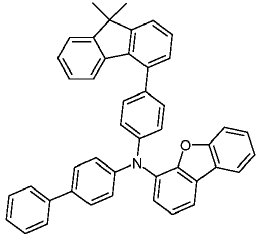 |
| 110 | 111 | 112 |
| 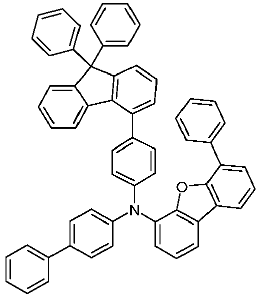 | 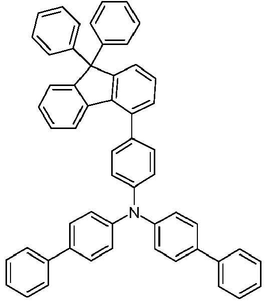 | 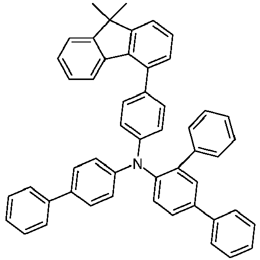 |
| 113 | 114 | 115 |
| 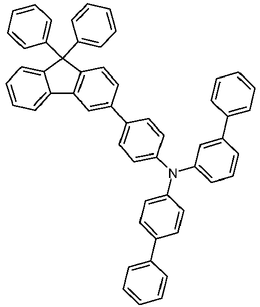 | 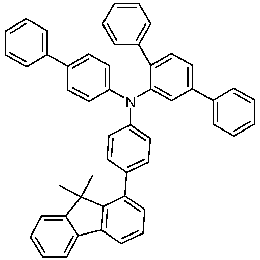 | 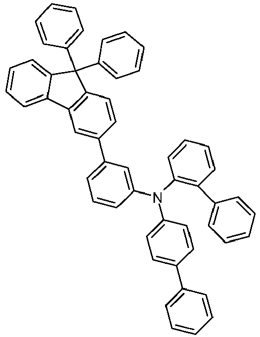 |
| 116 | 117 | 118 |
| 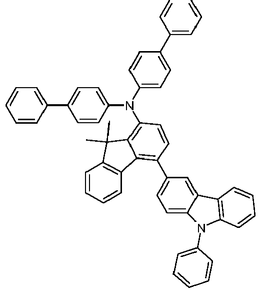 | 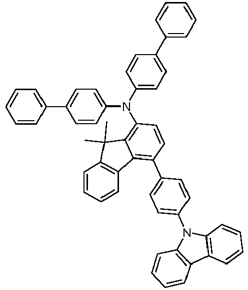 | 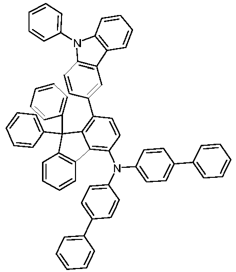 |
| 119 | 120 | 121 |
| 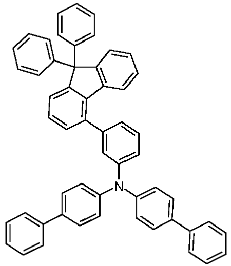 | 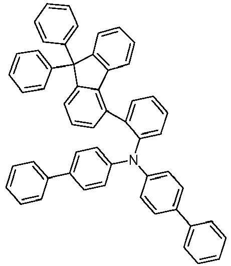 | 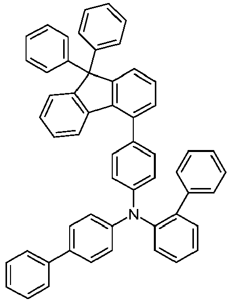 |
| 122 | 123 | 124 |
| 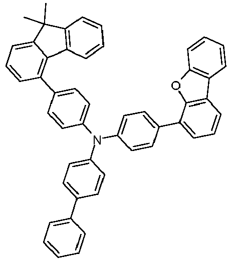 | 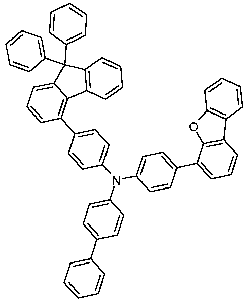 | 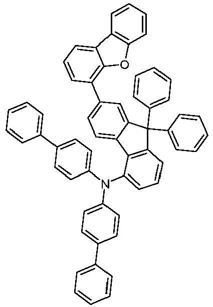 |
| 125 | 126 | 127 |
| 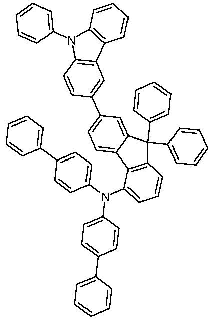 | 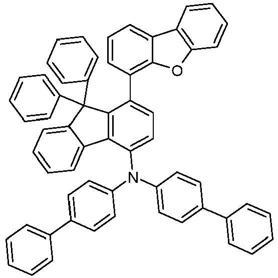 | 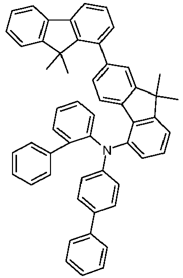 |
| 128 | 129 | 130 |
| 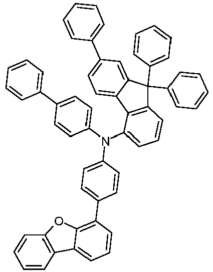 | 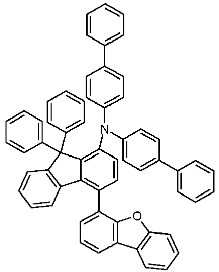 | 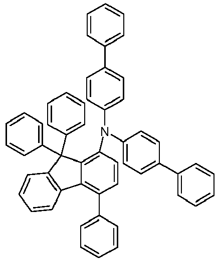 |
| 131 | 132 | 133 |
| 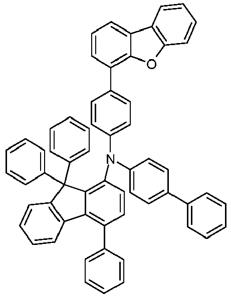 | 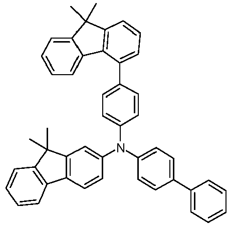 | 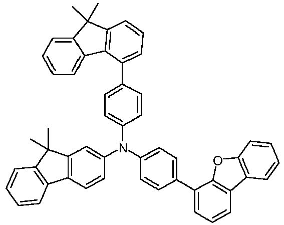 |
| 134 | 135 | 136 |
| 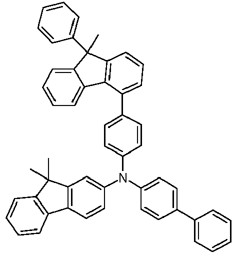 | 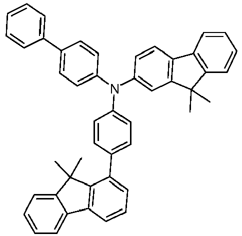 | 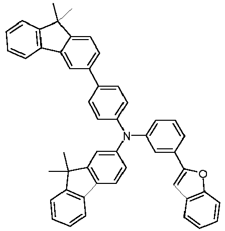 |
| 137 | 138 | 139 |
| 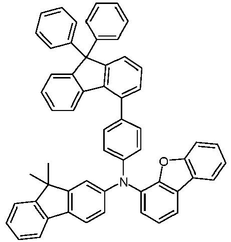 | 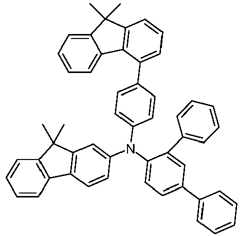 | 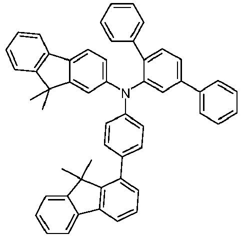 |
| 140 | 141 | 142 |
| 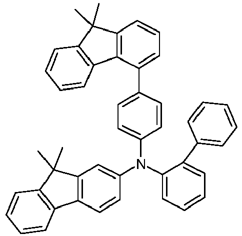 | 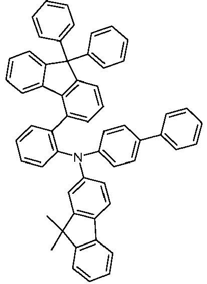 | 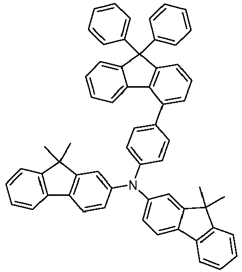 |
| 143 | 144 | 145 |
| 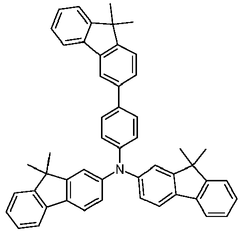 | 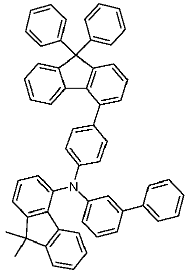 | 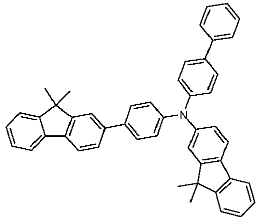 |
| 146 | 147 | 148 |
| 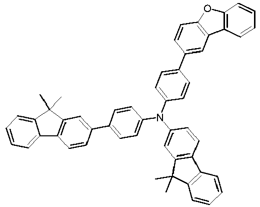 | 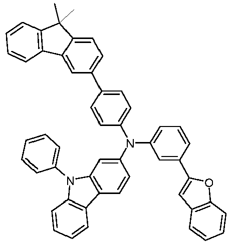 | 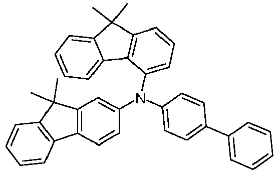 |
| 149 | 150 | 151 |
| 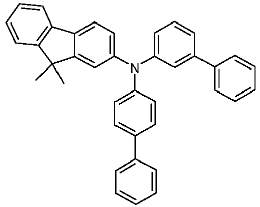 | 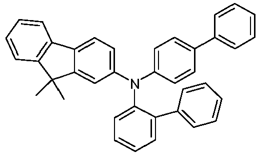 | 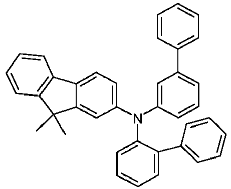 |
| 152 | 153 | 154 |
| 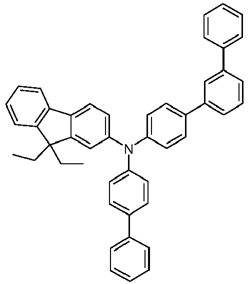 | 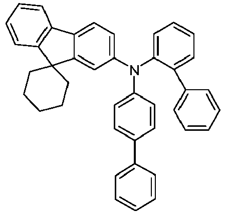 | 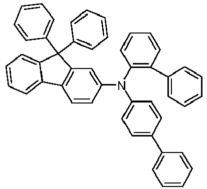 |
| 155 | 156 | 157 |
| 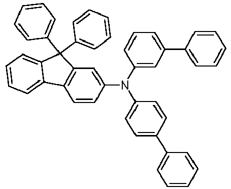 | 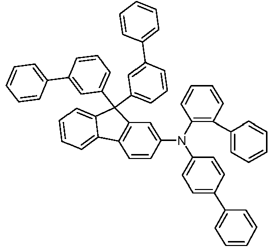 | 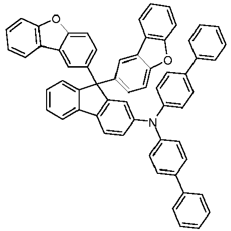 |
| 158 | 159 | 160 |
| 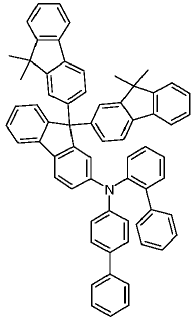 | 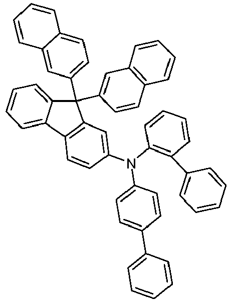 | 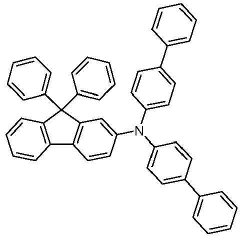 |
| 161 | 162 | 163 |
| 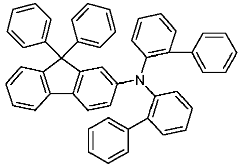 | 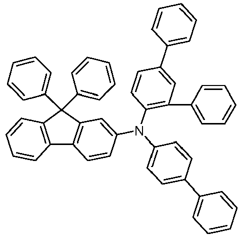 | 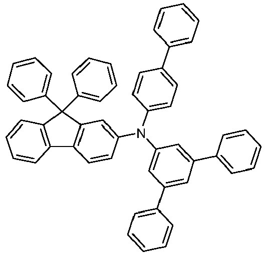 |
| 164 | 165 | 166 |
| 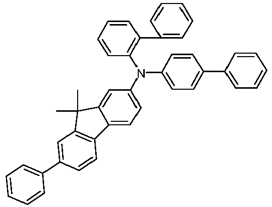 | 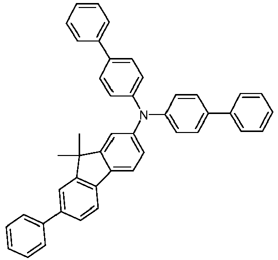 | 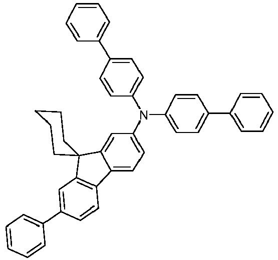 |
| 167 | 168 | 169 |
| 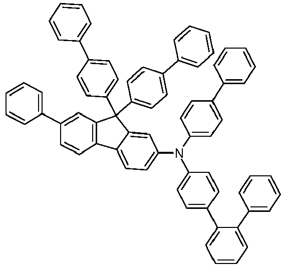 | 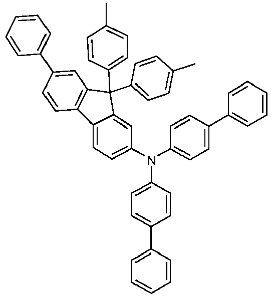 | 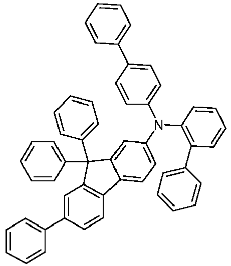 |
| 170 | 171 | 172 |
| 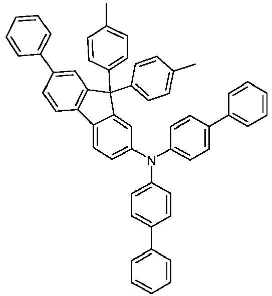 | 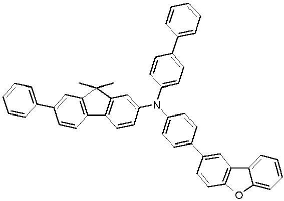 | 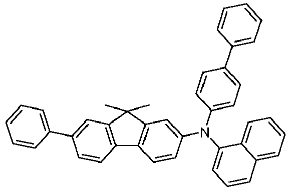 |
| 173 | 174 | 175 |
| 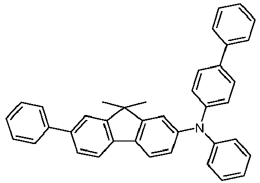 | 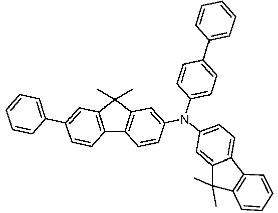 | 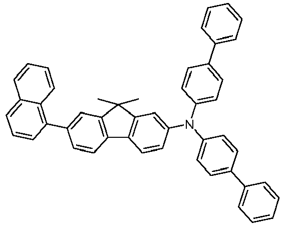 |
| 176 | 177 | 178 |
| 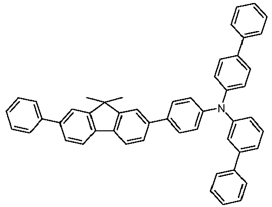 | 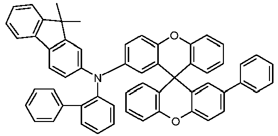 | 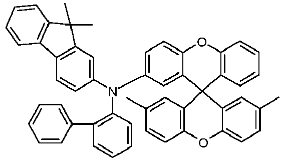 |
| 179 | 180 | 181 |
| 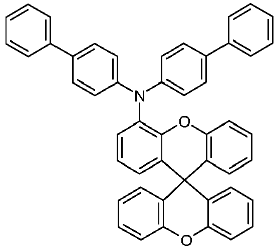 | 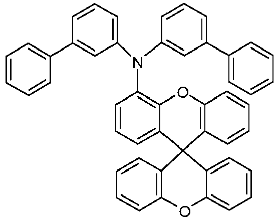 | 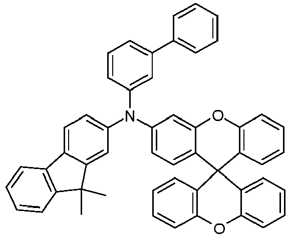 |
| 182 | 183 | 184 |
| 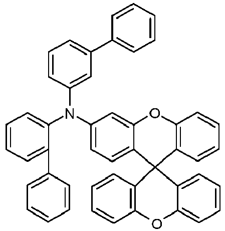 | 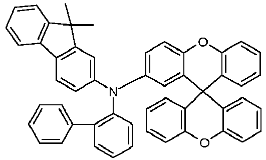 | 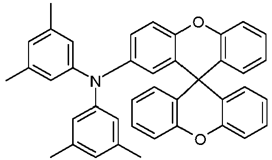 |
| 185 | 186 | 187 |
| 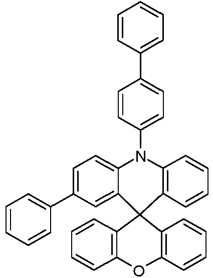 | 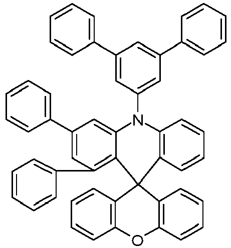 | 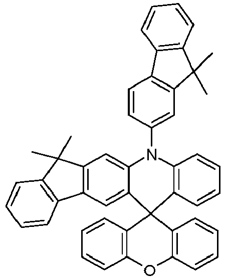 |
| 188 | 189 | 190 |
| 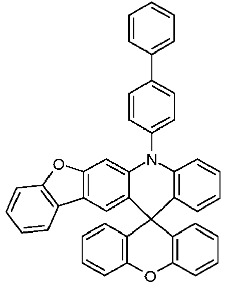 | 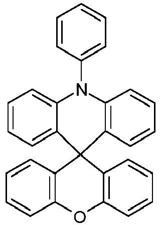 | 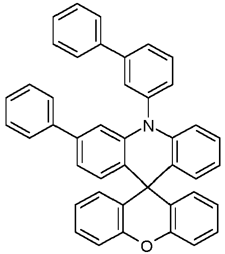 |
| 191 | 192 | 193 |
| 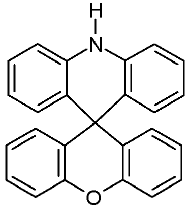 | 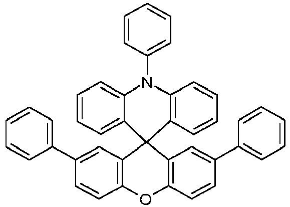 | 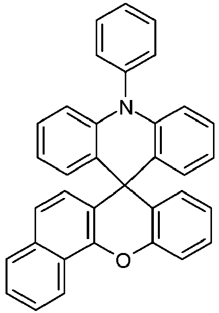 |
| 194 | 195 | 196 |
| 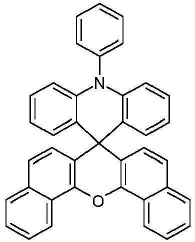 | 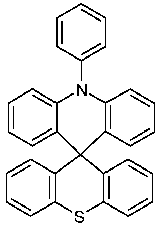 | 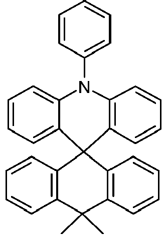 |
| 197 | 198 | 199 |
| 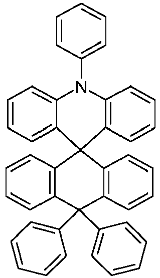 | 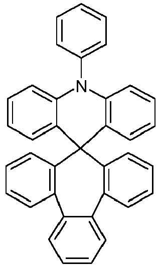 | 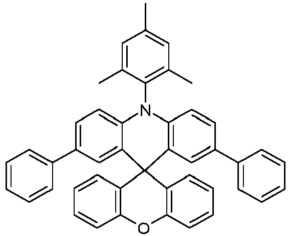 |
| 200 | 201 | 202 |
| 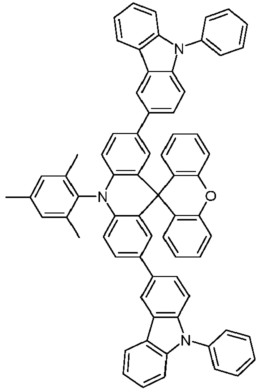 | 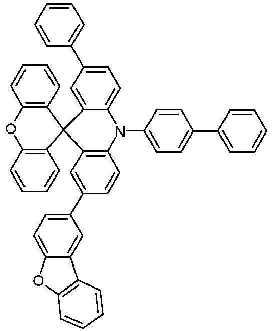 | 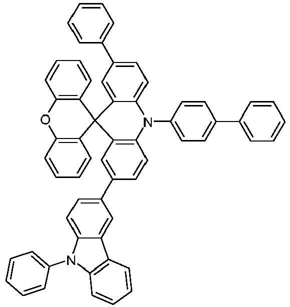 |
| 203 | 204 | 205 |
| 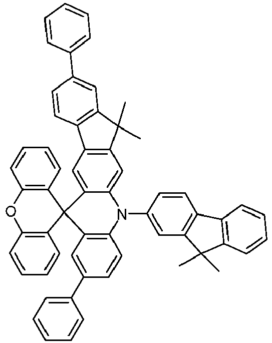 | 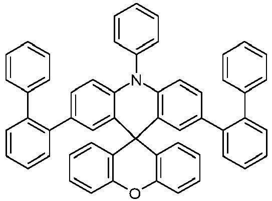 | 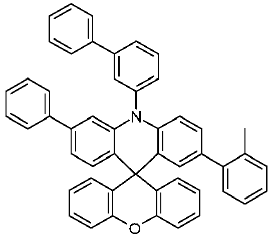 |
| 206 | 207 | 208 |
| 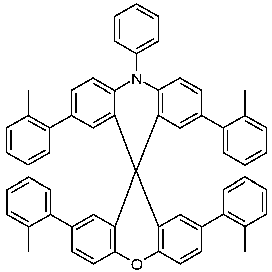 | 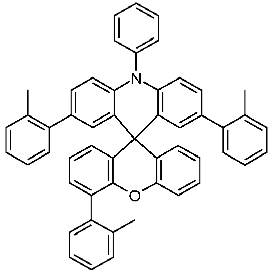 | 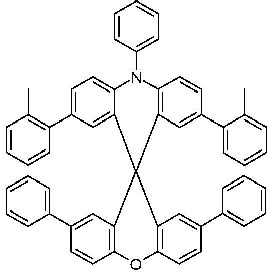 |
| 209 | 210 | 211 |
| 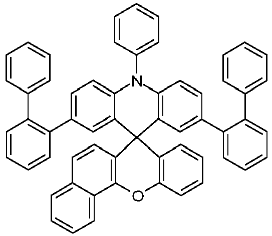 | 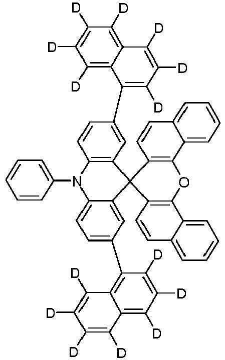 | 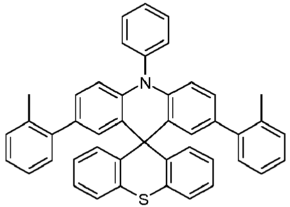 |
| 212 | 213 | 214 |
| 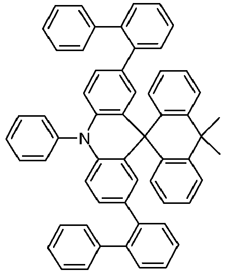 | 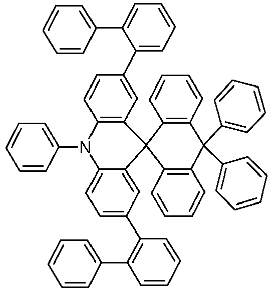 | 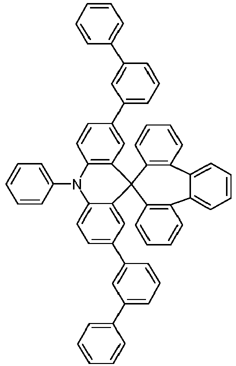 |
| 215 | 216 | 217 |
| 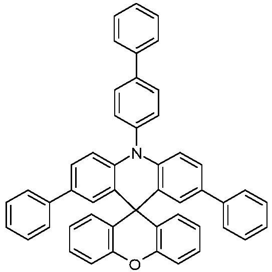 | 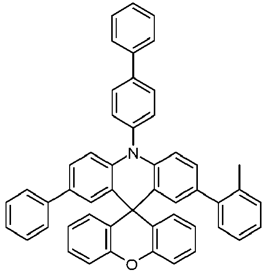 | 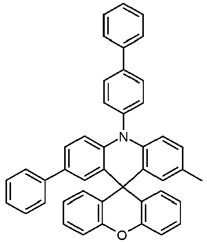 |
| 218 | 219 | 220 |
| 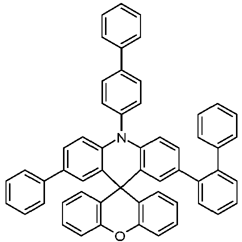 | 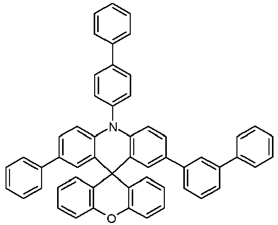 | 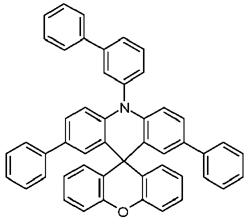 |
| 221 | 222 | 223 |
| 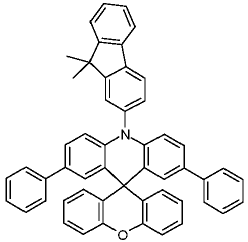 | 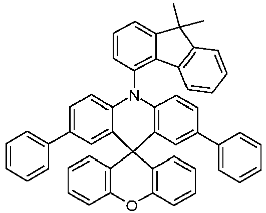 | |
| 224 | 225 | |
| 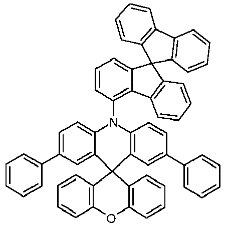 | 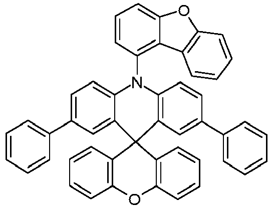 | 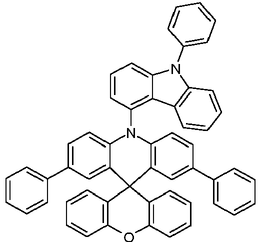 |
| 226 | 227 | 228 |
| 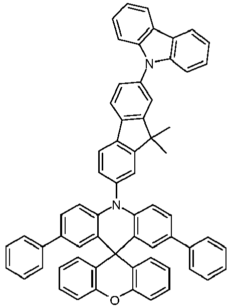 | 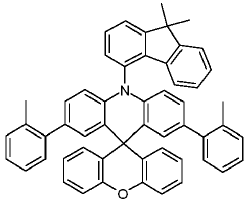 | 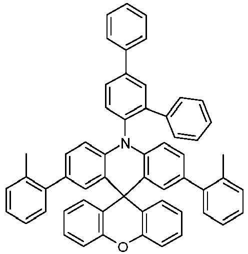 |
| 229 | 230 | 231 |
| 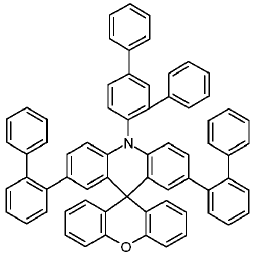 | 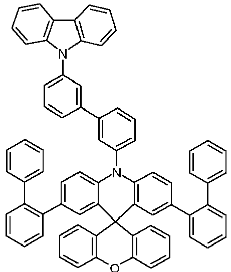 | 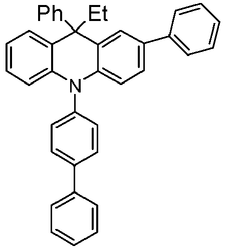 |
| 232 | 233 | 234 |
| 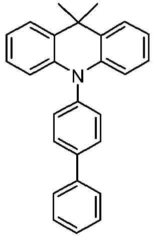 | 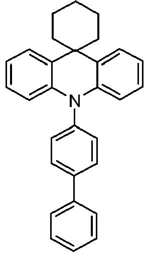 | 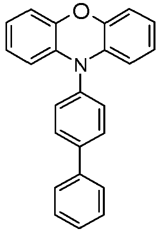 |
| 235 | 236 | 237 |
| 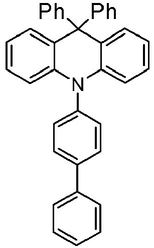 | 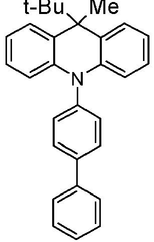 | 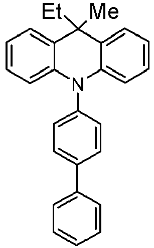 |
| 238 | 239 | 240 |
| 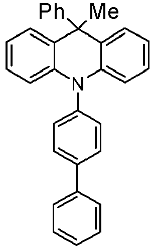 | 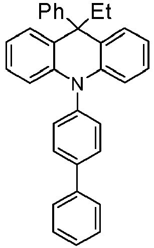 | 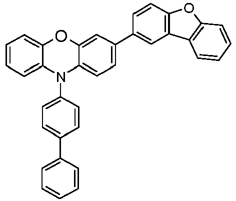 |
| 241 | 242 | 243 |
| 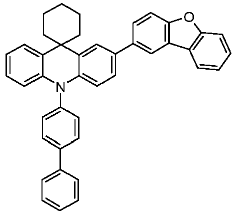 | 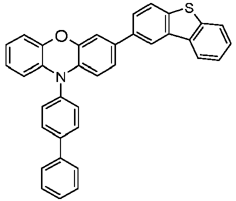 | 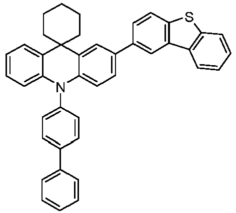 |
| 244 | 245 | 246 |
| 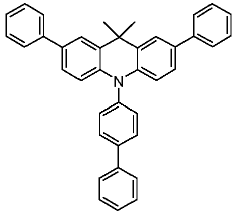 | 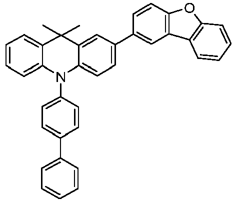 | 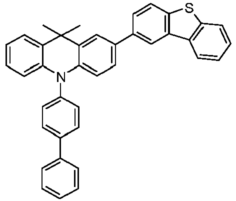 |
| 247 | 248 | 249 |
| 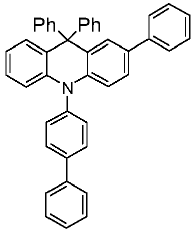 | 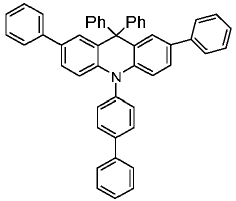 | 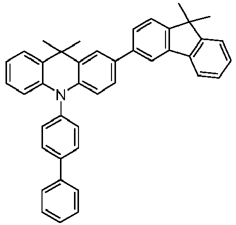 |
| 250 | 251 | 252 |
| 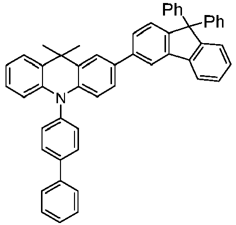 | 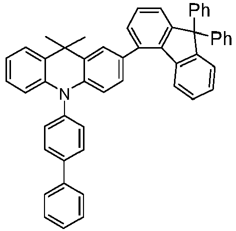 | 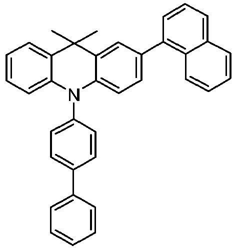 |
| 253 | 254 | 255 |
| 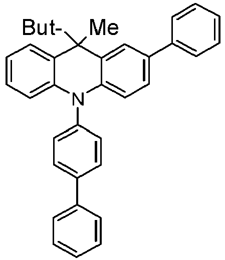 | 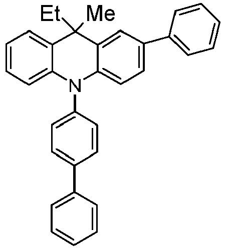 | 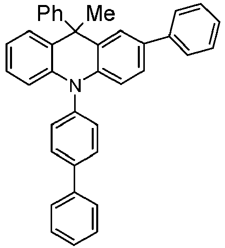 |
| 256 | 257 | 258 |
| 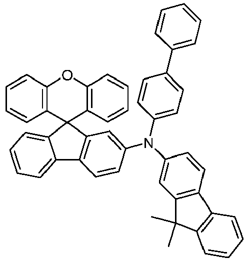 | 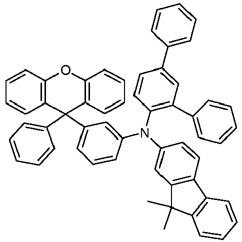 | 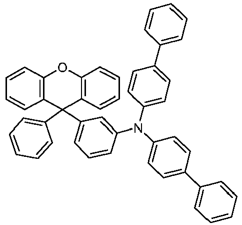 |
| 259 | 260 | 261 |
| 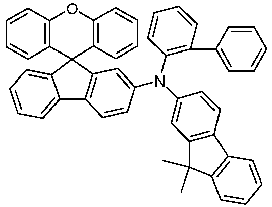 | 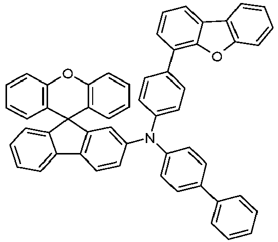 | 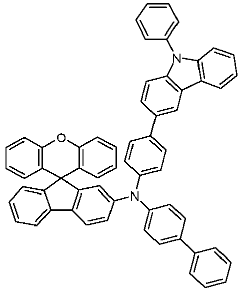 |
| 262 | 263 | 264 |
| 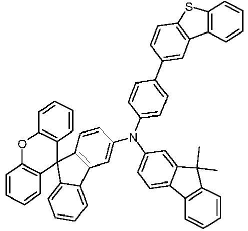 | 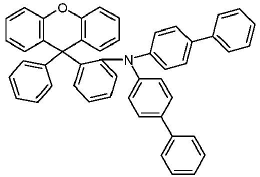 | 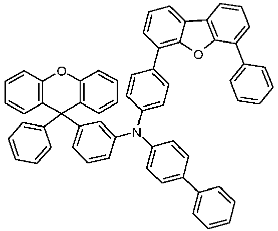 |
| 265 | 266 | 267 |
| 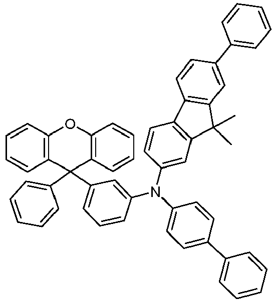 | 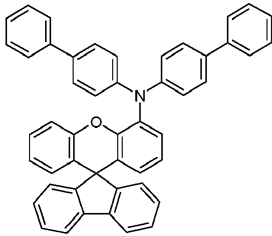 | 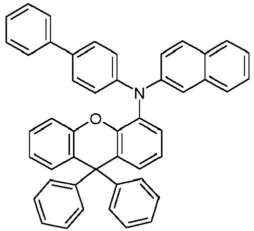 |
| 268 | 269 | 270 |
| 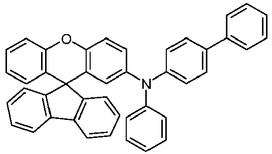 | 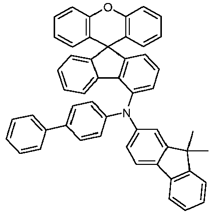 | 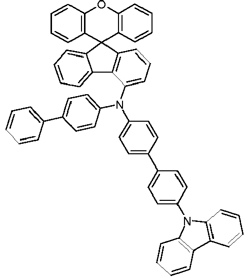 |
| 271 | 272 | 273 |
| 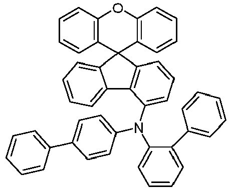 | 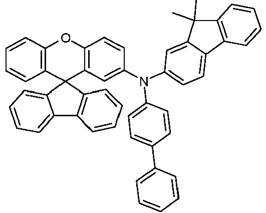 | 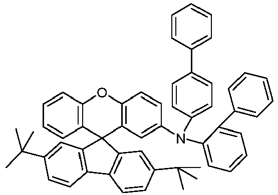 |
| 274 | 275 | 276 |
| 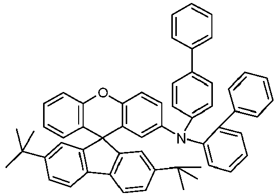 | 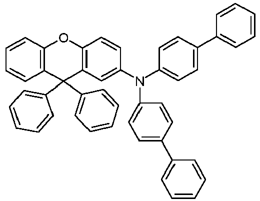 | 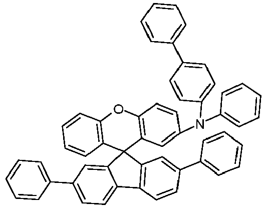 |
| 277 | 278 | 279 |
| 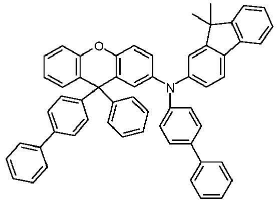 | 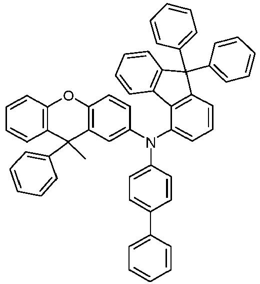 | |
| 280 | 281 | |
| 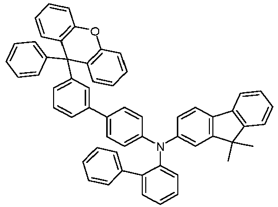 | 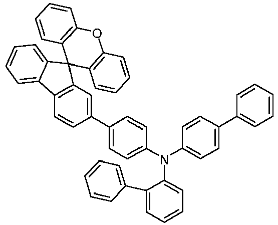 | 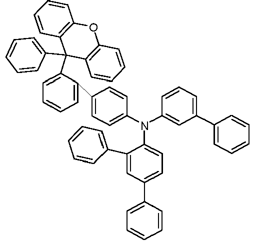 |
| 282 | 283 | 284 |
| 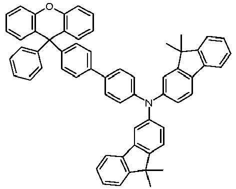 | 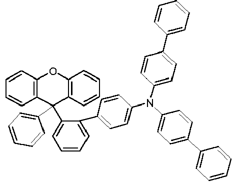 | 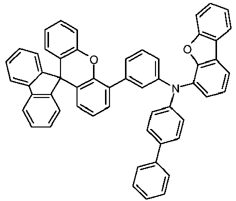 |
| 285 | 286 | 287 |
| 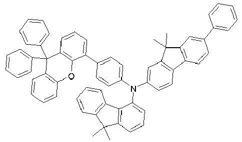 | 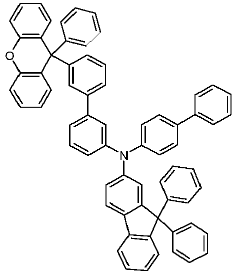 | 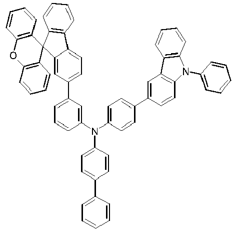 |
| 288 | 289 | 290 |
| 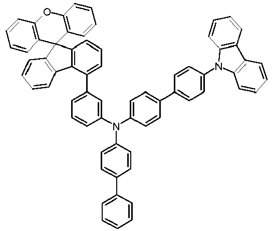 | 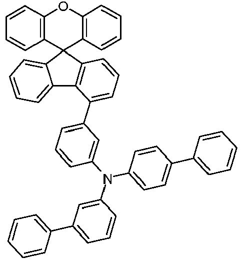 | 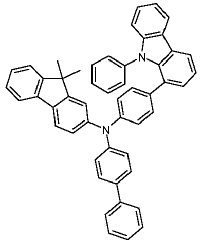 |
| 291 | 292 | 293 |
| 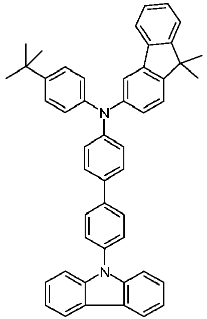 | 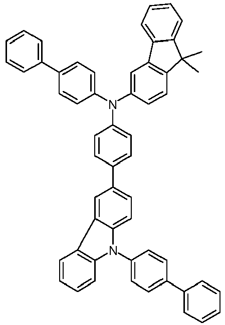 | 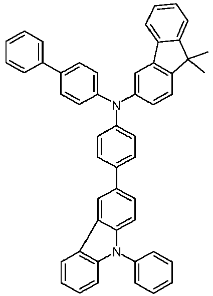 |
| 294 | 295 | 296 |
| 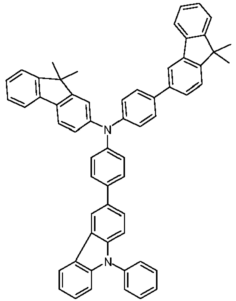 | 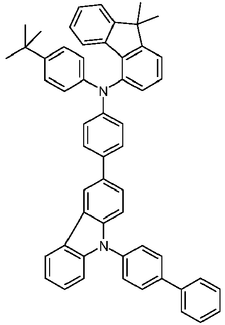 | 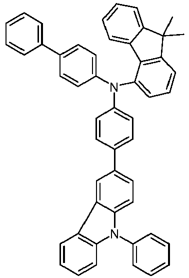 |
| 297 | 298 | 299 |
| 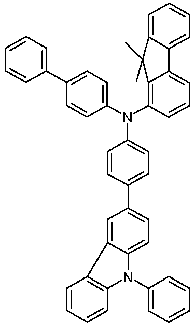 | 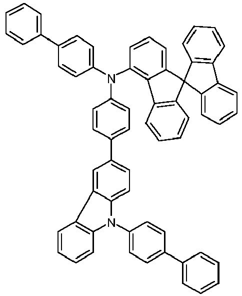 | 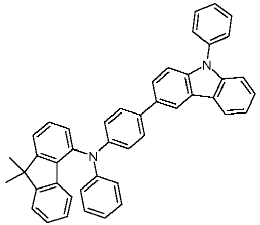 |
| 300 | 301 | 302 |
| 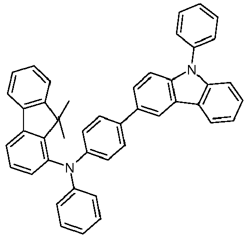 | 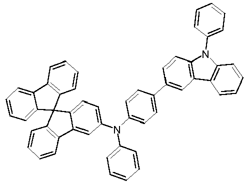 | 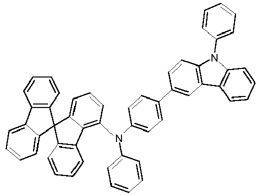 |
| 303 | 304 | 305 |
| 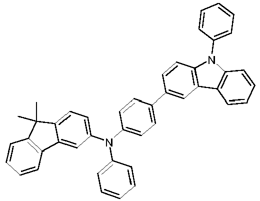 | 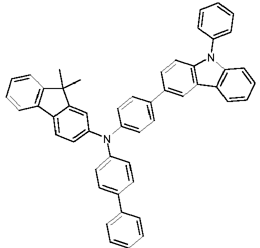 | 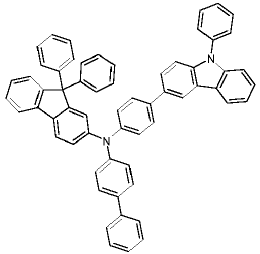 |
| 306 | 307 | 308 |
| 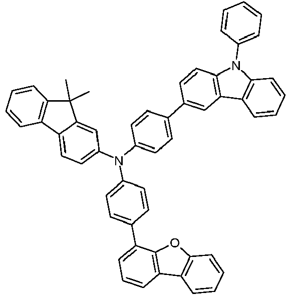 | | 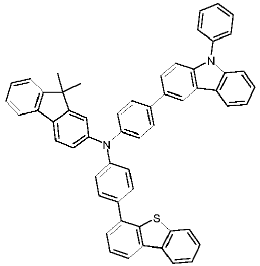 |
| 309 | | 310 |
| 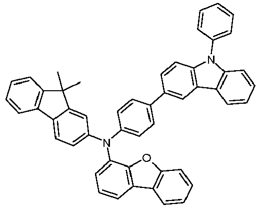 | 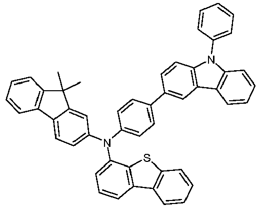 | 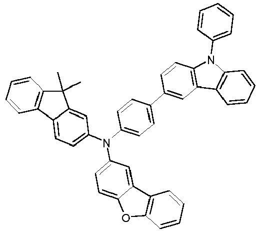 |
| 311 | 312 | 313 |
| 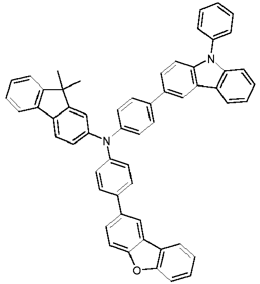 | 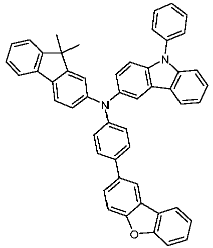 | 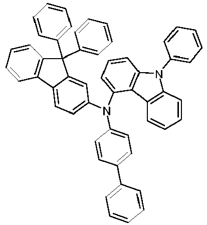 |
| 314 | 315 | 316 |
| 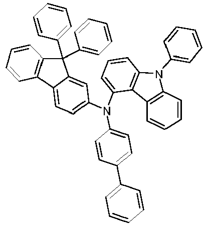 | 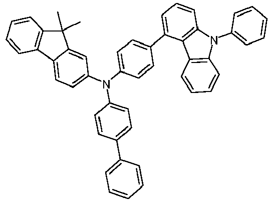 | 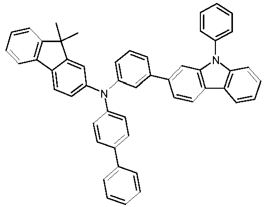 |
| 317 | 318 | 319 |
| 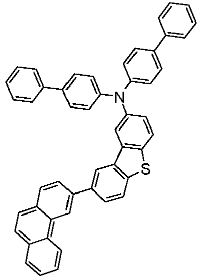 | 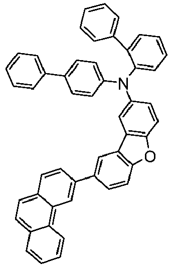 | 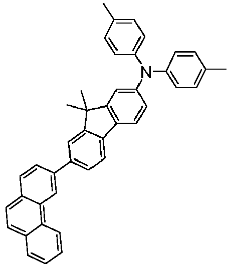 |
| 320 | 321 | 322 |
| 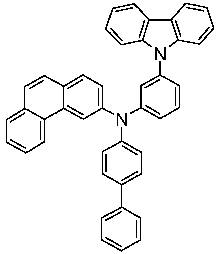 | 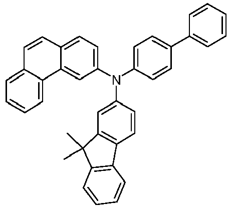 | 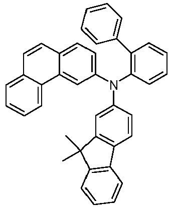 |
| 323 | 324 | 325 |
| 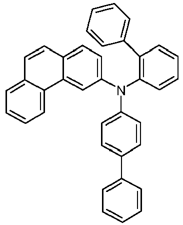 | 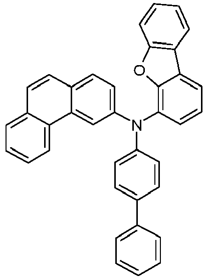 | 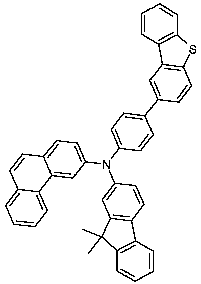 |
| 326 | 327 | 328 |
| 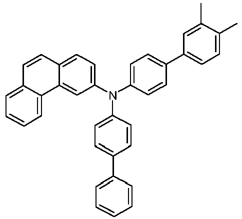 | 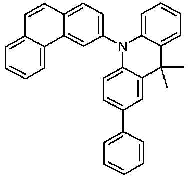 | 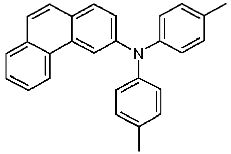 |
| 329 | 330 | 331 |
| 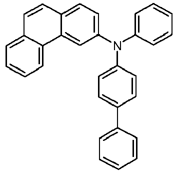 | 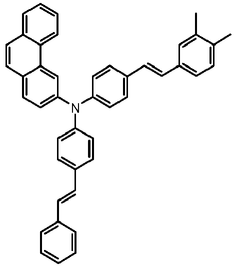 | 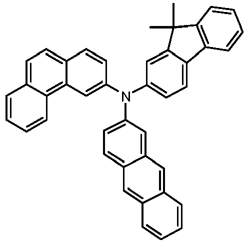 |
| 332 | 333 | 334 |
| 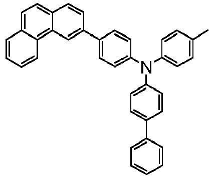 | 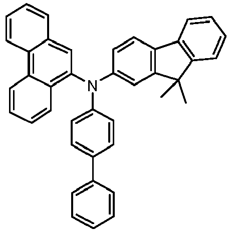 | 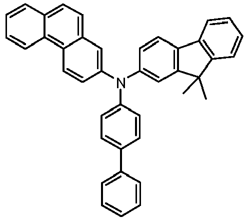 |
| 335 | 336 | 337 |
| 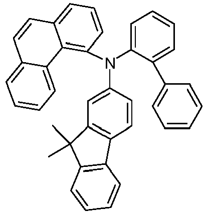 | 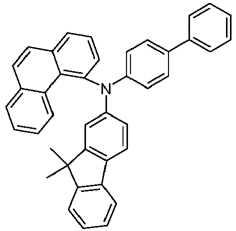 | 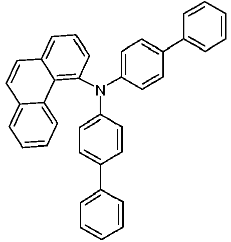 |
| 338 | 339 | 340 |
| 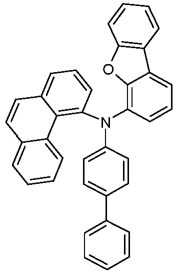 | 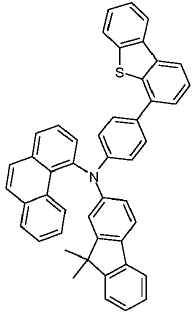 | 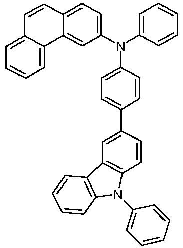 |
| 341 | 342 | 343 |
| 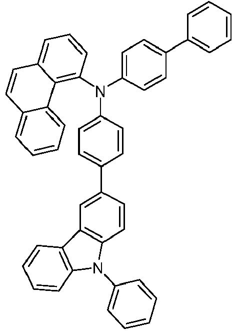 | 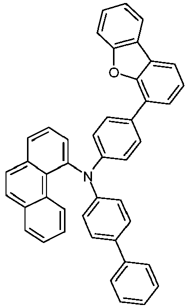 | 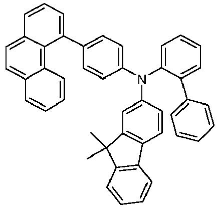 |
| 344 | 345 | 346 |
| 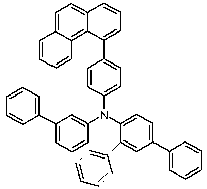 | 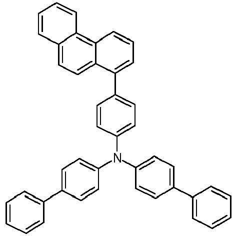 | 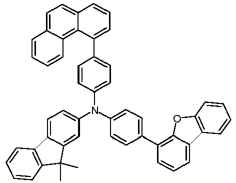 |
| 347 | 348 | 349 |
| 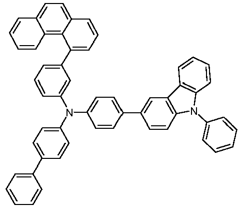 | 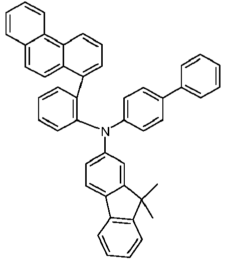 | 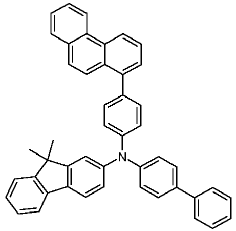 |
| 350 | 351 | 352 |
| 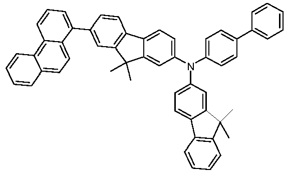 | 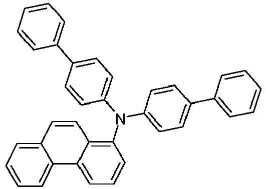 | 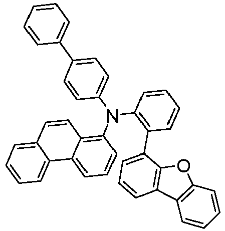 |
| 353 | 354 | 355 |
| 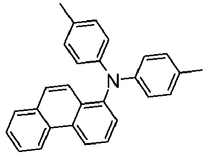 | 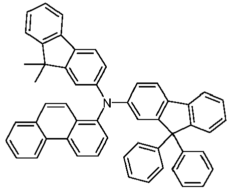 | 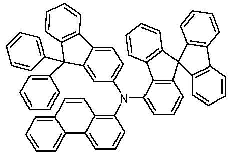 |
| 356 | 357 | 358 |
| 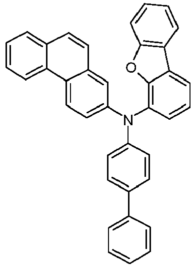 | 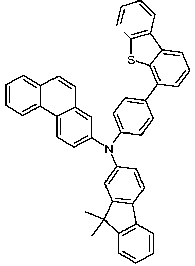 | 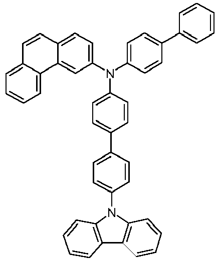 |
| 359 | 360 | 361 |
| 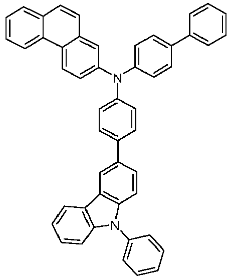 | 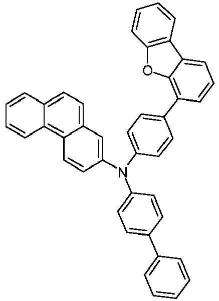 | 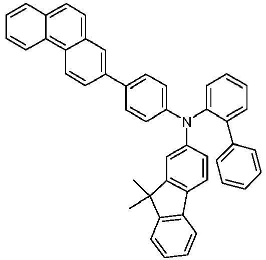 |
| 362 | 363 | 364 |
| 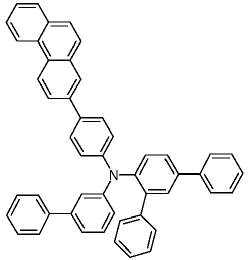 | 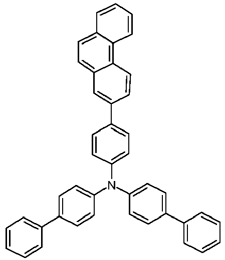 | 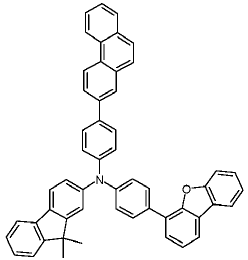 |
| 365 | 366 | 367 |
| 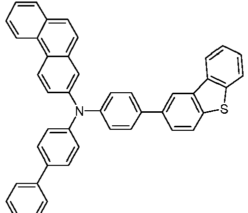 | 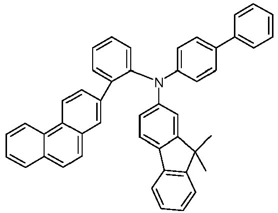 | 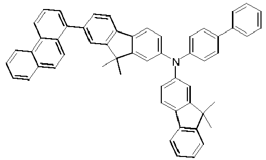 |
| 368 | 369 | 370 |
| 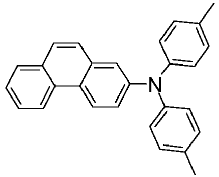 | 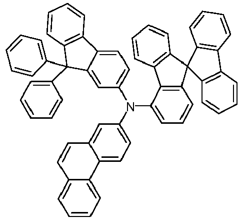 | 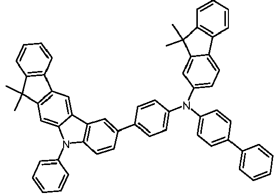 |
| 371 | 372 | 373 |
| 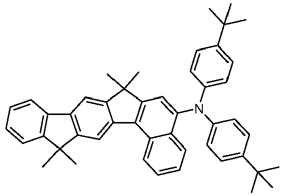 | 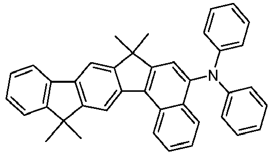 | 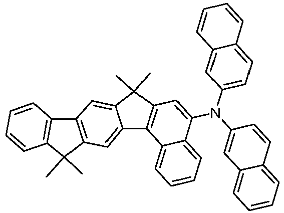 |
| 374 | 375 | 376 |
| 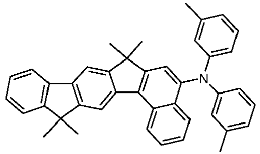 | 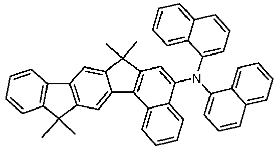 | 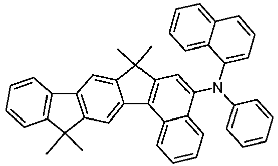 |
| 377 | 378 | 379 |
| 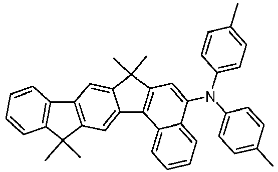 | 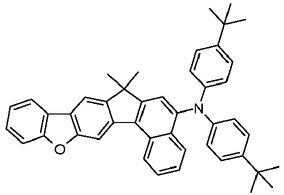 | 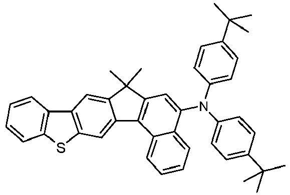 |
| 380 | 381 | 382 |
| 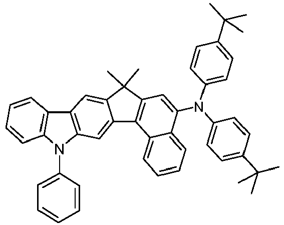 | 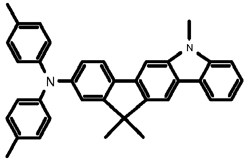 | 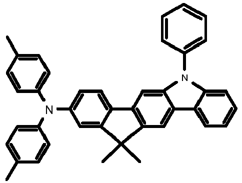 |
| 383 | 384 | 385 |
| 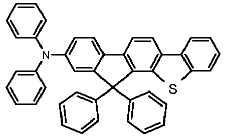 | 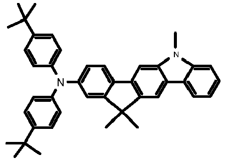 | 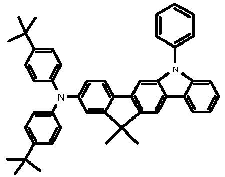 |
| 386 | 387 | 388 |
| 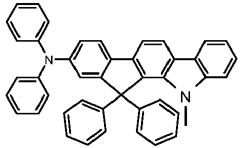 | 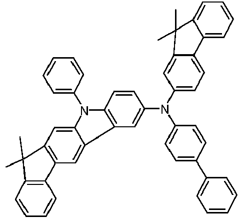 | 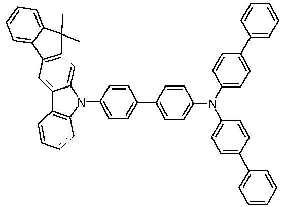 |
| 389 | 390 | 391 |
| 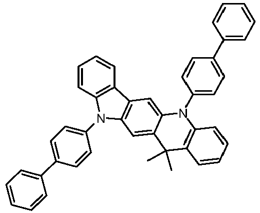 | | |
| 392 | | |

Herstellungsverfahren für die verschiedenen Ausführungsformen der Verbindung A sind im Stand der Technik bekannt. Unter anderem kann der Fachmann auf die Offenbarung der Dokumente WO 2006/122630, WO 2006/100896), EP 1661888, WO 2012/034627, WO 2013/120577, WO 2014/015937, WO 2014/015938, WO 2014/015935, WO 2013/083216 und WO 2012/150001 zurückgreifen.

Die Verbindung P ist ein p-Dotand im Sinne der oben genannten Definition. Ohne an diese Theorie gebunden zu sein, wird angenommen, dass die Verbindung P eine Lewis-Säure ist, die, wenn sie mit der Verbindung A gemischt vorliegt, einen Komplex mit der Verbindung A bildet. Die Verbindung A wirkt dabei als Lewis-Base. Der Komplex wird dabei, ohne an diese Theorie gebunden zu sein, dadurch gebildet, dass ein freies Elektronenpaar der Verbindung A in Wechselwirkung mit dem Metallatom Bismut der Verbindung P tritt.

Die Verbindung P kann ein mononuklearer Komplex von Bismut sein, ein binuklearer Komplex von Bismut oder ein polynuklearer Komplex von Bismut. Dabei ist es möglich, dass die Verbindung P ein mononuklearer Komplex von Bismut ist, sofern sie in der Gasphase vorliegt, und ein polynuklearer Komplex von Bismut ist, wenn sie in der Festkörperphase vorliegt. Das heißt, die Verbindung P kann, je nach Aggregatzustand, polymerisieren oder depolymerisieren.

Der Komplex von Bismut ist bevorzugt ein Komplex von Bismut in der Oxidationsstufe (II), (III) oder (V). Besonders bevorzugt ist er ein Komplex von Bismut in der Oxidationsstufe (III).

Bevorzugt weist der Komplex von Bismut mindestens einen Liganden L auf, der eine organische Verbindung ist. Der Ligand L ist bevorzugt gewählt aus einzähnigen, zweizähnigen und dreizähnigen Liganden, besonders bevorzugt aus einzähnigen Liganden. Weiterhin bevorzugt ist der Ligand L negativ geladen, bevorzugt dreifach, zweifach oder einfach negativ geladen, besonders bevorzugt einfach negativ geladen.

Die an das Bismutatom bindende Gruppe des Liganden L ist bevorzugt gewählt aus Carbonsäuregruppen, Thiocarbonsäuregruppen, im Besonderen Thiolsäuregruppen, Thionsäuregruppen und Dithiolsäuregruppen, Carbonsäureamidgruppen und Carbonsäureimidgruppen, besonders bevorzugt aus Carbonsäuregruppen.

Bevorzugt entspricht der Ligand L einer der folgenden Formeln (L-I), (L-II), (L-III) und (L-IV)

| | |
|---|---|
| 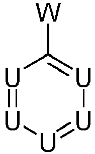 | 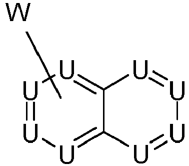 |
| Formel (L-I) | Formel (L-II) |
| 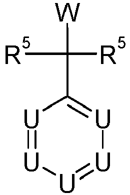 | 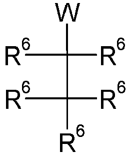 |
| Formel (L-III) | Formel (L-IV) |

wobei gilt:
W ist gewählt aus Carbonsäuregruppen, Thiocarbonsäuregruppen, im Besonderen Thiolsäuregruppen, Thionsäuregruppen und Dithiolsäuregruppen, Carbonsäureamidgruppen und Carbonsäureimidgruppen, besonders bevorzugt aus Carbonsäuregruppen;
U ist bei jedem Auftreten gleich oder verschieden gewählt ausN und CR³, wenn an es keine Gruppe W gebunden ist, und U ist gleich C, wenn an es eine Gruppe W gebunden ist; und
R³ ist bei jedem Auftreten gleich oder verschieden gewählt aus H, D, F, Cl, Br, I, CN, NO₂, CF₃, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)₂, C=O, C=NR⁴, -C(=O)O-, -C(=O)NR⁴-, P(=O)(R⁴), -O-, -S-, SO oder SO₂ ersetzt sein können; und
R⁴ ist bei jedem Auftreten gleich oder verschieden gewählt aus H, D, F, Cl, CN, NO₂, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkylgruppen, aromatischen Ringsysteme und heteroaromatischen Ringsysteme mit F, Cl, CN und NO₂ substituiert sein können; und
R⁵ ist bei jedem Auftreten gleich oder verschieden gewählt aus H, D, F, C(=O)R⁴, CN, Si(R⁴)₃, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)₂, C=O, C=NR⁴, -C(=O)O-, -C(=O)NR⁴-, P(=O)(R⁴), -O-, -S-, SO oder SO₂ ersetzt sein können; und
R⁶ ist bei jedem Auftreten gleich oder verschieden gewählt aus H, D, F, Cl, Br, I, CN, NO₂, CF₃, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)₂, C=O, C=NR⁴, -C(=O)O-, -C(=O)NR⁴-, P(=O)(R⁴), -O-, -S-, SO oder SO₂ ersetzt sein können.

Bevorzugt ist in den Formeln (L-I) bis (L-III) jeweils mindestens eine Gruppe R³ vorhanden, welche gewählt ist aus F, Cl, Br, I, CN, NO₂ und Alkylgruppen mit 1 bis 20 C-Atomen, welche mindestens einen Substituenten gewählt aus F, Cl, CN und NO₂ aufweisen. Unter den genannten Gruppen besonders bevorzugt sind F, Cl, CN und CF₃. Besonders bevorzugt sind eine, zwei, oder drei solcher Gruppen R³ vorhanden, ganz besonders bevorzugt drei.

Bevorzugt ist in Formel (L-IV) mindestens eine Gruppe R⁶ vorhanden, welche gewählt ist aus F, Cl, Br, I, CN, NO₂ und Alkylgruppen mit 1 bis 20 C-Atomen, welche mindestens einen Substituenten gewählt aus F, CI, CN und NO₂ aufweisen. Unter den genannten Gruppen besonders bevorzugt sind F, Cl, CN und CF₃.

Besonders bevorzugt sind eine, zwei, oder drei solcher Gruppen R⁶ vorhanden, ganz besonders bevorzugt drei.

Bevorzugte Liganden L sind gewählt aus fluorierten Benzoesäure-Derivaten, fluorierten oder nicht-fluorierten Phenylessigsäurederivaten und fluorierten oder nicht-fluorierten Essigsäurederivaten.

Beispiele für bevorzugte fluorierte Benzoesäure-Derivate sind: 2-(Trifluoromethyl)benzoesäure; 3,5-Difluorobenzoesäure; 3-Hydroxy-2,4,6-triiodobenzoesäure; 3-Fluoro-4-methylbenzoesäure; 3-(Trifluoromethoxy)benzoesäure; 4-(Trifluoromethoxy)benzoesäure; 4-Chloro-2,5-difluorobenzoesäure; 2-Chloro-4,5-difluorobenzoesäure; 2,4,5-Trifluorobenzoesäure; 2-Fluorobenzoesäure; 4-Fluorobenzoesäure; 2,3,4-Trifluorobenzoesäure; 2,3,5-Trifluorobenzoesäure; 2,3-Difluorobenzoesäure; 2,4-Bis(trifluoromethyl)benzoesäure; 2,4-Difluorobenzoesäure; 2,5-Difluorobenzoesäure; 2,6-Bis-(trifluoromethyl)benzoesäure; 2,6-Difluorobenzoesäure; 2-Chloro-6-fluorobenzoesäure; 2-Fluoro-4-(trifluoromethyl)benzoesäure; 2-Fluoro-5-(trifluoromethyl)benzoesäure; 2-Fluoro-6-(trifluoromethyl)benzoesäure; 3,4, 5-Trifluorobenzoesäure; 3,4-Difluorobenzoesäure; 3 ,5-Bis(trifluoromethyl)benzoesäure; 3-(Trifluoromethyl)benzoesäure; 3-Chloro-4-fluorobenzoesäure; 3-Fluoro-5-(trifluoromethyl)benzoesäure; 3-Fluorobenzoesäure; 4-Fluoro-2-(trifluoromethyl)benzoesäure; 4-Fluoro-3-(trifluoromethyl)benzoesäure; 5-Fluoro-2-methylbenzoesäure; 2-(Trifluoromethoxy)benzoesäure; 2,3,5-Trichlorobenzoesäure; 4-(Trifluoromethyl)benzoesäure; Pentafluorobenzoesäure; und 2,3,4,5-Tetrafluorobenzoesäure.

Beispiele für fluorierte oder nicht-fluorierte Phenylessigsäurederivate sind: 2-Fluor-Phenylessigsäure; 3-Fluor-Phenylessigsäure; 4-Fluor-Phenylessigsäure; 2,3-Difluor-Phenylessigsäure; 2,4-Difluor-Phenylessigsäure; 2,6-Difluor-Phenylessigsäure; 3,4-Difluor-Phenylessigsäure; 3,5-Difluor-Phenylessigsäure; Pentafluor-Phenylessigsäure; 2-Chloro-6-fluor-Phenylessigsäure; 2-Chloro-3,6-difluor-Phenylessigsäure; 3-Chloro-2,6-difluor-Phenylessigsäure; 3-Chloro-4-fluor-Phenylessigsäure; 5-Chloro-2-fluor-Phenylessigsäure; 2,3,4-Trifluor-Phenylessigsäure; 2,3,5-Trifluor-Phenylessigsäure; 2,3,6-Trifluor-Phenylessigsäure; 2,4,5-Trifluor-Phenylessigsäure; 2,4,6-Trifluor-Phenylessigsäure; 3,4,5-Trifluor-Phenylessigsäure; 3-Chloro-2-fluor-Phenylessigsäure; 6-Fluor-Phenylessigsäure; 4-Chloro-2-fluor-Phenylessigsäure; 2-Chloro-4-fluor-Phenylessigsäure.

Beispiele für fluorierte oder nicht-fluorierte Essigsäurederivate sind: Difluoroessigsäure; Trifluoroessigsäure; Chlorodifluoroessigsäure; (3-Chlorophenyl)-difluoroessigsäure; (3,5-Difluorophenyl)-difluoroessigsäure; (4-Butylphenyl) difluoroessigsäure; (4-tert-Butylphenyl)difluoroessigsäure; (3,4-Dimethylphenyl)-difluoroessigsäure; (3-Chloro-4-fluorophenyl)-difluoroessigsäure; (4-Chlorophenyl)-difluoroessigsäure; 2-Biphenyl-3',5'-difluoroessigsäure; 3-Biphenyl-3',5'-difluoroessigsäure; 4-Biphenyl-3',5'-difluoroessigsäure; 2-Biphenyl-3',4'-difluoroessigsäure; 3-Biphenyl-3',4'-difluoroessigsäure; 4-Biphenyl-3',4'-difluoroessigsäure und 2,2-Difluoro-propionsäure und deren höhere Homologe.

Die in der oben genannten Liste in deprotonierter Form genannten Verbindungen können auch erfindungsgemäß in protonierter Form vorliegen. Bevorzugt liegen sie in deprotonierter Form vor. Die in der oben genannten Liste in protonierter Form genannten Verbindungen können auch in deprotonierter Form vorliegen, was erfindungsgemäß bevorzugt ist.

Das erfindungsgemäße Material kann weitere Verbindungen enthalten. Bevorzugt enthält es im Wesentlichen ausschließlich genau eine Verbindung A und genau eine Verbindung P. Wenn weitere Verbindungen vorhanden sind, so sind dies bevorzugt Verbindungen gemäß der Formeln (A-I) bis (A-IX). In einer möglichen Ausführungsform der Erfindung liegen dabei genau zwei unterschiedliche Verbindungen A und genau eine Verbindung P in dem erfindungsgemäßen Material vor.

Die Verbindung P liegt bevorzugt als Dotand im erfindungsgemäßen Material vor. Es ist bevorzugt, dass das erfindungsgemäße Material die Verbindung P in einer Konzentration von 0.1 % bis 20 %, besonders bevorzugt 0.5 bis 12 %, ganz besonders bevorzugt 1 bis 8 % und am stärksten bevorzugt von 2 bis 6 % enthält.

Prozentangaben sind im Rahmen der vorliegenden Anmeldung so angegeben, dass darunter im Falle der Gasphasenabscheidung Vol.-%, und im Falle des Auftragens aus der flüssigen Phase Gew.-% zu verstehen sind.

Das erfindungsgemäße Material liegt bevorzugt in Form einer dünnen Schicht vor, besonders bevorzugt als Funktionsschicht einer elektronischen Vorrichtung. Gegenstand der vorliegenden Erfindung ist daher auch eine Schicht, bevorzugt eine Halbleiterschicht, enthaltend das erfindungsgemäße Material.

Die Schicht enthaltend das erfindungsgemäße Material hat bevorzugt eine Stärke von 1 bis 500 nm, besonders bevorzugt von 5 bis 300 nm und ganz besonders bevorzugt von 8 bis 250 nm. Sie wird bevorzugt als lochtransportierende Schicht in einer elektronischen Vorrichtung, bevorzugt einer OLED, eingesetzt, wie weiter unten im Detail ausgeführt wird.

Das erfindungsgemäße Material kann aus der Gasphase, beispielsweise mittels OVPD (Organic Vapour Phase Deposition) Verfahren oder mit Hilfe einer Trägergassublimation in Form einer Schicht aufgebracht werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301). Alternativ kann das Material auch aus flüssiger Phase, wie z. B. durch Spincoating, oder mit einem beliebigen Druckverfahren, wie z. B. Siebdruck, Flexodruck, Nozzle Printing oder Offsetdruck, bevorzugt LITI (Light Induced Thermal Imaging, Thermotransferdruck) oder Ink-Jet Druck (Tintenstrahldruck), hergestellt werden.

Für die Verarbeitung des erfindungsgemäßen Materials aus flüssiger Phase, beispielsweise durch die oben genannten Verfahren, sind Formulierungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend das erfindungsgemäße Material sowie mindestens ein Lösungsmittel, bevorzugt ein organisches Lösungsmittel. Wie eine solche Formulierung, insbesondere solche Lösungen hergestellt werden können, ist dem Fachmann bekannt und beispielsweise in WO 2002/072714, WO 2003/019694 und der darin zitierten Literatur beschrieben.

Bei der Gasphasenabscheidung werden sowohl Verbindung P als auch Verbindung A gemeinsam, bevorzugt aus unterschiedlichen Aufdampfquellen, im Hochvakuum verdampft und als Schicht abgeschieden. Bei der Auftragung aus flüssiger Phase werden die Verbindung P und die Verbindung A in Lösungsmittel gelöst und dann mittels der oben genannten Drucktechniken aufgetragen. Die Schicht enthaltend das erfindungsgemäße Material wird schließlich durch Verdampfen des Lösungsmittels erhalten.

Gegenstand der vorliegenden Anmeldung ist daher auch ein Verfahren zur Herstellung einer Schicht enthaltend das erfindungsgemäße Material, dadurch gekennzeichnet, dass Verbindung A und Verbindung P aus der Gasphase zusammen aufgebracht werden, oder dass eine Formulierung enthaltend das erfindungsgemäße Material aus flüssiger Phase aufgebracht wird.

Das erfindungsgemäße Material eignet sich für den Einsatz in elektronischen Vorrichtungen, bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Dabei kann das Material in unterschiedlichen Funktionen eingesetzt werden. Bevorzugt ist die Verwendung des Material in einer lochtransportierenden Schicht, beispielsweise einer Lochinjektionsschicht, einer Lochtransportschicht, einer Excitonenblockierschicht oder einer Elektronenblockierschicht. Die oben beschriebene Verwendung des Materials ist ebenfalls Gegenstand der Erfindung.

Eine Lochtransportschicht gemäß der vorliegenden Anmeldung ist eine Schicht mit lochtransportierender Funktion, welche sich zwischen Anode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Weiterer Gegenstand der Erfindung ist eine elektronische Vorrichtung, enthaltend das erfindungsgemäße Material, bevorzugt in Form einer Schicht. Dabei ist die elektronische Vorrichtung bevorzugt ausgewählt aus den oben genannten Vorrichtungen. Besonders bevorzugt ist die elektronische Vorrichtung eine OLED, enthaltend Anode, Kathode und mindestens eine emittierende Schicht, dadurch gekennzeichnet, dass mindestens eine Schicht, bevorzugt eine lochtransportierende Schicht, das erfindungsgemäße Material enthält.

Außer Kathode, Anode und emittierender Schicht enthält die OLED bevorzugt noch weitere Funktionsschichten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der OLED enthaltend das erfindungsgemäße Material ist bevorzugt die folgende:
- Anode
- Lochinjektionsschicht
- Lochtransportschicht
- wahlweise weitere Lochtransportschicht
- wahlweise Elektronenblockierschicht
- emittierende Schicht
- wahlweise Lochblockierschicht
- Elektronentransportschicht
- Elektroneninjektionsschicht
- Kathode.

Es müssen jedoch nicht alle der genannten Schichten vorhanden sein, und es können zusätzlich weitere Schichten vorhanden sein.

Die erfindungsgemäße OLED kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, gelbes, grünes, orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013).

Bevorzugt enthält die OLED das erfindungsgemäße Material in einer lochtransportierenden Schicht, welche zwischen Anode und emittierender Schicht angeordnet ist, wobei bevorzugt zwischen der Schicht enthaltend das erfindungsgemäße Material und der emittierenden Schicht noch eine oder mehrere weitere Schichten vorliegen. Bevorzugt sind diese weiteren Schichten Lochtransportschichten, besonders bevorzugt Elektronenblockierschichten. Die genannten weiteren Schichten können p-dotiert oder nicht p-dotiert sein.

Es kann aber auch bevorzugt sein, dass das erfindungsgemäße Material in einer lochtransportierenden Schicht angeordnet ist, welche sich direkt angrenzend an die emittierende Schicht befindet.

Die Lochtransportschichten, die zwischen der Schicht enthaltend das erfindungsgemäße Material und der emittierenden Schicht angeordnet sind, enthalten bevorzugt eine oder mehrere gleiche oder verschiedene Verbindungen gemäß einer der Formeln (A-I) bis (A-IX), bevorzugt verschiedene Verbindungen gemäß einer der Formeln (A-I) bis (A-IX).

Die erfindungsgemäße OLED kann eine Vielzahl von Lochtransportschichten aufweisen. Bevorzugt sind es ein, zwei, drei, vier oder fünf Lochtransportschichten, besonders bevorzugt zwei, drei oder vier Lochtransportschichten, von denen mindestens eine das erfindungsgemäße Material enthält.

Es ist weiterhin bevorzugt, dass die OLED das erfindungsgemäße Material in einer Schicht enthält, welche unmittelbar an die Anode angrenzt.

Besonders bevorzugt umfasst die erfindungsgemäße OLED eine der folgenden Schichtaufbauten:
a) Anode -- Schicht enthaltend das erfindungsgemäße Material-Elektronenblockierschicht -- emittierende Schicht;
b) Anode -- Schicht enthaltend das erfindungsgemäße Material-Lochtransportschicht -- Elektronenblockierschicht -- emittierende Schicht;
c) Anode -- erste Schicht enthaltend das erfindungsgemäße Material-Lochtransportschicht -- zweite Schicht enthaltend das erfindungsgemäße Material -- Elektronenblockierschicht -- emittierende Schicht.

Diejenigen Schichten, die sich dabei bevorzugt kathodenseitig an die emittierende Schicht anschließen, entsprechen den oben genannten bevorzugten Schichten in diesen Positionen, nämlich eine oder mehrere Lochblockierschichten, Elektronentransportschichten und Elektroneninjektionsschichten.

In Aufbau a) können dabei sowohl die Schicht enthaltend das erfindungsgemäße Material als auch die Elektronenblockierschicht dieselbe Verbindung gemäß einer der Formeln (A-I) bis (A-IX) enthalten. In Aufbau c) kann sowohl die Schicht enthaltend das erfindungsgemäße Material als auch die Lochtransportschicht dieselbe Verbindung gemäß einer der Formeln (A-I) bis (A-IX) enthalten, und/oder sowohl die zweite Schicht enthaltend das erfindungsgemäße Material als auch die Elektronenblockierschicht dieselbe Verbindung gemäß einer der Formeln (A-I) bis (A-IX) enthalten.

Weiterhin ist es bevorzugt, dass die Elektronenblockierschicht in der erfindungsgemäßen OLED, bzw. diejenige Schicht, die anodenseitig direkt an die emittierende Schicht angrenzt, eine Verbindung gemäß einer der Formeln (A-I) bis (A-IX) enthält, insbesondere eine Verbindung gemäß einer der Formeln (A-I) bis (A-IX), die ein Mono-Arylamin ist.

Es ist bevorzugt, wenn das erfindungsgemäße Material in einer OLED enthaltend einen oder mehrere phosphoreszierende emittierende Verbindungen eingesetzt wird. Vom Begriff phosphoreszierende emittierende Verbindungen sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Beispiele für die oben beschriebenen emittierenden Verbindungen können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit dem erfindungsgemäßen Material in einer OLED einsetzen.

In einer alternativen Ausführungsform ist es bevorzugt, dass das Material in einer OLED eingesetzt wird, welche in ihrer emittierenden Schicht eine fluoreszierende emittierende Verbindung enthält. Bevorzugt enthält die emittierende Schicht in diesem Fall eine Arylaminoverbindung als fluoreszierende emittierende Verbindung, besonders bevorzugt in Kombination mit einem Hostmaterial. Das Hostmaterial ist in diesem Fall bevorzugt gewählt aus Verbindungen enthaltend eine oder mehrere Anthracengruppen.

Im Folgenden werden bevorzugte Ausführungsformen für die verschiedenen Funktionsmaterialien der elektronischen Vorrichtung aufgeführt.

Bevorzugte phosphoreszierende emittierende Verbindungen sind die oben genannten Verbindungen.

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine und die in WO 2014/111269 offenbarten erweiterten Benzoindenofluorene.

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, sowie die in EP 1749809, EP 1905754 und US 2012/0187826 offenbarten Pyren-Verbindungen.

Bevorzugte Matrixmaterialien für phosphoreszierende emittierende Verbindungen sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den Verbindungen der Formeln (A-I) bis (A-IX) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Beispiele für bevorzugte Materialien, die in einer Lochtransport-, Lochinjektions- oder Elektronenblockierschicht in der erfindungsgemäßen Elektrolumineszenzvorrichtung verwendet werden können, sind neben den Verbindungen der Formeln (A-I) bis (A-IX) Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 und WO 2013/120577), Fluoren-Amine (z. B. gemäß WO 2014/015937, WO 2014/015938 und WO 2014/015935), Spiro-Dibenzopyran-Amine (z. B. gemäß WO 2013/083216) und Dihydroacridin-Derivate (z. B. gemäß WO 2012/150001).

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar. Bevorzugte Verfahren zur Auftragung von Schichten aus der Gasphase sind weiter oben beschrieben, und gelten allgemein für die Herstellung von Schichten in den erfindungsgemäßen OLEDs.

In einer alternativen Ausführungsform ist die elektronische Vorrichtung dadurch gekennzeichnet, dass eine oder mehrere Schichten aus Lösung aufgetragen werden. Bevorzugte Verfahren zur Auftragung von Schichten aus Lösung sind weiter oben beschrieben, und gelten allgemein für die Herstellung von Schichten in den erfindungsgemäßen OLEDs.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Die erfindungsgemäßen OLEDs können in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthese von Bis[[3,5-bis(trifluoromethyl)benzoyl]oxy]bismuthanyl 3,5-bis(trifluoromethyl)benzoat

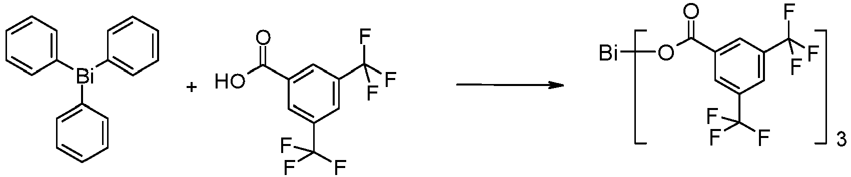

50 g (113,56 mmol) Triphenyl-bismuthan (CAS.Nr.: 603-33-8) und 89,40 g 3,5-Bis-trifluoromethyl-benzoesäure (340,36 mmol) werden in einem unter Argon inertisierten Kolben vorgelegt und mit 1 L getrocknetem Toluol versetzt. Der Ansatz wird langsam auf 80°C erwärmt und dann 12 Stunden bei dieser Temperatur weiter gerührt. Das Gemisch wird im Anschluss auf Raumtemperatur abgekühlt und über eine Schutzgasfritte abfiltriert, dreimal mit Toluol gewaschen, an der Vakuumpumpe getrocknet und abschließend im Hochvakuum sublimiert.

### B) Device-Beispiele

In den folgenden Beispielen E1 bis E8 und V1 bis V2 werden die Daten verschiedener OLEDs vorgestellt. Die Beispiele V1 bis V2 sind Vergleichsbeispiele gemäß dem Stand der Technik, die Beispiele E1 bis E8 zeigen Daten von erfindungsgemäßen OLEDs.

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird. Glasplättchen, die mit strukturiertem ITO (Indium Zinn Oxid) der Dicke 50 nm beschichtet sind, bilden die Substrate für die OLEDs. Die Substrate werden nass gereinigt (Spülmaschine, Reiniger Merck Extran), anschliessend 15 min lang bei 250°C ausgeheizt und vor der Beschichtung mit einem Sauerstoffplasma behandelt.

Auf die vorbehandelten Substrate werden verschiedene Schichten aufgebracht: Erste Lochtransportschicht (HTL1) / zweite Lochtransportschicht (HTL2) / Emissionsschicht (EML) / Elektronentransportschicht (ETL) / Elektroneninjektionsschicht (EIL) und abschließend eine 100 nm dicke Aluminiumkathode. Der genaue Aufbau der OLEDs ist Tabelle 1 zu entnehmen. Die zur Herstellung der OLEDs verwendeten Materialien sind in Tabelle 2 gezeigt.

Alle Materialien werden in einer Vakuumkammer thermisch aufgedampft. Dabei besteht die Emissionsschicht immer aus mindestens einem Matrixmaterial und einer emittierenden Verbindung, die dem Matrixmaterial bzw. den Matrixmaterialien durch Coverdampfung in einem bestimmten Volumenanteil beigemischt wird. Eine Angabe wie M1:D1 (95%:5%) bedeutet hierbei, dass das Material M1 in einem Volumenanteil von 95% und D1 in einem Volumenanteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Als Lebensdauer LT80 wird die Zeit definiert, nach der die Leuchtdichte bei Betrieb mit konstanter Stromdichte von der Startleuchtdichte auf 80 % des Startwertes abfällt.

**Tabelle 1: Aufbau der OLEDs**

| Bsp | HTL1 Dicke | HTL2 Dicke | EML Dicke | ETL Dicke | EIL Dicke |
|---|---|---|---|---|---|
| V1 | NPB:BiC | NPB | M1:D1 | ST1:LiQ (50%:50%) | LiQ |
| | (95%:5%) 20nm | 180nm | (95%:5%) 20nm | 30nm | 1nm |
| V2 | DA1:BiC | DA1 | M1:D1 | ST1:LiQ (50%:50%) | LiQ |
| | (95%:5%) 20nm | 180nm | (95%:5%) 20nm | 30nm | 1nm |
| E1 | MA2:BiC | MA2 | M1:D1 | ST1:LiQ (50%:50%) | LiQ |
| | (95%:5%) 20nm | 180nm | (95%:5%) 20nm | 30nm | 1nm |
| E2 | MA4:BiC | MA4 | M1:D1 | ST1:LiQ (50%:50%) | LiQ |
| | (95%:5%) 20nm | 180nm | (95%:5%) 20nm | 30nm | 1nm |
| E3 | MA5:BiC | MA5 | M1:D1 | ST1:LiQ (50%:50%) | LiQ |
| | (95%:5%) 20nm | 180nm | (95%:5%) 20nm | 30nm | 1nm |
| E4 | MA6:BiC | MA6 | M1:D1 | ST1:LiQ (50%:50%) | LiQ |
| | (95%:5%) 20nm | 180nm | (95%:5%) 20nm | 30nm | 1nm |
| E5 | MA7:BiC | MA7 | M1:D1 | ST1:LiQ (50%:50%) | LiQ |
| | (95%:5%) 20nm | 180nm | (95%:5%) 20nm | 30nm | 1nm |
| E6 | MA8:BiC | MA8 | M1:D1 | ST1:LiQ (50%:50%) | LiQ |
| | (95%:5%) 20nm | 180nm | (95%:5%) 20nm | 30nm | 1nm |
| E7 | MA9:BiC | MA9 | M1:D1 | ST1:LiQ (50%:50%) | LiQ |
| | (95%:5%) 20nm | 180nm | (95%:5%) 20nm | 30nm | 1nm |
| E8 | DA2:BiC | DA2 | M1:D1 | ST1:LiQ (50%:50%) | LiQ |
| | (95%:5%) 20nm | 180nm | (95%:5%) 20nm | 30nm | 1nm |

**Tabelle 2: Strukturformeln der Materialien für die OLEDs**

| | |
|---|---|
| 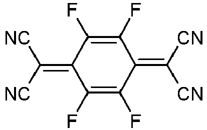 | 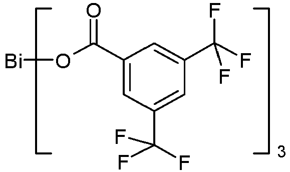 |
| F4TCNQ | BiC |
| 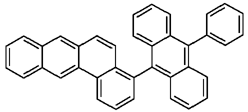 | 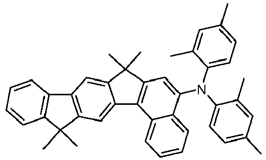 |
| M1 | D1 |
| 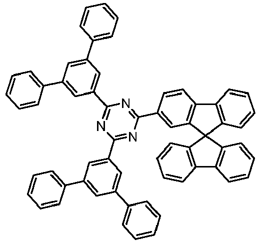 | 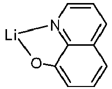 |
| ST1 | LiQ |
| 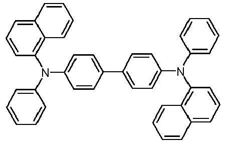 | 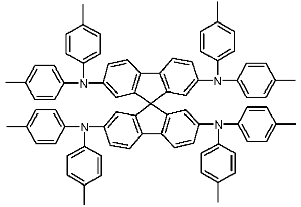 |
| NPB | DA1 |
| 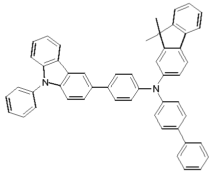 | 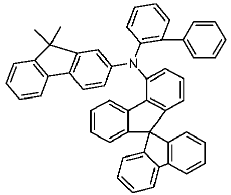 |
| DA2 | MA2 |
| 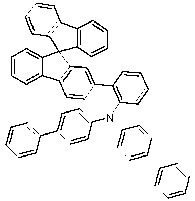 | 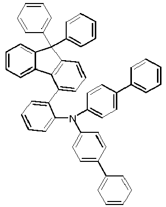 |
| MA4 | MA5 |
| 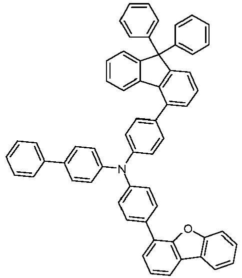 | 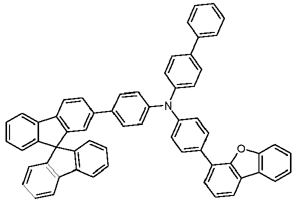 |
| MA6 | MA7 |
| 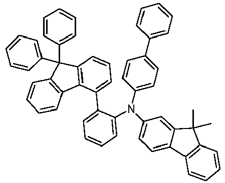 | 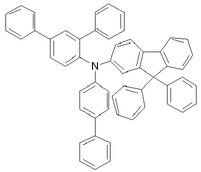 |
| MA8 | MA9 |

Im Folgenden werden die Beispiele näher erläutert, um die Vorteile der erfindungsgemäßen OLEDs zu verdeutlichen.

Die erfindungsgemäßen Proben E1 bis E8 werden den Vergleichsproben V1 und V2 gegenübergestellt. Erstere unterscheiden sich von V1 und V2 dadurch, dass sie ein Mono-Arylamin als Material der HTL enthalten, und kein Diamin oder Tetraamin. In allen Fällen wird der p-Dotand BiC in der ersten der zwei vorhandenen lochtransportierenden Schichten eingesetzt. Alle erfindungsgemäßen Proben E1 bis E8 haben eine bessere Lebensdauer und Effizienz als die Vergleichsproben V1 und V2 (Tabelle 3), bei ähnlichen Werten für die Spannung.

**Tabelle 3: Ergebnisse der OLEDs**

| Bsp | U @ 10 mA/cm² | EQE @ 10 mA/cm² | LT80 @ 60 mA/cm² |
|---|---|---|---|
| V1 | 4,3 | 5,7 | 195 |
| V2 | 3,8 | 4,1 | 50 |
| E1 | 4,2 | 8,3 | 310 |
| E2 | 4,0 | 7,1 | 375 |
| E3 | 4,2 | 7,9 | 365 |
| E4 | 4,6 | 7,7 | 270 |
| E5 | 4,2 | 7,0 | 250 |
| E6 | 4,3 | 8,4 | 270 |
| E7 | 3,9 | 7,3 | 330 |
| E8 | 4,2 | 7,8 | 210 |

Die gezeigten Beispiele verdeutlichen die Vorteile der erfindungsgemäßen Kombination von Bismut-Komplexen mit Mono-Arylaminen der Formeln (A-I) bis (A-IX) als Lochtransportmaterialien in OLEDs. Sie sind nicht einschränkend auszulegen. Die Vorteile der erfindungsgemäßen Kombination erstrecken sich auf den gesamten Umfang der in den Ansprüchen definierten Materialkombinationen.

## Patentansprüche

1. Material, enthaltend eine Verbindung P, die ein Komplex von Bismut ist, und eine Verbindung A , die gewählt ist aus Verbindungen gemäß einer der Formeln (A-II) und (A-IV) bis (A-IX)
| | |
|---|---|
| | 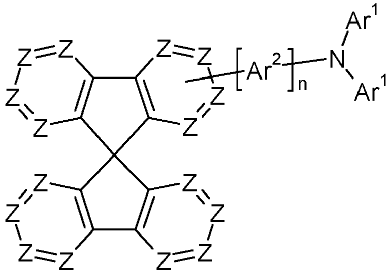 |
| | Formel (A-II) |
| 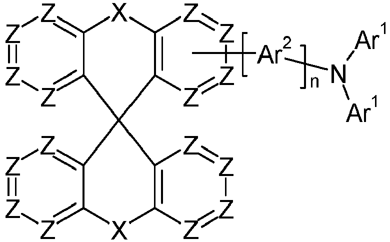 | |
| Formel (A-IV) | |
| 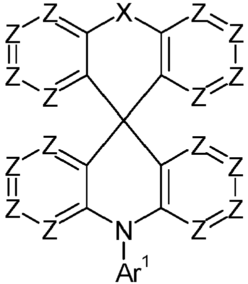 | 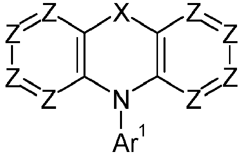 |
| Formel (A-V) | Formel (A-VI) |
| 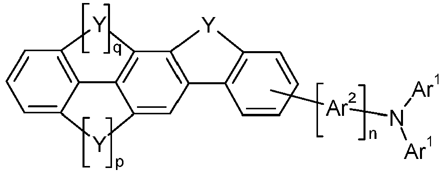 | 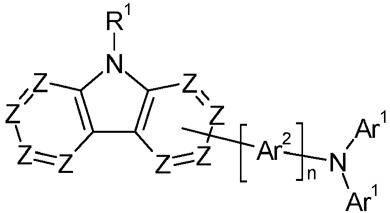 |
| Formel (A-VII) | Formel (A-VIII) |
| 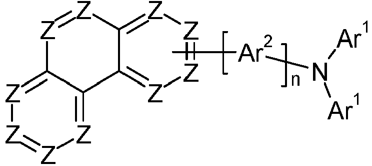 | |
| Formel (A-IX) | |
wobei an allen unsubstituiert dargestellten Positionen ein oder mehrere Reste R¹ gebunden sein können, und für die auftretenden Variablen gilt:
Z ist bei jedem Auftreten gleich oder verschieden CR¹ oder N;
X ist bei jedem Auftreten gleich oder verschieden eine Einfachbindung, O, S, C(R¹)₂, Si(R¹)₂, PR¹, C(R¹)₂-C(R¹)₂, oder CR¹=CR¹;
Y ist bei jedem Auftreten gleich oder verschieden O, S, C(R¹)₂, Si(R¹)₂, PR¹, NR¹, C(R¹)₂-C(R¹)₂, oder CR¹=CR¹;
Ar¹ ist bei jedem Auftreten gleich oder verschieden ein aromatisches Ringsystem mit 6 bis 60 aromatischen Ringatomen, das mit einem oder mehreren Resten R¹ substituiert sein kann, oder ein heteroaromatisches Ringsystem mit 5 bis 60 aromatischen Ringatomen, das mit einem oder mehreren Resten R¹ substituiert sein kann; dabei können Gruppen Ar¹ untereinander über Reste R¹ verbunden sein;
Ar² ist ein aromatisches Ringsystem mit 6 bis 20 aromatischen Ringatomen, das mit einem oder mehreren Resten R¹ substituiert sein kann, oder ein heteroaromatisches Ringsystem mit 5 bis 20 aromatischen Ringatomen, das mit einem oder mehreren Resten R¹ substituiert sein kann;
R¹ ist bei jedem Auftreten gleich oder verschieden gewählt aus H, D, F, C(=O)R², CN, Si(R²)₃, P(=O)(R²)₂, OR², S(=O)R², S(=O)₂R², geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R² substituiert sein können; und wobei eine oder mehrere CH₂-Gruppen in den genannten Alkyl-, Alkoxy-, Alkenyl- und Alkinylgruppen durch -R²C=CR²-, -C=C-, Si(R²)₂, C=O, C=NR², -C(=O)O-, -C(=O)NR²-, P(=O)(R²), -O-, -S-, SO oder SO₂ ersetzt sein können;
R² ist bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R² miteinander verknüpft sein können und einen Ring bilden können; und wobei die genannten Alkylgruppen, aromatischen Ringsysteme und heteroaromatischen Ringsysteme mit F oder CN substituiert sein können;
n,p,q ist gleich oder verschieden 0 oder 1;
oder die gewählt ist aus Verbindungen gemäß Formel (A-I) und (A-III):
| |
|---|
| 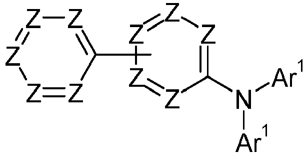 |
| Formel (A-I) |
| 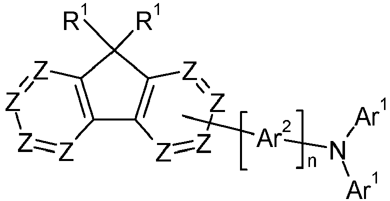 |
| Formel (A-III), |
wobei an allen unsubstituiert dargestellten Positionen ein oder mehrere Reste R¹ gebunden sein können, und
mindestens eine Gruppe Ar¹ bei jedem Auftreten gleich oder verschieden gewählt ist aus den folgenden Gruppen, die jeweils an allen unsubstituiert dargestellten Positionen mit einem oder mehreren Resten R¹ substituiert sein können: 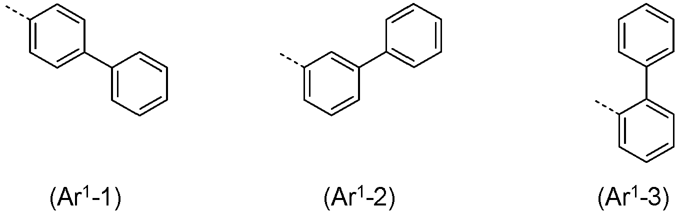 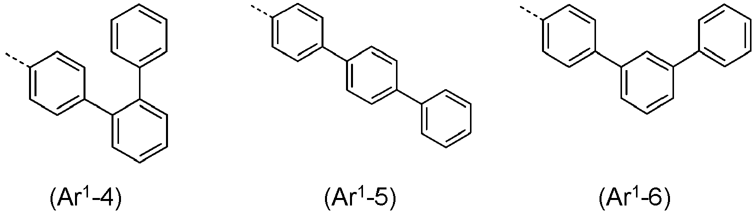 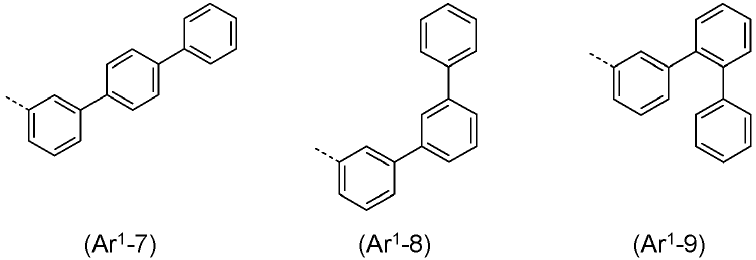 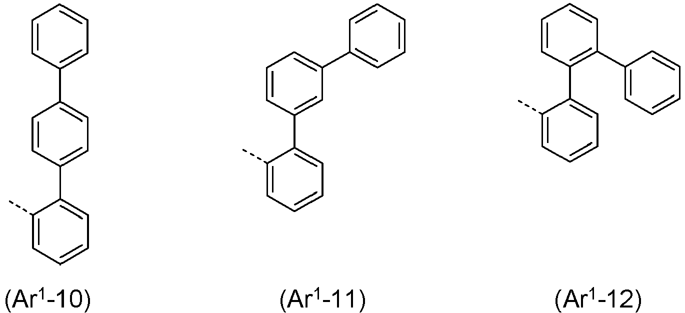 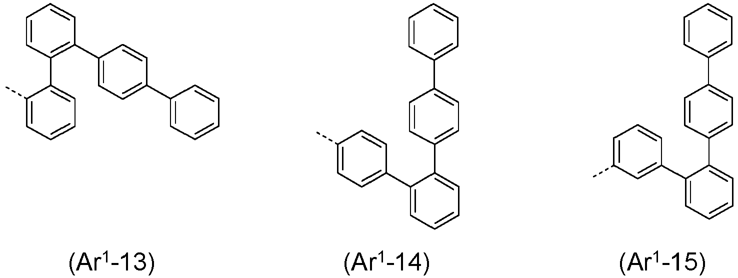 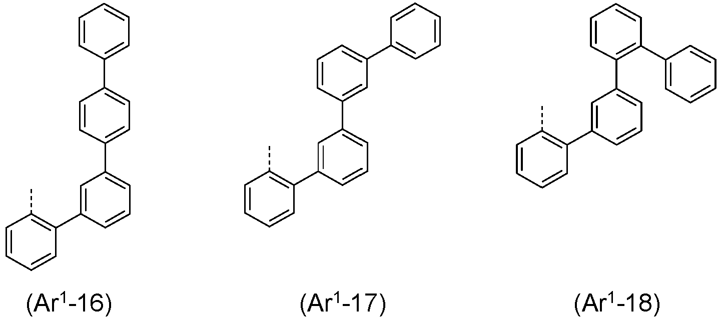 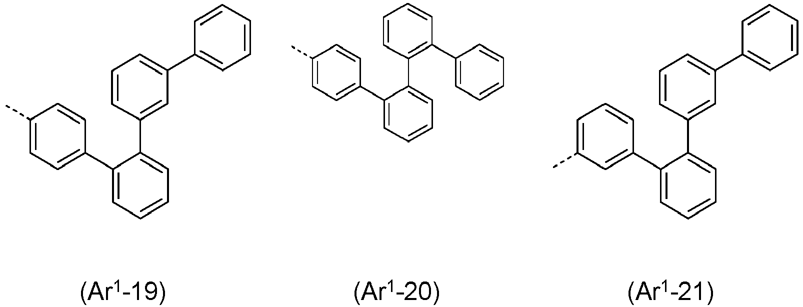 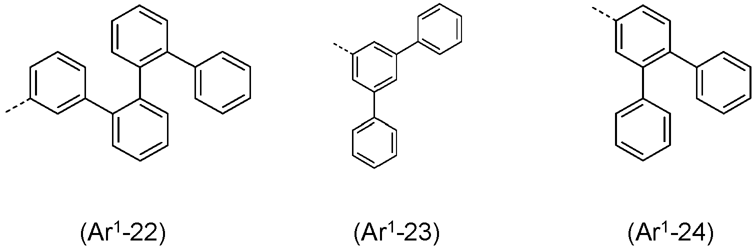 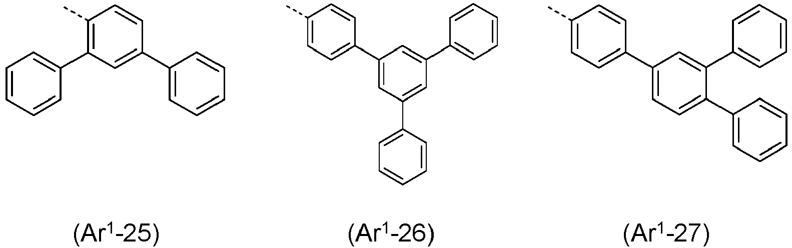 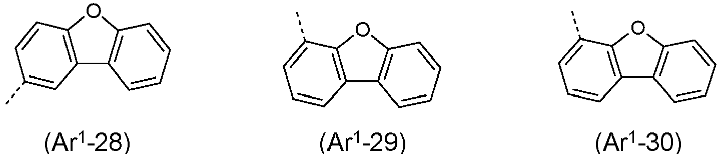 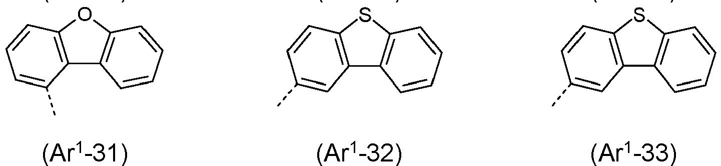 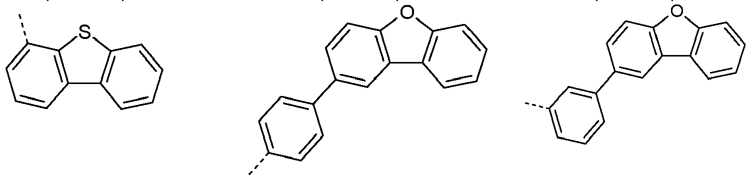 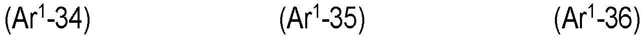 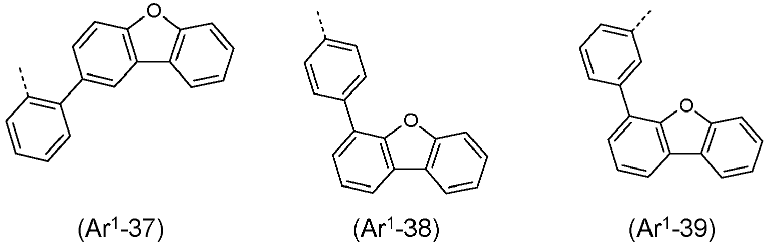 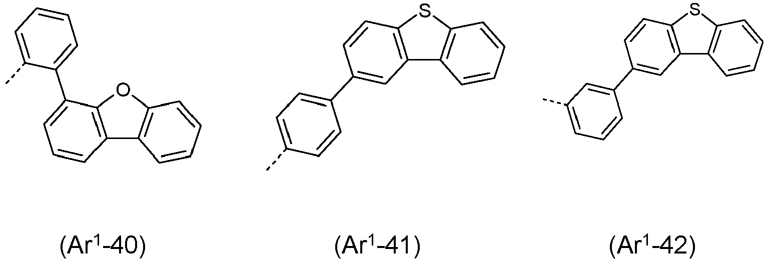 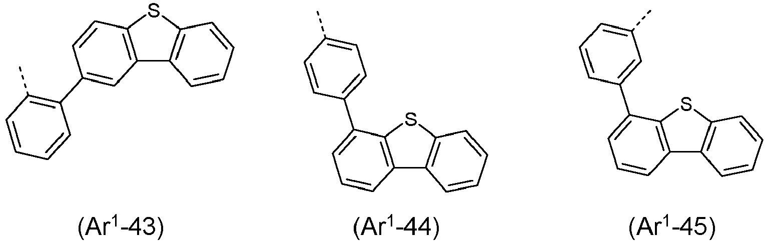 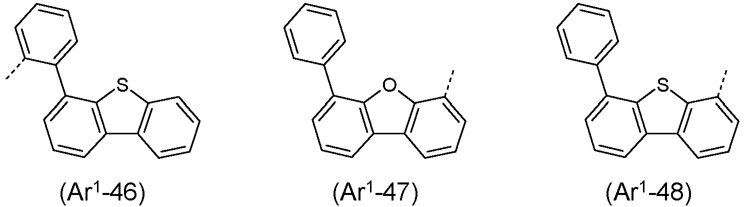 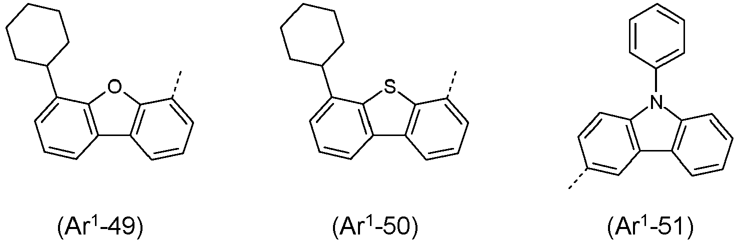 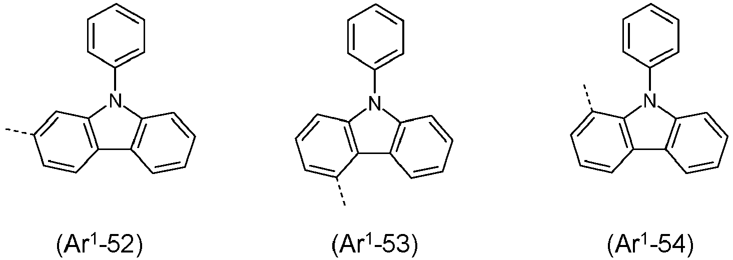 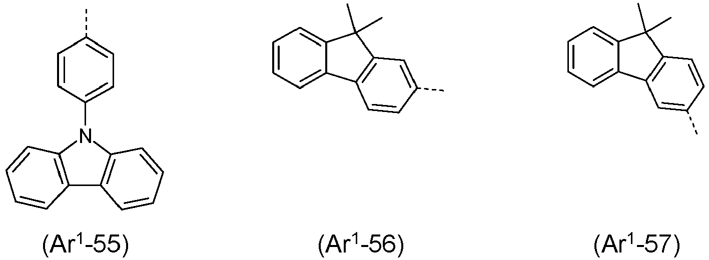 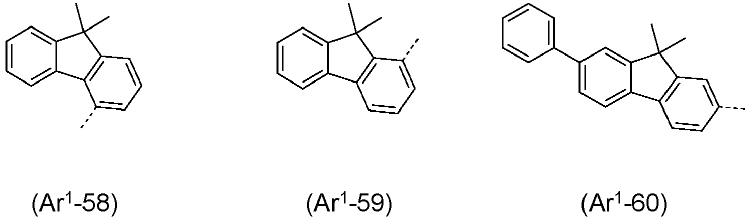 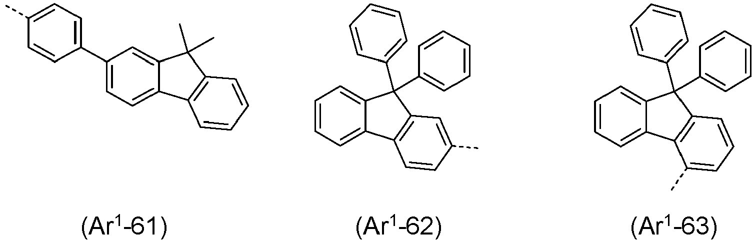 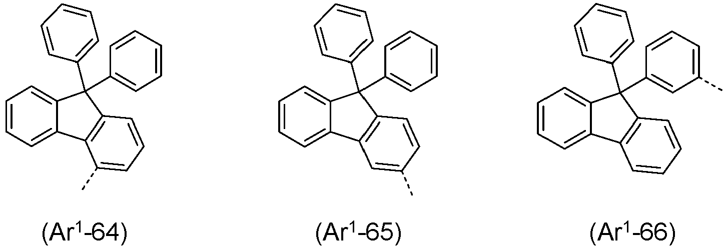 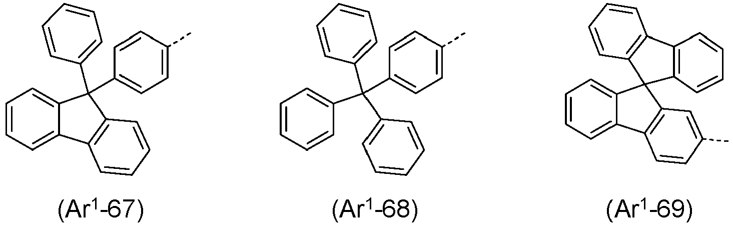 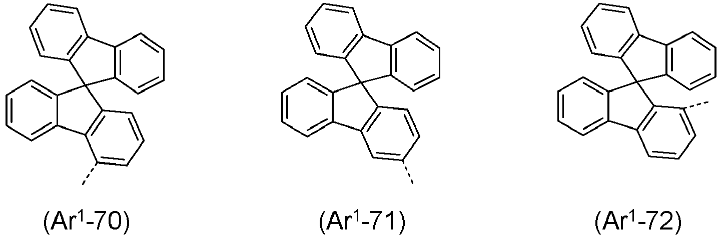 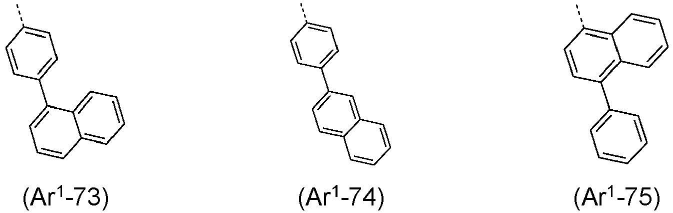 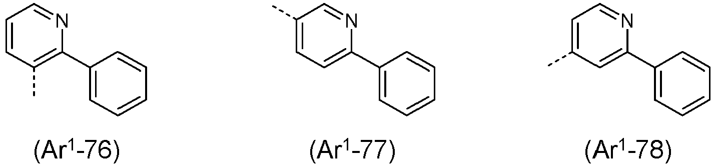 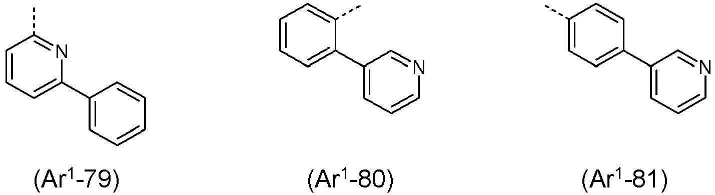 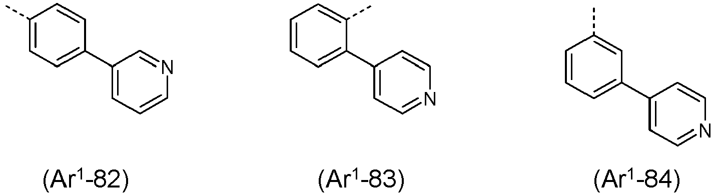 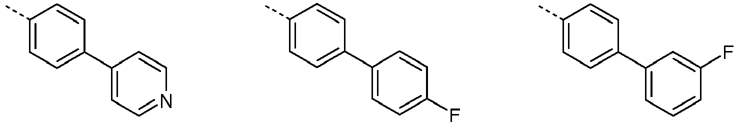 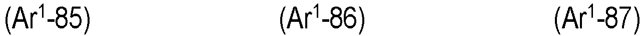 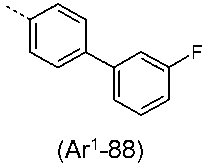
und wobei die sonstigen Variablen wie oben definiert sind;
wobei vom Umfang der Formeln (A-I) bis (A-IX) jeweils Verbindungen der folgenden Formel (B) ausgenommen sind 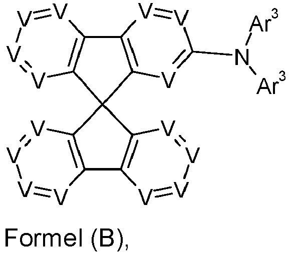
bei der für die neu auftretenden Variablen gilt:
V ist gleich CR¹;
Ar³ ist bei jedem Auftreten gleich oder verschieden ein aromatisches Ringsystem mit 6 bis 30 aromatischen Ringatomen, das mit einem oder mehreren Resten R¹ substituiert sein kann, oder ein heteroaromatisches Ringsystem mit 5 bis 30 aromatischen Ringatomen, das mit einem oder mehreren Resten R¹ substituiert sein kann, dabei können Gruppen Ar¹ untereinander über Reste R¹ verbunden sein.

2. Material nach Anspruch 1, **dadurch gekennzeichnet, dass** die Verbindung A eine einzige Aminogruppe aufweist.

3. Material nach Anspruch 1 oder 2, **dadurch gekennzeichnet, dass** die Verbindung A keine kondensierte Arylgruppe mit mehr als 10 aromatischen Ringatomen und keine kondensierte Heteroarylgruppe mit mehr als 14 aromatischen Ringatomen enthält.

4. Material nach einem oder mehreren der Ansprüche 1 bis 3, **dadurch gekennzeichnet, dass** in der Verbindung A in Formeln (A-II) und (A-IV) bis (A-IX) die Gruppe Ar¹ bei jedem Auftreten gleich oder verschieden ein aromatisches Ringsystem mit 6 bis 40 aromatischen Ringatomen, das mit einem oder mehreren Resten R¹ substituiert sein kann, oder ein heteroaromatisches Ringsystem mit 5 bis 40 aromatischen Ringatomen, das mit einem oder mehreren Resten R¹ substituiert sein kann, ist.

5. Material nach einem oder mehreren der Ansprüche 1 bis 4, **dadurch gekennzeichnet, dass** in der Verbindung A in Formeln (A-II) und (A-IV) bis (A-IX) die Gruppe Ar¹ eine wahlweise mit einem oder mehreren Resten R¹ substituierte Gruppe gewählt aus Phenyl, Biphenyl, Terphenyl, Quaterphenyl, Naphthyl, Phenanthryl, Fluoranthenyl, Fluorenyl, Indenofluorenyl, Spirobifluorenyl, Dibenzofuranyl, Dibenzothiophenyl, Carbazolyl, Acridyl und Phenanthridyl ist.

6. Material nach einem oder mehreren der Ansprüche 1 bis 5, **dadurch gekennzeichnet, dass** die Verbindung P ein Komplex von Bismut in der Oxidationsstufe (III) ist.

7. Material nach einem oder mehreren der Ansprüche 1 bis 6, **dadurch gekennzeichnet, dass** die Verbindung P ein Komplex von Bismut ist, der mindestens einen Liganden L aufweist, der eine organische Verbindung ist.

8. Material nach Anspruch 7, **dadurch gekennzeichnet, dass** der Ligand L einfach negativ geladen ist.

9. Material nach Anspruch 7 oder 8, **dadurch gekennzeichnet, dass** die an das Bismutatom bindende Gruppe des Liganden L gewählt ist aus Carbonsäuregruppen, Thiocarbonsäuregruppen, Carbonsäureamidgruppen und Carbonsäureimidgruppen.

10. Material nach Anspruch 7, **dadurch gekennzeichnet, dass** der Ligand L gewählt ist aus fluorierten Benzoesäure-Derivaten, fluorierten oder nicht-fluorierten Phenylessigsäurederivaten und fluorierten oder nicht-fluorierten Essigsäurederivaten.

11. Material nach einem oder mehreren der Ansprüche 1 bis 10, **dadurch gekennzeichnet, dass** die Verbindung P als Dotand in einer Konzentration von 0.1 % bis 20 % im Material vorliegt.

12. Schicht, enthaltend ein Material nach einem oder mehreren der Ansprüche 1 bis 11.

13. Formulierung, enthaltend ein Material nach einem oder mehreren der Ansprüche 1 bis 11 sowie mindestens ein Lösungsmittel.

14. Verfahren zur Herstellung einer Schicht nach Anspruch 12, **dadurch gekennzeichnet, dass** Verbindung A und Verbindung P aus der Gasphase zusammen aufgebracht werden, oder dass eine Formulierung enthaltend das erfindungsgemäße Material aus flüssiger Phase aufgebracht wird.

15. Verwendung eines Materials nach einem oder mehreren der Ansprüche 1 bis 11 in einer elektronischen Vorrichtung, gewählt aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, organischen lichtemittierenden elektrochemischen Zellen und organischen Laserdioden.

16. Elektronische Vorrichtung, gewählt aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, organischen lichtemittierenden elektrochemischen Zellen und organischen Laserdioden, enthaltend ein Material nach einem oder mehreren der Ansprüche 1 bis 11.

17. Organische Elektrolumineszenzvorrichtung nach Anspruch 16, **dadurch gekennzeichnet, dass** sie das Material in einer lochtransportierenden Schicht aufweist, welche zwischen Anode und emittierender Schicht angeordnet ist, wobei zwischen der Schicht enthaltend das Material und der emittierenden Schicht eine oder mehrere weitere Schichten vorliegen.

18. Organische Elektrolumineszenzvorrichtung nach Anspruch 17, **dadurch gekennzeichnet, dass** die eine oder mehreren weiteren Schichten, die zwischen der Schicht enthaltend das Material und der emittierenden Schicht angeordnet sind, eine oder mehrere gleiche oder verschiedene Verbindungen gemäß einer der Formeln (A-I) bis (A-IX) enthalten.

19. Organische Elektrolumineszenzvorrichtung nach einem oder mehreren der Ansprüche 16 bis 18, **dadurch gekennzeichnet, dass** sie das Material in einer Schicht enthält, welche unmittelbar an die Anode angrenzt.

## Claims

1. Material comprising a compound P which is a complex of bismuth, and a compound A selected from compounds according to any one of the formulae (A-II) and (A-IV) to (A-IX)
| | |
|---|---|
| | 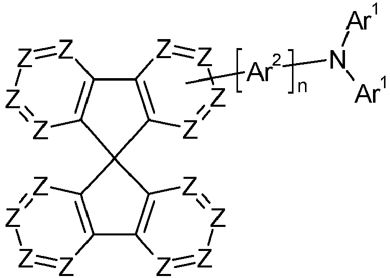 |
| | Formula (A-II) |
| 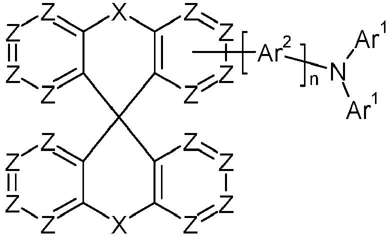 | |
| Formula (A-IV) | |
| 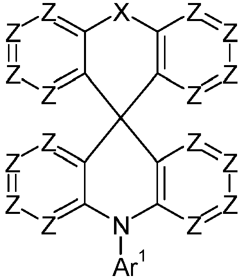 | 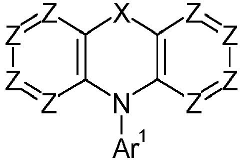 |
| Formula (A-V) | Formula (A-VI) |
| 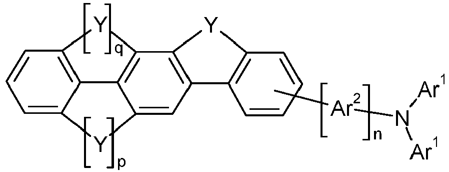 | 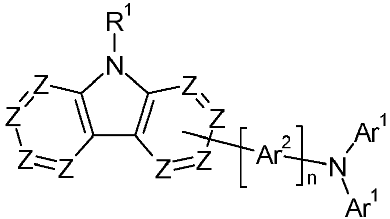 |
| Formula (A-VII) | Formula (A-VIII) |
| 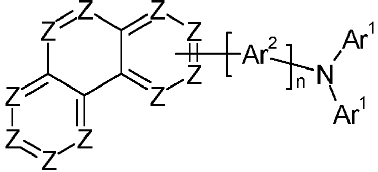 | |
| Formula (A-IX) | |
wherein one or more radicals R¹ may be bonded to all positions shown as unsubstituted, and the following applies to the ocurring variables:
Z is on each occurrence, identically or differently, CR¹ or N;
X is on each occurrence, identically or differently, a single bond, O, S, C(R¹)₂, Si(R¹)₂, PR¹, C(R¹)₂-C(R¹)₂, or CR¹=CR¹;
Y is on each occurrence, identically or differently, O, S, C(R¹)₂, Si(R¹)₂, PR¹, NR¹, C(R¹)₂-C(R¹)₂, or CR¹=CR¹;
Ar¹ is on each occurrence, identically or differently, an aromatic ring system with 6 to 60 aromatic ring atoms which may be substituted by one or more radicals R¹, or a heteroaromatic ring system with 5 to 60 aromatic ring atoms which may be substituted by one or more R¹ radicals; groups Ar¹ may be connected to each other via R¹ radicals;
Ar² is an aromatic ring system with 6 to 20 aromatic ring atoms, which may be substituted by one or more radicals R¹, or a heteroaromatic ring system with 5 to 20 aromatic ring atoms, which may be substituted by one or more radicals R¹;
R¹ is selected on each occurrence, identically or differently, from H, D, F, C(=O)R², CN, Si(R²)₃, P(=O)(R²)₂, OR², S(=O)R², S(=O)₂R², straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; wherein two or more radicals R¹ may be linked to one another and may form a ring; here the said alkyl, alkoxy, alkenyl and alkynyl groups and the said aromatic ring systems and heteroaromatic ring systems may in each case be substituted by one or more R² radicals; and wherein one or more CH₂ groups in the said alkyl, alkoxy, alkenyl and alkynyl groups may be replaced by -R²C=CR²-, -C=C-, Si(R²)₂, C=O, C=NR², -C(=O)O-, -C(=O)NR²-, P(=O)(R²), -O-, -S-, SO or SO₂;
R² is selected on each occurrence, identically or differently, from H, D, F, CN, alkyl groups having 1 to 20 C atoms, aromatic ring systems having 6 to 40 aromatic ring atoms and heteroaromatic ring systems having 5 to 40 aromatic ring atoms; wherein two or more radicals R² may be linked to one another and may form a ring; and wherein the said alkyl groups, aromatic ring systems and heteroaromatic ring systems may be substituted by F or CN;
n,p,q is, identically or differently, 0 or 1;
or selected from compounds according to any one of the formulae (A-I) and (A-III):
| |
|---|
| 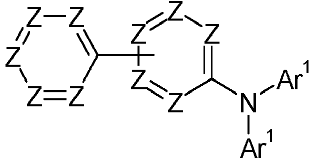 |
| Formel (A-I) |
| 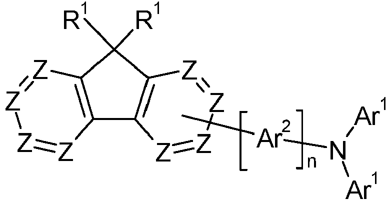 |
| Formel (A-III), |
wherein one or more radicals R¹ may be bonded to all positions shown as unsubstituted, and
at least one group Ar¹ is selected in each occurrence identically or differently from the following groups, each of which may be substituted at all positions shown as unsubstituted by one or more radicals R¹: 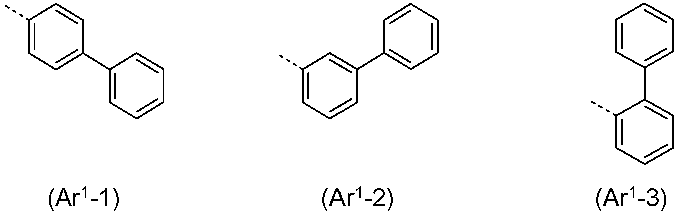 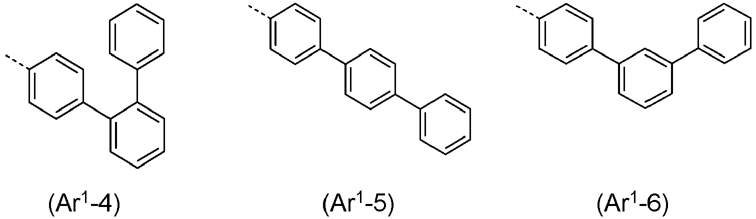 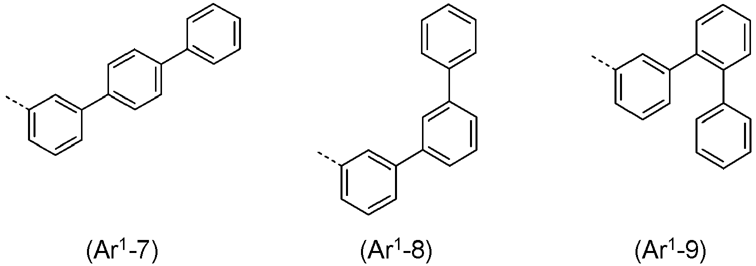 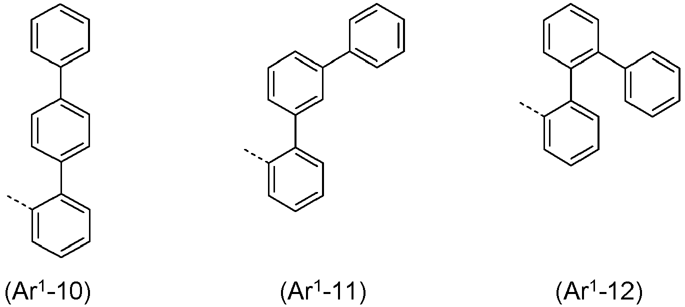 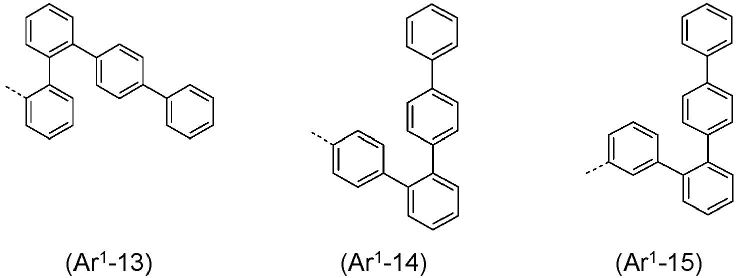 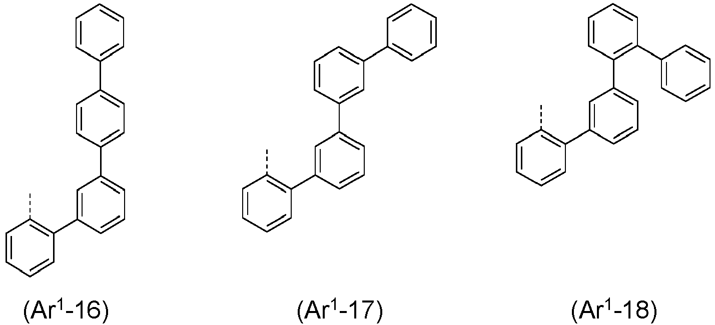 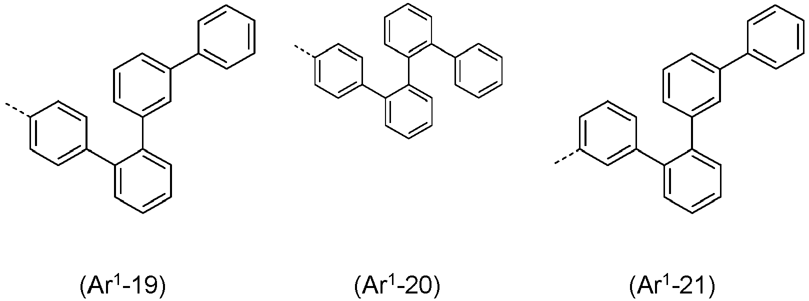 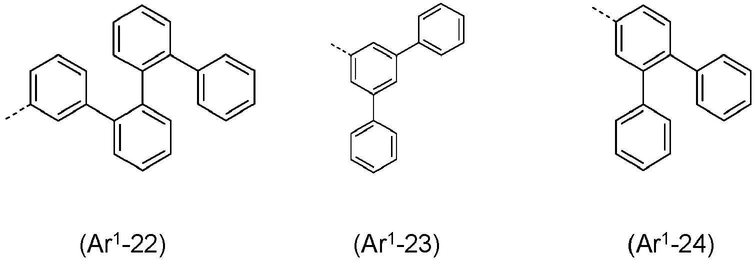 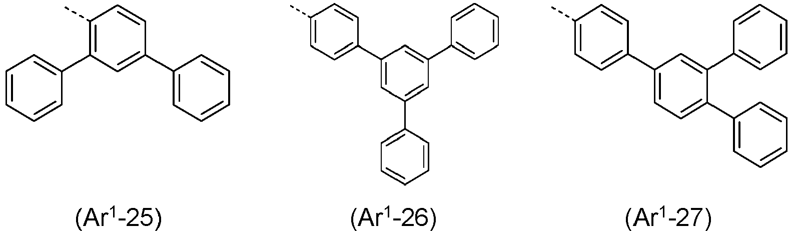 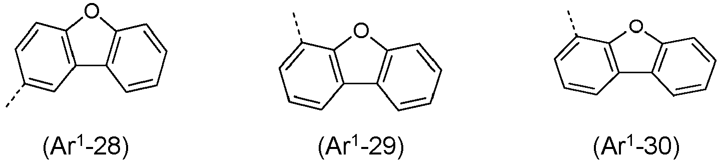 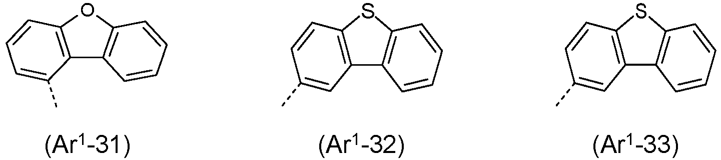 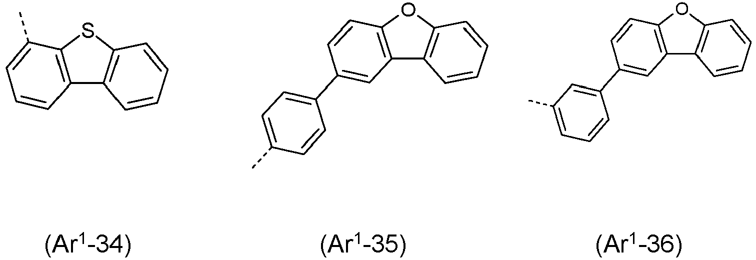 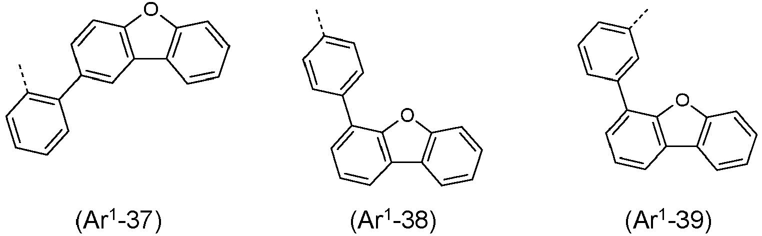 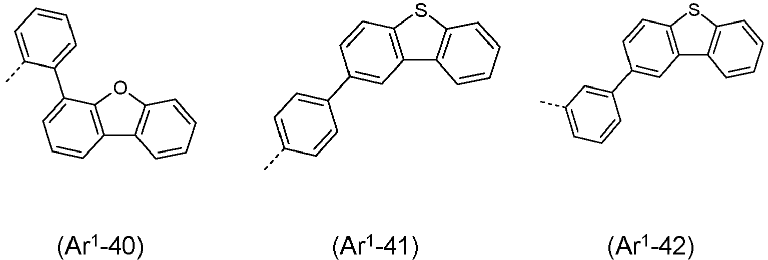 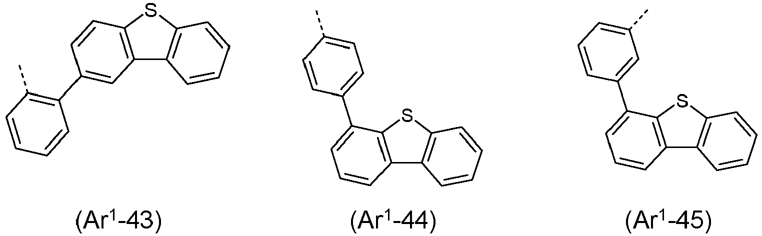 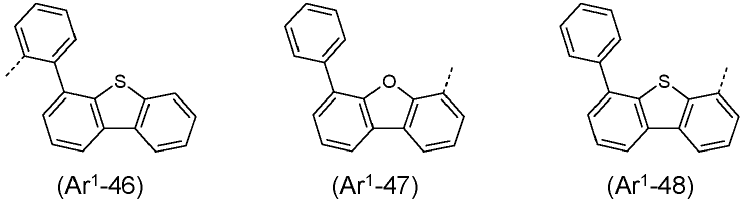 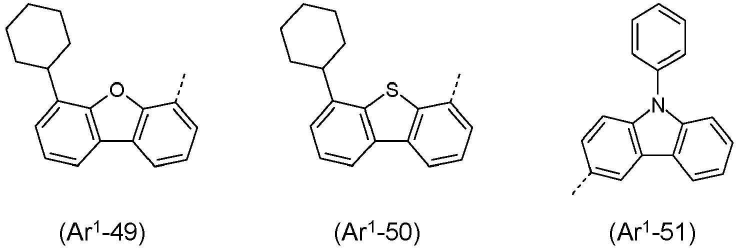 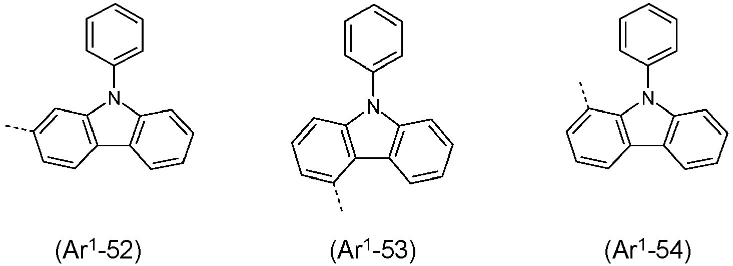 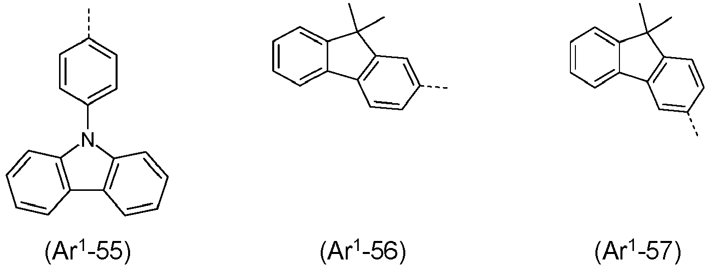 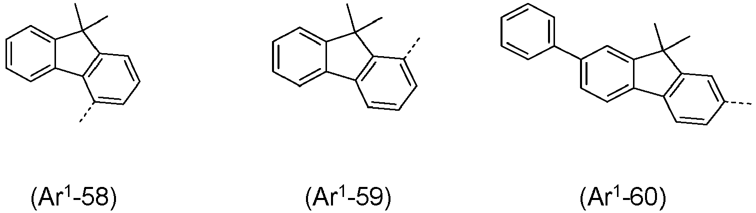 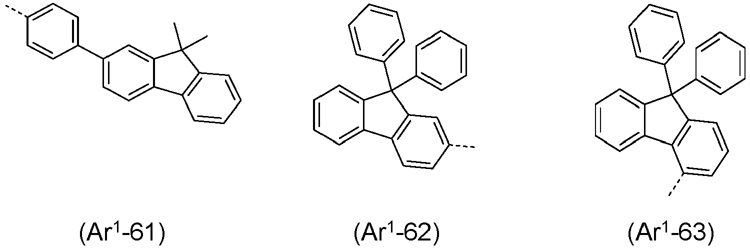 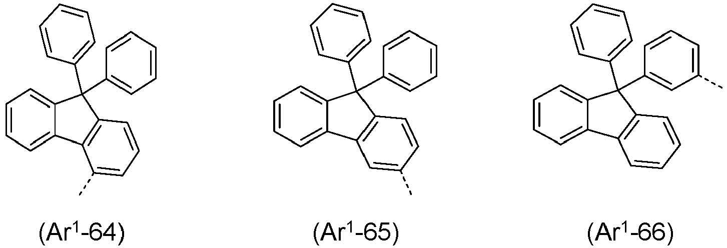 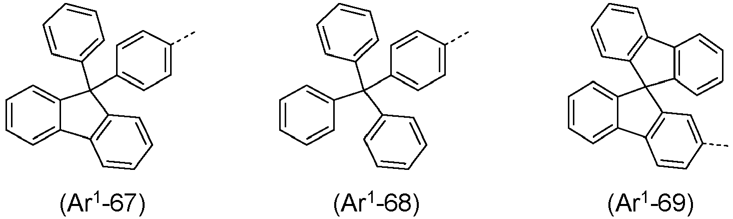 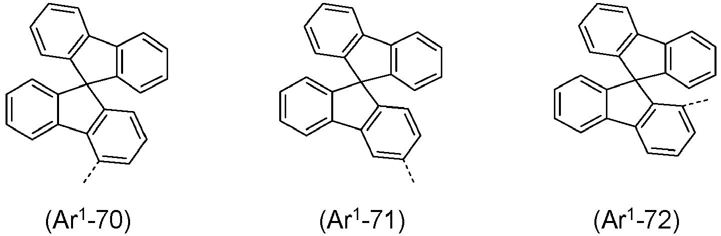 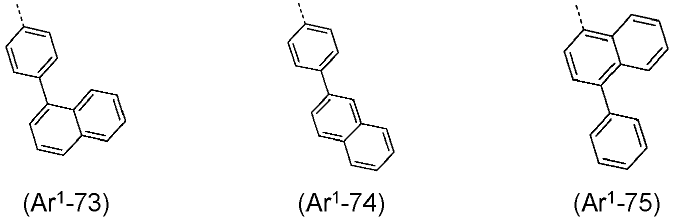 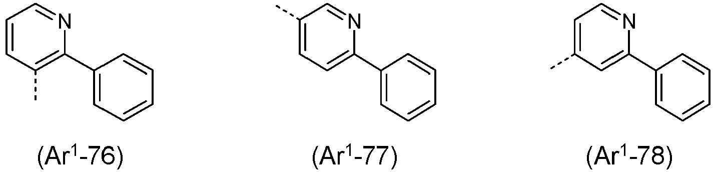 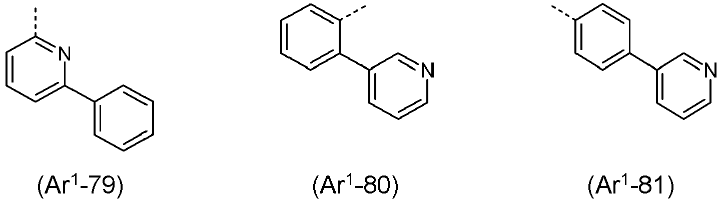 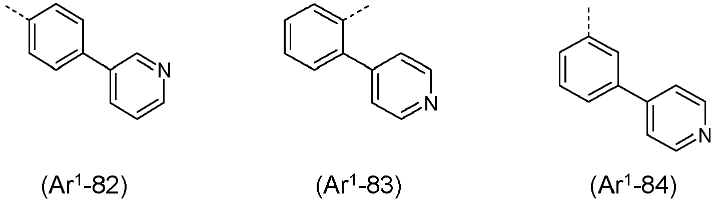 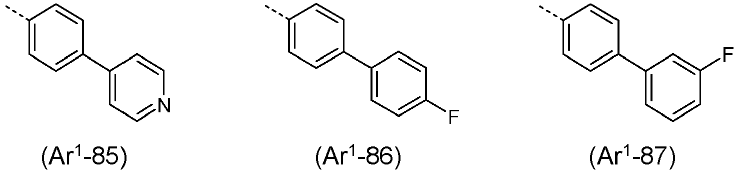 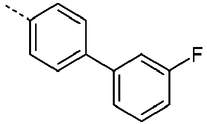 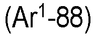
and wherein the remaining variables are defined as above;
wherein compounds of the following formula (B) are excluded in each case from the scope of formulae (A-I) to (A-IX) 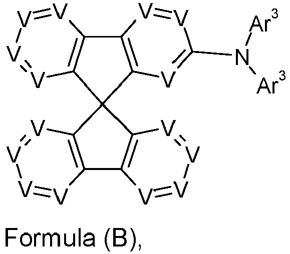 in which the following applies to the newly occurring variables:
V is equal to CR¹;
Ar³ is on each occurrence, identically or differently, an aromatic ring system having 6 to 30 aromatic ring atoms, which may be substituted by one or more radicals R¹, or a heteroaromatic ring system having 5 to 30 aromatic ring atoms, which may be substituted by one or more radicals R¹; groups Ar¹ may be connected to one another via radicals R¹;.

2. Material according to Claim 1, **characterised in that** compound A comprises a single amino group.

3. Material according to Claim 1 or 2, **characterised in that** compound A comprises no condensed aryl group having more than 10 aromatic ring atoms and no condensed heteroaryl group having more than 14 aromatic ring atoms.

4. Material according to one or more of Claims 1 to 3, **characterized in that** in compound A in formulae (A-II) and (A-IV) to (A-IX) the group Ar¹ in each occurrence is, identically or differently, an aromatic ring system having 6 to 40 aromatic ring atoms, which may be substituted by one or more radicals R¹, or a heteroaromatic ring system having 5 to 40 aromatic ring atoms, which may be substituted by one or more radicals R¹.

5. Material according to one or more of Claims 1 to 4, **characterized in that** in compound A in formulae (A-II) and (A-IV) to (A-IX) the group Ar¹ is a group optionally substituted with one or more radicals R¹ selected from phenyl, biphenyl, terphenyl, quaterphenyl, naphthyl, phenanthryl, fluoranthenyl, fluorenyl, indenofluorenyl, spirobifluorenyl, dibenzofuranyl, dibenzothiophenyl, carbazolyl, acridyl and phenanthridyl.

6. Material according to one or more of Claims 1 to 5, **characterized in that** the compound P is a complex of bismuth in oxidation state (III).

7. Material according to one or more of Claims 1 to 6, **characterised in that** compound P is a complex of bismuth which comprises at least one ligand L which is an organic compound.

8. Material according to Claim 7, **characterised in that** the ligand L has a single negative charge.

9. Material according to Claim 7 or 8, **characterised in that** the group of the ligand L that is bonded to the bismuth atom is selected from carboxyl groups, thiocarboxyl groups, carboxamide groups and carboximide groups.

10. Material according to Claim 7, **characterized in that** the ligand L is selected from fluorinated benzoic acid derivatives, fluorinated or nonfluorinated phenylacetic acid derivatives and fluorinated or nonfluorinated acetic acid derivatives.

11. Material according to one or more of Claims 1 to 10, **characterized in that** the compound P is present as a dopant in a concentration of 0.1 % to 20 % in the material.

12. Layer comprising a material according to one or more of Claims 1 to 11.

13. Formulation comprising a material according to one or more of Claims 1 to 11 and at least one solvent.

14. Process for the production of a layer according to Claim 12, **characterised in that** compound A and compound P are applied together from the gas phase, or **in that** a formulation comprising the material according to the invention is applied from the liquid phase.

15. Use of a material according to one or more of Claims 1 to 11 in an electronic device, selected from organic electroluminescent devices, organic integrated circuits, organic field-effect transistors, organic thin-film transistors, organic light-emitting transistors, organic solar cells, organic optical detectors, organic photoreceptors, organic fieldquench devices, organic light-emitting electrochemical cells and organic laser diodes.

16. Electronic device, selected from organic electroluminescent devices, organic integrated circuits, organic field-effect transistors, organic thin-film transistors, organic light-emitting transistors, organic solar cells, organic optical detectors, organic photoreceptors, organic fieldquench devices, organic light-emitting electrochemical cells and organic laser diodes, comprising a material according to one or more of Claims 1 to 11.

17. Organic electroluminescent device according to Claim 16, **characterised in that** it comprises the material in a hole-transporting layer which is arranged between anode and emitting layer, where one or more further layers are present between the layer comprising the material and the emitting layer.

18. Organic electroluminescent device according to claim 17, **characterized in that** the one or more further layers that are arranged between the layer comprising the material and the emitting layer comprise one or more identical or different compounds according to one of the formulae (A-I) to (A-IX).

19. Organic electroluminescent device according to one or more of Claims 16 to 18, **characterised in that** it comprises the material in a layer that is directly adjacent to the anode.

## Revendications

1. Matériau comprenant un composé P qui est un complexe de bismuth et un composé A qui est sélectionné parmi les composés selon l'une des formules (A-II) et (A-IV) à (A-IX)
| | |
|---|---|
| | 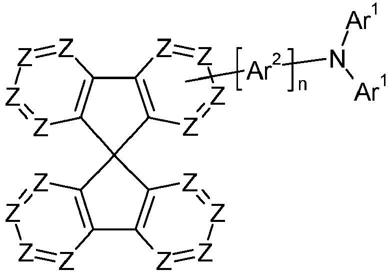 |
| | Formule (A-II) |
| 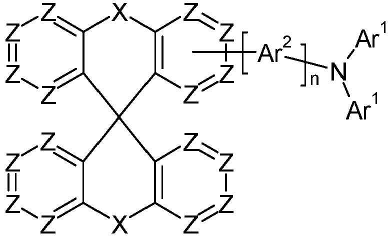 | |
| Formule (A-IV) | |
| 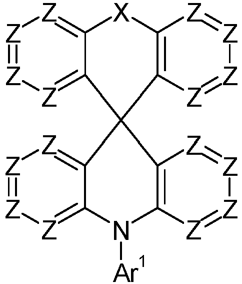 | 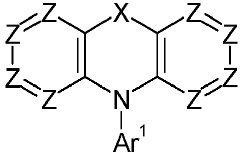 |
| Formule (A-V) | Formule (A-VI) |
| 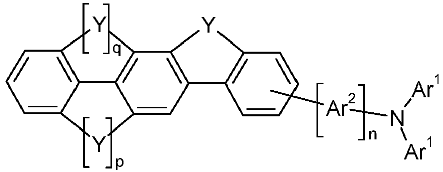 | 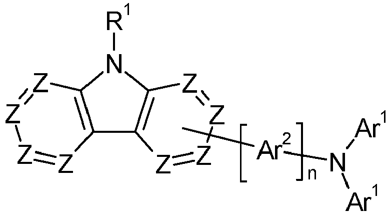 |
| Formule (A-VII) | Formule (A-VIII) |
| 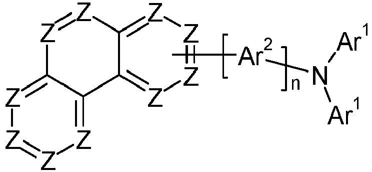 | |
| Formule (A-IX) | |
où un ou plusieurs radicaux R¹ peuvent être liés à toutes les positions présentées comme non substituées, et ce qui suit s'applique aux variables présentes:
Z est pour chaque occurrence, de manière identique ou différente, CR1 ou N;
X est pour chaque occurrence, de manière identique ou différente, une liaison simple, O, S, C(R¹)₂, Si(R¹)₂, PR¹, C(R¹)₂-C(R¹)₂, ou CR¹=CR¹;
Y est pour chaque occurrence, de manière identique ou différente, O, S, C(R¹)₂, Si(R¹)₂, PR¹, NR¹, C(R¹)₂-C(R¹)₂, ou CR¹=CR¹;
Ar¹ Ar¹ est pour chaque occurrence, de manière identique ou différente, un système de cycle aromatique qui comporte de 6 à 60 atomes de cycle aromatique, lequel peut être substitué par un radical ou par plusieurs radicaux R¹, ou un système de cycle hétéroaromatique qui comporte de 5 à 60 atomes de cycle aromatique, lequel peut être substitué par un radical ou par plusieurs radicaux R¹; les groupes Ar¹ peuvent être liés l'un à l'autre ou les uns aux autres via des radicaux R¹;
Ar² est à chaque occurrence, de manière identique ou différente, un système de cycle aromatique qui comporte de 6 à 60 atomes de cycle aromatique qui peuvent être substitués par un ou plusieurs radicaux R¹, ou un système de cycle hétéroaromatique qui comporte de 5 à 60 atomes de cycle aromatique qui peuvent être substitués par un ou plusieurs radicaux R¹; les groupes Ar¹ peuvent être liés les uns aux autres via des radicaux R¹;
R¹ est sélectionné pour chaque occurrence, de manière identique ou différente, parmi H, D, F, C(=O)R², CN, Si(R²)₃, P(=O)(R²)₂, OR², S(=O)R², S(=O)₂R², des groupes alkyle ou alcoxy en chaîne droite qui comportent de 1 à 20 atome(s) de C, des groupes alkyle ou alcoxy ramifiés ou cycliques qui comportent de 3 à 20 atomes de C, des groupes alkényle ou alkynyle qui comportent de 2 à 20 atomes de C, des systèmes de cycle aromatique qui comportent de 6 à 40 atomes de cycle aromatique, et des systèmes de cycle hétéroaromatique qui comportent de 5 à 40 atomes de cycle aromatique; où deux radicaux R¹ ou plus peuvent être liés l'un à l'autre ou les uns aux autres et peuvent former un cycle; où lesdits groupes alkyle, alcoxy, alkényle et alkynyle et lesdits systèmes de cycle aromatique et lesdits systèmes de cycle hétéroaromatique peuvent dans chaque cas être substitués par un radical ou par plusieurs radicaux R²; et où un ou plusieurs groupe(s) CH₂ dans lesdits groupes alkyle, alcoxy, alkényle et alkynyle peut/peuvent être remplacé(s) par -R²C=CR²-, -C≡C-, Si(R²)₂, C=O, C=NR², -C(=O)O-,-C(=O)NR²-, P(=O)(R²), -O-, -S-, SO ou SO₂;
R² est sélectionné pour chaque occurrence, de manière identique ou différente, parmi H, D, F, CN, des groupes alkyle qui comportent de 1 à 20 atome(s) de C, des systèmes de cycle aromatique qui comportent de 6 à 40 atomes de cycle aromatique et des systèmes de cycle hétéroaromatique qui comportent de 5 à 40 atomes de cycle aromatique; où deux radicaux R² ou plus peuvent être liés l'un à l'autre ou les uns aux autres et peuvent former un cycle; et où lesdits groupes alkyle, lesdits systèmes de cycle aromatique et lesdits systèmes de cycle hétéroaromatique peuvent être substitués par F ou par CN;
n,p,q est, de manière identique ou différente, 0 ou 1;
ou sélectionné parmi les composés selon l'une des formules (A-I) et (A-III):
| |
|---|
| 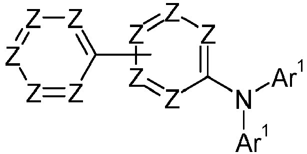 |
| Formule (A-I) |
| 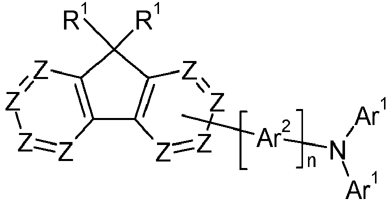 |
| Formule (A-III), |
dans lequel un ou plusieurs radicaux R¹ peuvent être liés à toutes les positions présentées comme non substituées, et
au moins un groupe Ar¹ est sélectionné pour chaque occurrence, de manière identique ou différente, parmi les groupes suivants, chacun pouvant être substitué à toutes les positions indiquées comme non substitué par un ou plusieurs radicaux R¹: 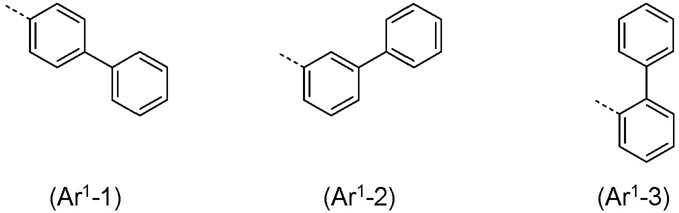 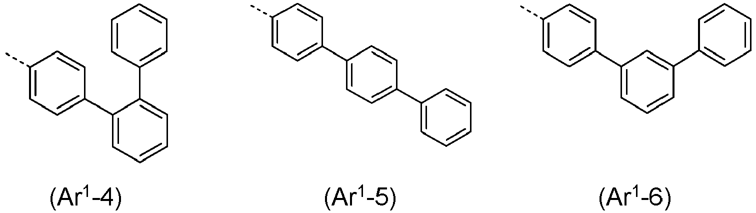 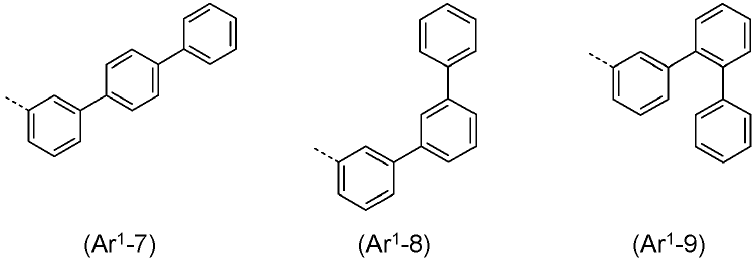 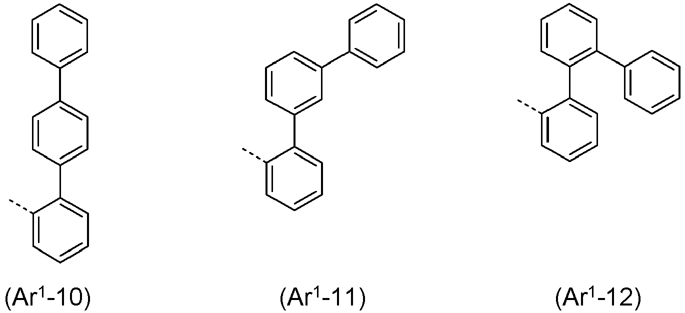 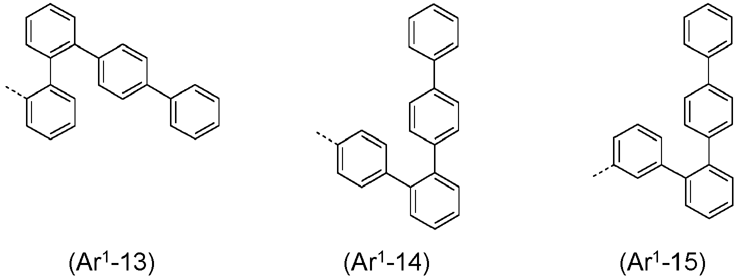 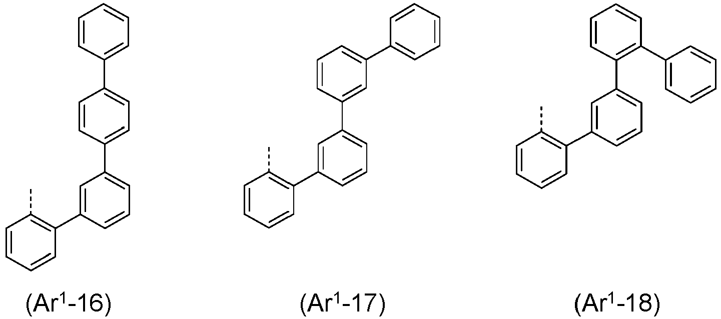 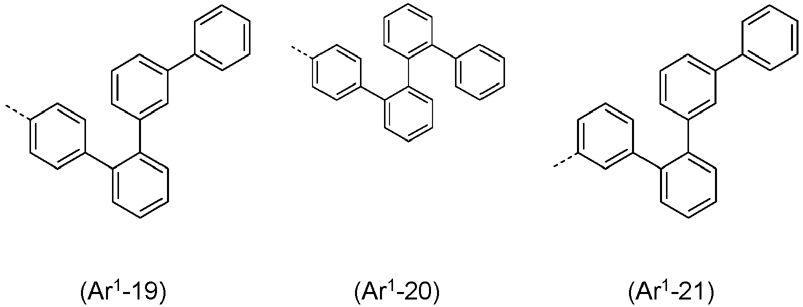 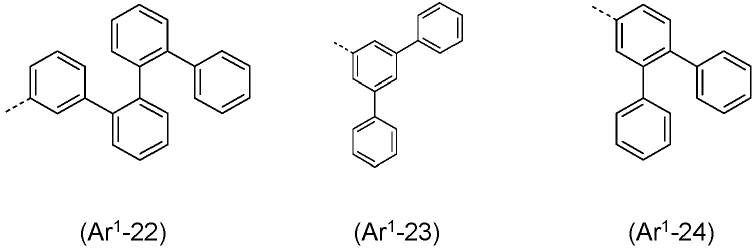 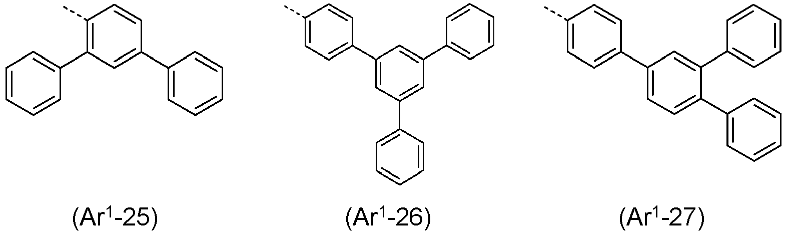 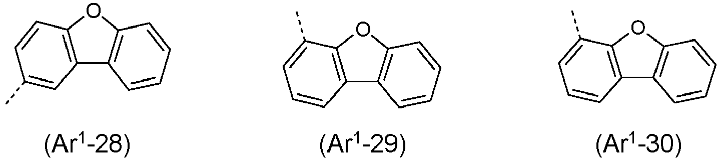 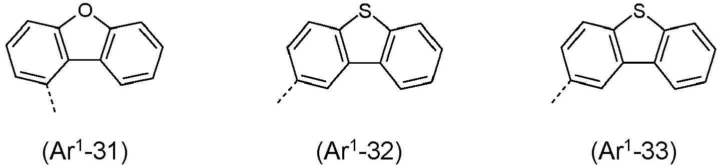 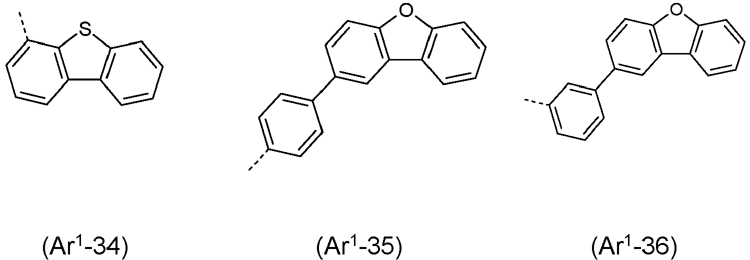 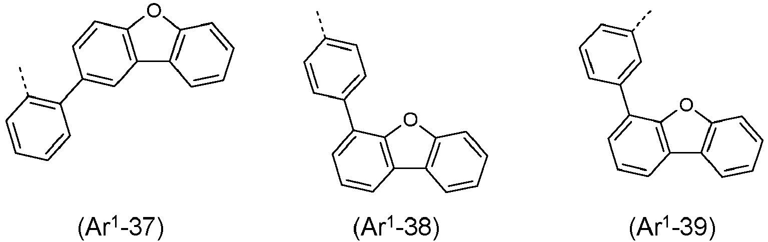 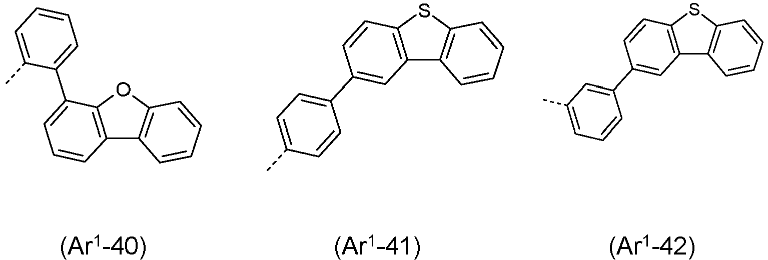 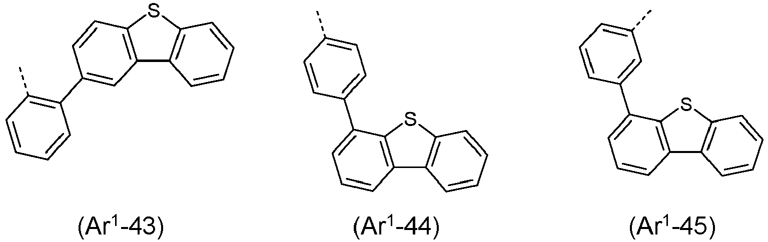 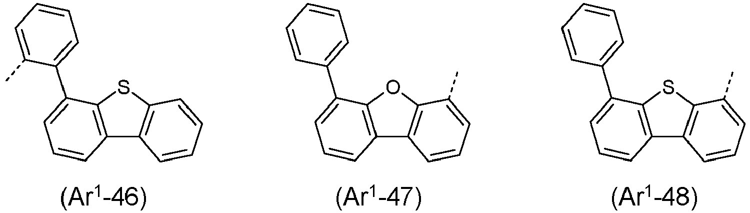 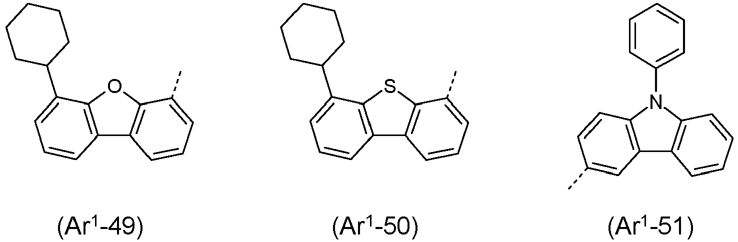 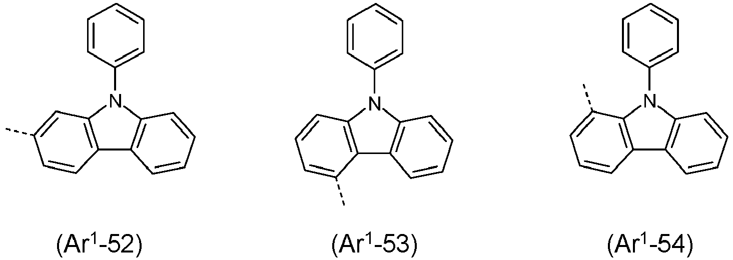 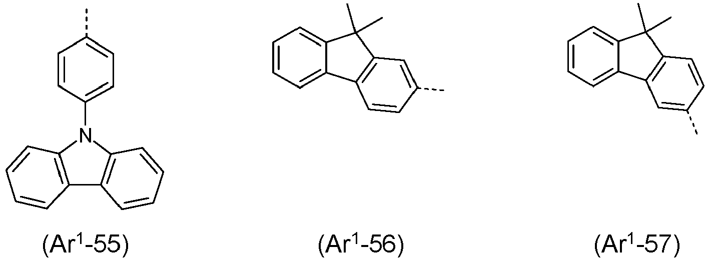 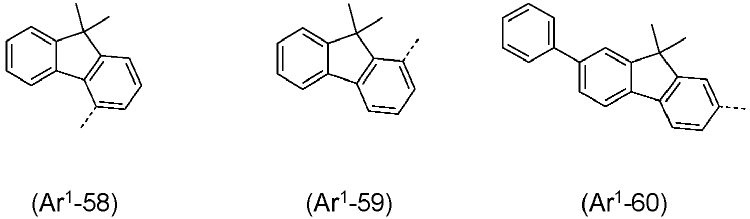 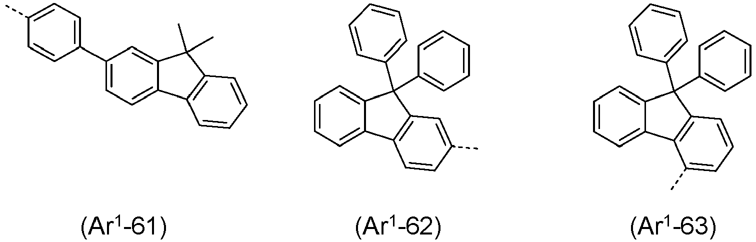 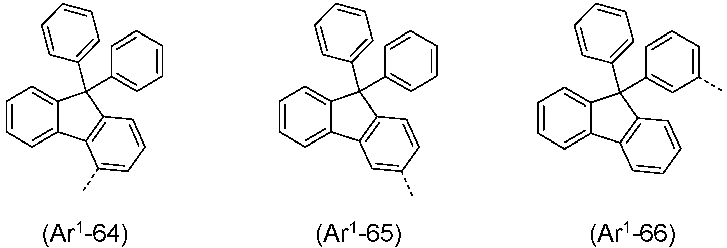 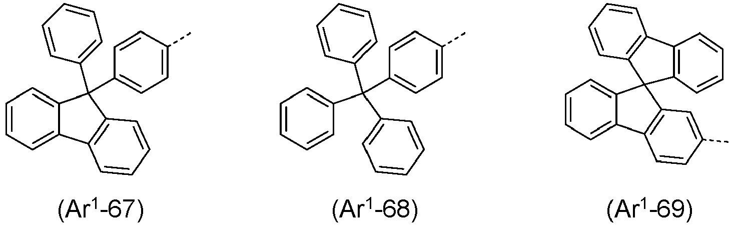 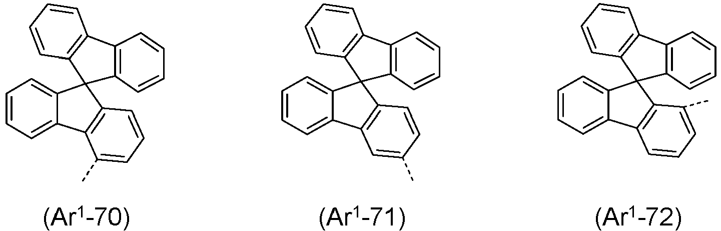 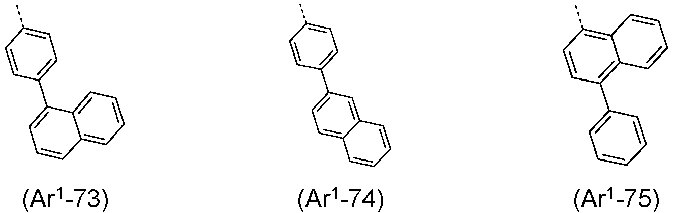 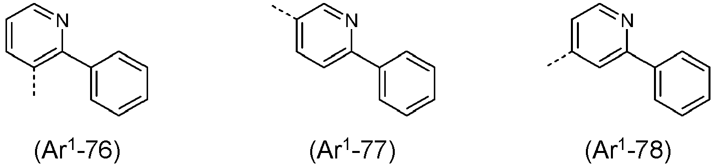 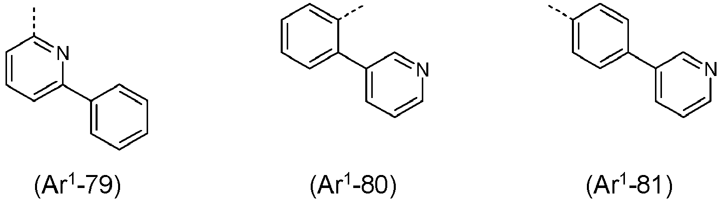 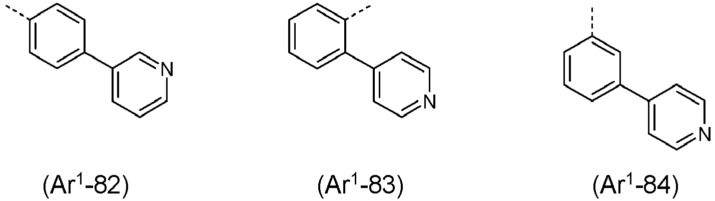 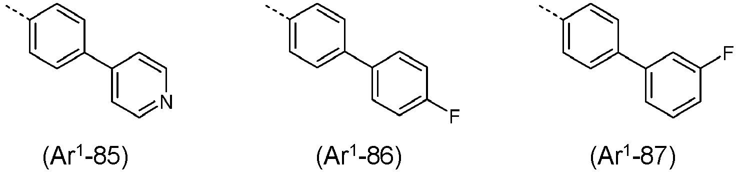 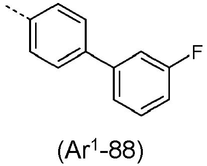
et où les autres variables sont définies comme ci-dessus;
dans lequel les composés de Formule (B) suivante sont exclus dans chaque cas du portée des formules (A-I) à (A-IX) 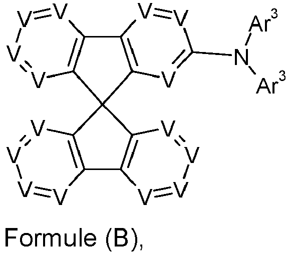
dans laquelle ce qui suit s'applique aux variables nouvellement présentes:
V est égal à CR¹;
Ar³ est pour chaque occurrence, de manière identique ou différente, un système de cycle aromatique qui comporte de 6 à 30 atomes de cycle aromatique, lequel peut être substitué par un radical ou par plusieurs radicaux R¹, ou un système de cycle hétéroaromatique qui comporte de 5 à 30 atomes de cycle aromatique, lequel peut être substitué par un radical ou par plusieurs radicaux R¹; les groupes Ar¹ peuvent être liés l'un à l'autre ou les uns aux autres via des radicaux R¹.

2. Matériau selon la revendication 1, **caractérisé en ce que** le composé A contient un unique groupe amino.

3. Matériau selon la revendication 1 ou 2, **caractérisé en ce que** le composé A ne contient pas de groupe aryle condensé comportant plus de 10 atomes de cycle aromatique et pas de groupe hétéroaryle condensé comportant plus de 14 atomes de cycle aromatique.

4. Matériau selon une ou plusieurs des revendications 1 à 3, **caractérisé en ce que** le groupe Ar¹ dans le composé A dans les formules (A-II) et (A-IV) à (A-IX) est pour chaque occurrence, de manière identique ou différente, un système de cycle aromatique qui comporte de 6 à 40 atomes de cycle aromatique, lequel peut être substitué par un radical ou par plusieurs radicaux R¹, ou un système de cycle hétéroaromatique qui comporte de 5 à 40 atomes de cycle aromatique, lequel peut être substitué par un radical ou par plusieurs radicaux R¹.

5. Matériau selon une ou plusieurs des revendications 1 à 4, **caractérisé en ce que** le groupe Ar¹ dans le composé A dans les formules (A-II) et (A-IV) à (A-IX) est pour chaque occurrence, de manière identique ou différente, est un groupe optionnellement substitué par un ou plusieurs radicaux R¹ sélectionnés parmi phényle, biphényle, terphényle, quaterphényle, naphtyle, phénanthryle, fluoranthényle, fluorényle, indénofluorényle, spirobifluorényle, dibenzofuranyle, dibenzothiophényle, carbazolyle, acridyle et phénanthridyle.

6. Matériau selon une ou plusieurs des revendications 1 à 5, **caractérisé en ce que** le composé P est un complexe de bismuth dans un état d'oxydation (III).

7. Matériau selon une ou plusieurs des revendications 1 à 6, **caractérisé en ce que** le composé P est un complexe de bismuth qui contient au moins un ligand L qui est un composé organique.

8. Matériau selon la revendication 7, **caractérisé en ce que** le ligand L présente une unique charge négative.

9. Matériau selon la revendication 7 ou 8, **caractérisé en ce que** le groupe du ligand L qui est lié à l'atome de bismuth est sélectionné parmi les groupes carboxyle, les groupes thiocarboxyle, les groupes carboxamide et les groupes carboximide.

10. Matériau selon la revendication 7, **caractérisé en ce que** le ligand L est sélectionné parmi les dérivés d'acide benzoïque fluorés, les dérivés d'acide phénylacétique fluorés ou non fluorés et les dérivés d'acide acétique fluorés ou non fluorés.

11. Matériau selon une ou plusieurs des revendications 1 à 10, **caractérisé en ce que** le composé P est présent dans le matériau en tant que dopant selon une concentration de 0,1% à 20%.

12. Couche comprenant un matériau selon une ou plusieurs des revendications 1 à 11.

13. Formulation comprenant un matériau selon une ou plusieurs des revendications 1 à 11 et au moins un solvant.

14. Procédé pour la production d'une couche selon la revendication 12, **caractérisé en ce que** le composé A et le composé P sont appliqués en association à partir de la phase gazeuse, ou **en ce qu'**une formulation qui comprend le matériau selon l'invention est appliquée à partir de la phase liquide.

15. Utilisation d'un matériau selon une ou plusieurs des revendications 1 à 11 dans un dispositif électronique, lequel est sélectionné parmi les dispositifs électroluminescents organiques, les circuits intégrés organiques, les transistors à effet de champ organiques, les transistors à film mince organiques, les transistors à émission de lumière organiques, les cellules solaires organiques, les détecteurs optiques organiques, les photorécepteurs organiques, les dispositifs à extinction de champ organiques, les cellules électrochimiques à émission de lumière organiques et les diodes laser organiques.

16. Dispositif électronique, sélectionné parmi les dispositifs électroluminescents organiques, les circuits intégrés organiques, les transistors à effet de champ organiques, les transistors à film mince organiques, les transistors à émission de lumière organiques, les cellules solaires organiques, les détecteurs optiques organiques, les photorécepteurs organiques, les dispositifs à extinction de champ organiques, les cellules électrochimiques à émission de lumière organiques et les diodes laser organiques, comprenant un matériau selon une ou plusieurs des revendications 1 à 11.

17. Dispositif électroluminescent organique selon la revendication 16, **caractérisé en ce qu'**il comprend le matériau dans une couche de transport de trous qui est agencée entre une anode et une couche d'émission, où une ou plusieurs autre(s) couche(s) est/sont présente(s) entre la couche qui comprend le matériau et la couche d'émission.

18. Dispositif électroluminescent organique selon la revendication 17, **caractérisé en ce que** les une ou plusieurs autres couches qui sont agencées entre la couche qui comprend le matériau et la couche d'émission comprennent un ou plusieurs composés identiques ou différents selon l'une des formules (A-I) à (A-IX).

19. Dispositif électroluminescent organique selon une ou plusieurs des revendications 16 à 18, **caractérisé en ce qu'**il contient le matériau dans une couche qui est directement adjacente à l'anode.
